# EUROPÄISCHE PATENTSCHRIFT

(11) **EP 2 616 448 B1**
(45) Veröffentlichungstag und Bekanntmachung des Hinweises auf die Patenterteilung: **20.07.2016**
(21) Anmeldenummer: 11758447.4
(22) Anmeldetag: 14.09.2011
(51) Int. Cl.: C07D 249/12, C07D 409/04, A61K 31/4196, A61P 9/00

(54) **SUBSTITUIERTE PHENYLACET- UND PHENYLPROPANAMIDE UND IHRE VERWENDUNG**
SUBSTITUTED PHENYLACETATE AND PHENYLPROPANE AMIDES AND USE THEREOF
PHÉNYLACÉTAMIDES ET PHÉNYLPROPANAMIDES SUBSTITUÉS ET LEUR UTILISATION

(30) Priorität: 16.09.2010 DE 102010040924
(43) Veröffentlichungstag der Anmeldung: 24.07.2013
(73) Patentinhaber: Bayer Intellectual Property GmbH, 40789 Monheim (DE)
(72) Erfinder: FÜRSTNER, Chantal, 45478 Mülheim (DE); KELDENICH, Jörg, 42113 Wuppertal (DE); DELBECK, Martina, 42579 Heiligenhaus (DE); KOLKHOF, Peter, 42113 Wuppertal (DE); KRETSCHMER, Axel, 42113 Wuppertal (DE); POOK, Elisabeth, 42109 Wuppertal (DE); SCHMECK, Carsten, 45472 Mülheim (DE); TRÜBEL, Hubert, 42281 Wuppertal (DE)
(74) Vertreter: BIP Patents
(86) Internationale Anmeldenummer: PCT/EP2011/065961
(87) Internationale Veröffentlichungsnummer: WO 2012/035075

(56) Entgegenhaltungen:
- DE-A1-102006 024 024

## Beschreibung

Die vorliegende Anmeldung betrifft neue, substituierte Phenylacet- und Phenylpropanamide, Verfahren zu ihrer Herstellung sowie ihre Verwendung zur Herstellung von Arzneimitteln zur Behandlung und/oder Prävention von Krankheiten, insbesondere zur Behandlung und/oder Prävention von kardiovaskulären Erkrankungen.

Der Flüssigkeitsgehalt des menschlichen Körpers unterliegt verschiedenen physiologischen Kontrollmechanismen, die auf seine Konstanterhaltung abzielen (Volumenhomöostase). Dabei werden sowohl die Volumenfüllung des Blutgefaßsystems als auch die Osmolarität des Blutplasmas kontinuierlich von entsprechenden Sensoren (Barorezeptoren und Osmorezeptoren) registriert. Die Informationen, die diese Sensoren an die zuständigen Zentren des Hirns liefern, regulieren das Trinkverhalten und steuern vermittels humoraler und nervaler Signale die Flüssigkeitsausscheidung über die Nieren. Eine zentrale Bedeutung kommt hierbei dem Peptidhormon Vasopressin zu [Schrier R.W., Abraham, W.T., New Engl. J. Med. 341, 577-585 (1999)].

Vasopressin wird in spezialisierten, endokrinen Neuronen im *Nucleus supraopticus* und *N. paraventricularis* in der Wand des dritten Ventrikels (Hypothalamus) produziert und von dort entlang ihrer Nervenfortsätze in den Hypophysenhinterlappen (Neurohypophyse) verbracht. Dort wird das Hormon je nach Stimulus in die Blutbahn abgegeben. Ein Volumenverlust, z.B. infolge akuter Blutung, starkem Schwitzen, langem Dursten oder Diarrhöe, ist ein Stimulus für die verstärkte Ausschüttung des Hormons. Umgekehrt wird die Sekretion von Vasopressin durch eine Erhöhung des Intravasalvolumens, z.B. infolge gesteigerter Flüssigkeitszufuhr, gehemmt.

Vasopressin entfaltet seine Wirkung hauptsächlich über Bindung an drei Rezeptoren, die als V1a-, V1b- und V2-Rezeptoren klassifiziert werden und zur Familie der G-Protein-gekoppelten Rezeptoren gehören. V1a-Rezeptoren sind vorwiegend auf den Zellen der glatten Gefäßmuskulatur lokalisiert. Ihre Aktivierung ruft eine Vasokonstriktion hervor, wodurch der periphere Widerstand und Blutdruck ansteigen. Daneben sind V1a-Rezeptoren auch in der Leber nachweisbar. V1b-Rezeptoren (auch V3-Rezeptoren genannt) sind im zentralen Nervensystem nachweisbar. Zusammen mit dem Corticotropin-Releasing-Hormon (CRH) reguliert Vasopressin über den V1b-Rezeptor die basale und stressinduzierte Sekretion des Adrenocorticotropen Hormons (ACTH). V2-Rezeptoren finden sich im distalen Tubulusepithel und dem Epithel der Sammelrohre der Niere. Ihre Aktivierung macht diese Epithelien für Wasser durchlässig. Diesem Phänomen liegt der Einbau von Aquaporinen (speziellen Wasserkanälen) in die luminale Membran der Epithelzellen zugrunde.

Welche Bedeutung Vasopressin für die Rückresorption von Wasser aus dem Harn in der Niere hat, wird durch das Krankheitsbild des Diabetes insipidus deutlich, das durch einen Mangel des Hormons - z.B. infolge einer Hypophysenschädigung - hervorgerufen wird. Patienten, die an diesem Krankheitsbild leiden, scheiden bis zu 20 Liter Harn pro 24 Stunden aus, sofern ihnen das Hormon nicht substituiert wird. Dieses Volumen entspricht in etwa 10% des Primärharns. Wegen seiner großen Bedeutung für die Rückresorption von Wasser aus dem Harn wird Vasopressin auch synonym als Antidiuretisches Hormon (ADH) bezeichnet. Folgerichtig führt eine pharmakologische Hemmung der Vasopressin/ADH-Wirkung am V2-Rezeptor zu einer vermehrten Urinausscheidung. Im Gegensatz zu der Wirkung anderer Diuretika (Thiazide und Schleifendiuretika) jedoch bewirken V2-Rezeptor-Antagonisten eine vermehrte Wasserausscheidung, ohne die Ausscheidung von Elektrolyten maßgeblich zu steigern. Das bedeutet, dass durch V2-antagonistische Pharmaka die Volumenhomöostase wiederhergestellt werden kann, ohne dabei in die Elektrolyt-Homöostase einzugreifen. Daher erscheinen V2-antagonistisch wirksame Pharmaka besonders geeignet für die Behandlung aller krankhaften Zustände, die mit einer Überfrachtung des Organismus mit Wasser einhergehen, ohne dass parallel die Elektrolyte adäquat erhöht sind. Eine bedeutende Elektrolyt-Abnormalität ist klinisch-chemisch als Hyponatriämie (Natriumkonzentration < 135 mmol/L) messbar; sie stellt die wichtigste Elektrolyt-Abnormalität bei Krankenhauspatienten dar mit einer Häufigkeit von ca. 5% bzw. 250.000 Fällen pro Jahr allein in den USA. Bei einem Absinken der Plasma-Natriumkonzentration unter 115 mmol/L drohen komatöse Zustände und Tod.

Entsprechend der zugrunde liegenden Ursache unterscheidet man die hypovolämische, euvolämische und hypervolämische Hyponatriämie. Klinisch bedeutsam sind die Formen der Hypervolämie mit Ödembildung. Typische Beispiele hierfür sind das Syndrom der inadäquaten ADH/Vasopressin-Sekretion (SIAD) (z.B. nach Schädel-Hirn-Trauma oder als Paraneoplasie bei Carcinomen) und die hypervolämische Hyponatriämie bei Leberzirrhose, verschiedenen Nierenerkrankungen und Herzinsuffizienz [De Luca L. et al., Am. J. Cardiol. 96 (suppl.), 19L-23L (2005)]. Gerade Patienten mit Herzinsuffizienz weisen trotz ihrer relativen Hyponatriämie und Hypervolämie häufig erhöhte Vasopressin-Spiegel auf, was als die Folge einer generell gestörten neurohumoralen Regulation bei der Herzinsuffizienz angesehen wird [Francis G.S. et al., Circulation 82, 1724-1729 (1990)].

Die gestörte neurohumorale Regulation manifestiert sich im Wesentlichen in einer Erhöhung des Sympathicotonus und einer inadäquaten Aktivierung des Renin-Angiotensin-Aldosteron-Systems. Während die Hemmung dieser Komponenten durch Beta-Rezeptoren-Blocker einerseits und durch ACE-Inhibitoren bzw. Angiotensin-Rezeptor-Blocker andererseits heute fester Bestandteil der pharmakologischen Behandlung der Herzinsuffizienz ist, ist die inadäquate Steigerung der Vasopressin-Sekretion in der fortgeschrittenen Herzinsuffizienz derzeit noch nicht ausreichend therapierbar.

Nebem der durch V2-Rezeptoren vermittelten Retention von Wasser und den damit verbundenen ungünstigen hämodynamischen Folgen im Sinne einer Nachlasterhöhung werden auch durch V1a-vermittelte Vasokonstriktion die Entleerung des linken Ventrikels, der Druck in den Pulmonalgefäßen und die Herzleistung negativ beeinflusst. Darüber hinaus wird aufgrund tierexperimenteller Daten dem Vasopressin auch eine direkte Hypertrophie-fördernde Wirkung am Herzmuskel beigemessen. Im Unterschied zur renalen Wirkung der Volumenexpansion, die über Aktivierung von V2-Rezeptoren vermittelt ist, wird die direkte Wirkung am Herzmuskel durch Aktivierung von V1a-Rezeptoren ausgelöst.

Aus diesen Gründen erscheinen Substanzen, die die Wirkung des Vasopressins am V2- und/oder am V1a-Rezeptor hemmen, zur Behandlung der Herzinsuffizienz geeignet. Vor allem Verbindungen mit kombinierter Aktivität an beiden Vasopressin-Rezeptoren (V1a und V2) sollten sowohl wünschenswerte renale als auch hämodynamische Effekte bewirken und somit ein besonders ideales Profil für die Behandlung von Patienten mit Herzinsuffizienz bieten. Die Bereitstellung derartig kombinierter Vasopressin-Antagonisten erscheint auch insofern sinnvoll, als ein allein über V2-Rezeptor-Blockade vermittelter Volumenentzug die Erregung von Osmorezeptoren und in der Folge eine weitere kompensatorische Steigerung der Vasopressin-Ausschüttung nach sich ziehen kann. Hierdurch könnten - bei Fehlen einer gleichzeitig den V1a-Rezeptor blockierenden Komponente - die schädlichen Wirkungen des Vasopressins, wie z.B. Vasokonstriktion und Herzmuskelhypertrophie, noch verstärkt werden [Saghi P. et al., Europ. Heart J. 26, 538-543 (2005)].

In WO 99/54315 werden substituierte Triazolone mit neuroprotektiver Wirkung offenbart, und in WO 2006/117657 werden Triazolon-Derivate als anti-inflammatorische Agentien beschrieben. Ferner werden in EP 503 548-A1 und EP 587 134-A2 cyclische Harnstoff-Derivate und ihre Verwendung zur Behandlung von Thrombosen beansprucht. Substituierte Triazolthione als Ionenkanal-Modulatoren werden in WO 2005/097112 offenbart. WO 2007/134862 beschreibt substituierte Imidazol-2-one und 1,2,4-Triazolone als Vasopressin Rezeptor Antagonisten zur Behandlung kardiovaskulärer Erkrankungen. Verschiedenartig-substituierte Triazolone als Vasopressin Rezeptor Antagonisten werden in WO 2010/105770, WO 2010/105750, WO 2011/104322 und WO 2011/023703 offenbart.

Aufgabe der vorliegenden Erfindung ist die Bereitstellung neuer Verbindungen, die als potente, selektive duale V1a/V2-Rezeptor-Antagonisten wirken und eine erhöhte Löslichkeit in wässrigen Medien aufweisen, und als solche zur Behandlung und/oder Prävention von Krankheiten, insbesondere zur Behandlung und/oder Prävention von kardiovaskulären Erkrankungen geeignet sind.

Gegenstand der vorliegenden Erfindung sind Verbindungen der allgemeinen Formel (I) 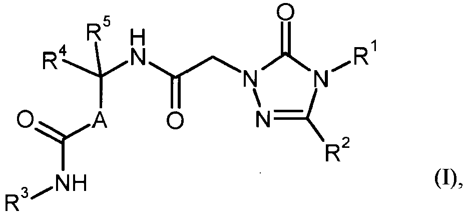 in welcher
- A: für eine Bindung oder -C(R^{6A}R^{6B})- steht,
wobei
R^{6A} für Wasserstoff, (C₁-C₄)-Alkyl oder Trifluormethyl steht,
R^{6B} für Wasserstoff oder (C₁-C₄)-Alkyl steht,
- R¹: für (C₁-C₆)-Alkyl, (C₂-C₆)-Alkenyl, (C₂-C₆)-Alkinyl oder (C₃-C₇)-Cycloalkyl steht,
wobei (C₁-C₆)-Alkyl, (C₂-C₆)-Alkenyl und (C₂-C₆)-Alkinyl mit 1 bis 3 Substituenten unabhängig voneinander ausgewählt aus der Gruppe Deuterium, Halogen, Cyano, Oxo, Hydroxy, Trifluormethyl, (C₃-C₇)-Cycloalkyl, (C₁-C₆)-Alkoxy, Trifluormethoxy und Phenyl substituiert sein können,
worin (C₃-C₇)-Cycloalkyl mit 1 oder 2 Substituenten unabhängig voneinander ausgewählt aus der Gruppe (C₁-C₄)-Alkyl, Oxo, Hydroxy, (C₁-C₄)-Alkoxy und Amino substituiert sein kann,
und
worin (C₁-C₆)-Alkoxy mit 1 oder 2 Substituenten unabhängig voneinander ausgewählt aus der Gruppe Amino, Hydroxy, (C₁-C₄)-Alkoxy, Hydroxycarbonyl und (C₁-C₄)-Alkoxycarbonyl substituiert sein kann
und
worin Phenyl mit 1 bis 3 Substituenten unabhängig voneinander ausgewählt aus der Gruppe Halogen, Cyano, Nitro, (C₁-C₄)-Alkyl, Trifluormethyl, Hydroxy, Hydroxymethyl, (C₁-C₄)-Alkoxy, Trifluormethoxy, (C₁-C₄)-Alkoxymethyl, Hydroxycarbonyl, (C₁-C₄)-Alkoxycarbonyl, Aminocarbonyl, Mono-(C₁-C₄)-alkyl-aminocarbonyl und Di-(C₁-C₄)-alkylaminocarbonyl substituiert sein kann,
und
wobei (C₃-C₇)-Cycloalkyl mit 1 oder 2 Substituenten unabhängig voneinander ausgewählt aus der Gruppe Fluor, (C₁-C₄)-Alkyl, (C_{I}-C₄)-Alkoxy, Hydroxy, Amino und Oxo substituiert sein kann,
- R²: für Benzothienyl, Phenyl, Thienyl oder Furyl steht,
wobei Benzothienyl, Phenyl, Thienyl und Furyl mit 1 bis 3 Substituenten unabhängig voneinander ausgewählt aus der Gruppe Halogen, Cyano, Nitro, (C₁-C₄)-Alkyl, Trifluormethyl, Hydroxy, (C₁-C₄)-Alkoxy und Trifluormethoxy substituiert sein können,
- R³: für eine Gruppe der Formel 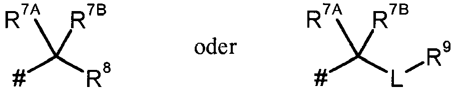 steht, wobei
# für die Anknüpfstelle an das Stickstoffatom steht,
L für (C₁-C₄)-Alkandiyl steht,
wobei (C₁-C₄)-Alkandiyl mit 1 bis 3 Substituenten unabhängig voneinander ausgewählt aus der Gruppe Fluor und (C₁-C₄)-Alkyl substituiert sein kann,
R^{7A} für Wasserstoff oder (C₁-C₄)-Alkyl steht,
R^{7B} für Wasserstoff oder (C₁-C₄)-Alkyl steht,
oder
R^{7A} und R^{7B} zusammen mit dem Kohlenstoffatom, an das sie gebunden sind, einen 3- bis 6-gliedrigen Carbocyclus bilden,
R⁸ für Hydroxycarbonyl, (C₁-C₄)-Alkoxycarbonyl, Aminocarbonyl, Mono-(C₁-C₄)-alkylaminocarbonyl oder Di-(C₁-C₄)-alkylaminocarbonyl steht,
R⁹ für Hydroxy, Trifluormethoxy, (C₁-C₄)-Alkoxy, Hydroxycarbonyl, (C₁-C₄)-Alkoxycarbonyl, Aminocarbonyl, Mono-(C₁-C₄)-alkylaminocarbonyl oder Di-(C₁-C₄)-alkylaminocarbonyl steht,
- R⁴: für Phenyl, Naphthyl oder 5- bis 10-gliedriges Heteroaryl steht,
wobei Phenyl, Naphthyl und 5- bis 10-gliedriges Heteroaryl mit 1 bis 3 Substituenten unabhängig voneinander ausgewählt aus der Gruppe Halogen, Cyano, Nitro, (C₁-C₄)-Alkyl, Difluormethyl, Trifluormethyl, Hydroxy, (C_{I}-C₄)-Alkoxy, Difluormethoxy und Trifluor-methoxy substituiert sein können,
- R⁵: für Wasserstoff, Deuterium, Trifluormethyl oder (C₁-C₄)-Alkyl steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 6 bzw. 1 bis 4 Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl.
Alkandiyl steht im Rahmen der Erfindung für einen linearen divalenten Alkylrest mit 1 bis 4 Kohlenstoffatomen. Beispielhaft und vorzugsweise seien genannt: Methylen, Ethan-1,2-diyl, Propan-1,3-diyl und Butan-1,4-diyl.
Cycloalkyl steht in Rahmen der Erfindung für einen monocyclischen, gesättigten Alkylrest mit 3 bis 7 bzw. 3 bis 6 Kohlenstoffatomen. Beispielhaft und vorzugsweise seien genannt: Cyclopropyl, Cyclobutyl, Cyclopentyl, Cyclohexyl und Cycloheptyl.
Carbocyclus steht in Rahmen der Erfindung für einen monocyclischen, gesättigten Alkylrest mit 3 bis 6 Kohlenstoffatomen. Beispielhaft und vorzugsweise seien genannt: Cyclopropyl, Cyclobutyl, Cyclopentyl und Cyclohexyl.
Alkenyl steht im Rahmen der Erfindung für einen linearen oder verzweigten Alkenylrest mit 2 bis 6 Kohlenstoffatomen und einer oder zwei Doppelbindungen. Bevorzugt ist ein geradkettiger oder verzweigter Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.
Alkinyl steht im Rahmen der Erfindung für einen linearen oder verzweigten Alkinylrest mit 2 bis 6 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.
Alkoxy steht im Rahmen der Erfindung für einen linearen oder verzweigten Alkoxyrest mit 1 bis 6 bzw. 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.
Alkoxycarbonyl stehen im Rahmen der Erfindung für einen linearen oder verzweigten Alkoxyrest mit 1 bis 4 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Mono-alkylaminocarbonyl steht im Rahmen der Erfindung für eine Amino-Gruppe, die über eine Carbonylgruppe verknüpft ist und die einen linearen oder verzweigten Alkylsubstituenten mit 1 bis 4 Kohlenstoffatomen aufweist. Beispielhaft und vorzugsweise seien genannt: Methylaminocarbonyl, Ethylaminocarbonyl, *n*-Propylaminocarbonyl, Isopropylaminocarbonyl, *n*-Butylaminocarbonyl und *tert.*-Butylaminocarbonyl.
Di-alkylaminocarbonyl steht im Rahmen der Erfindung für eine Amino-Gruppe, die über eine Carbonylgruppe verknüpft ist und die zwei gleiche oder verschiedene lineare oder verzweigte Alkylsubstituenten mit jeweils 1 bis 4 Kohlenstoffatomen aufweist. Beispielhaft und vorzugsweise seien genannt: *N,N-*Dimethylaminocarbonyl, *N,N-*Diethylaminocarbonyl, *N*-Ethyl-*N*-methylaminocarbonyl, *N*-Methyl-*N*-*n*-propylaminocarbonyl, *N*-*n*-Butyl-*N*-methylaminocarbonyl und *N*-tert.-Butyl-*N*-methylaminocarbonyl.
Heteroaryl steht im Rahmen der Erfindung für einen mono- oder gegebenenfalls bicyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl. Bevorzugt sind monocyclische 5- oder 6-gliedrige Heteroaryl-Reste mit bis zu drei Ring-Heteroatomen aus der Reihe N, O und/oder S wie beispielsweise Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl, Triazinyl.
Halogen schließt im Rahmen der Erfindung Fluor, Chlor, Brom und Iod ein. Bevorzugt sind Chlor oder Fluor.
Eine Oxo-Gruppe steht im Rahmen der Erfindung für ein Sauerstoffatom, das über eine Doppelbindung an ein Kohlenstoffatom gebunden ist.

In den Formeln der Gruppen, für die R³ bzw. R⁴ stehen kann, steht der Endpunkt der Linie, an dem das Zeichen # bzw. * steht, nicht für ein Kohlenstoffatom beziehungsweise eine CH2-Gruppe, sondern ist Bestandteil der Bindung zu dem jeweils bezeichneten Atom, an das R³ bzw. R⁴ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind im Rahmen der vorliegenden Erfindung Verbindungen der Formel (I), in welcher
- A: für eine Bindung oder -C(R^{6A}R^{6B})- steht,
wobei
R^{6A} für Wasserstoff steht,
R^{6B} für Wasserstoff steht,
- R¹: für (C₁-C₆)-Alkyl, (C₂-C₆)-Alkenyl oder (C₃-C₆)-Cycloalkyl steht,
wobei (C₁-C₆)-Alkyl und (C₂-C₆)-Alkenyl mit 1 bis 3 Substituenten unabhängig voneinander ausgewählt aus der Gruppe Fluor, Chlor, Cyano, Oxo, Hydroxy, Trifluormethyl, (C₃-C₆)-Cycloalkyl, (C_{I}-C₄)-Alkoxy, Trifluormethoxy und Phenyl substituiert sein können,
worin (C₃-C₆)-Cycloalkyl mit 1 oder 2 Substituenten unabhängig voneinander ausgewählt aus der Gruppe Methyl, Ethyl, Oxo, Hydroxy, Methoxy, Ethoxy und Amino substituiert sein kann,
und
worin Phenyl mit einem Substituenten ausgewählt aus der Gruppe Fluor, Chlor, Cyano, Methyl, Ethyl, Trifluormethyl, Methoxy, Ethoxy, Trifluormethoxy,
Methoxymethyl, Ethoxymethyl, Hydroxycarbonyl, Methoxycarbonyl, Ethoxy-carbonyl und Aminocarbonyl substituiert sein kann,
und
wobei (C₃-C₆)-Cycloalkyl mit 1 oder 2 Substituenten unabhängig voneinander ausgewählt aus der Gruppe Fluor, Methyl, Ethyl, Methoxy, Ethoxy, Hydroxy, Amino und Oxo substituiert sein kann,
- R²: für Phenyl oder Thienyl steht,
wobei Phenyl und Thienyl mit 1 oder 2 Substituenten unabhängig voneinander ausgewählt aus der Gruppe Fluor, Chlor, Brom, Methyl, Ethyl, Trifluormethyl, Hydroxy, Methoxy, Ethoxy und Trifluormethoxy substituiert sein können,
- R³: für eine Gruppe der Formel 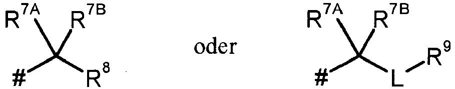 steht, wobei
# für die Anknüpfstelle an das Stickstoffatom steht,
L für Methylen steht,
wobei Methylen mit 1 oder 2 Substituenten Methyl substituiert sein kann,
R^{7A} für Wasserstoff oder Methyl steht,
R^{7B} für Wasserstoff oder Methyl steht,
oder
R^{7B} und R^{7B} zusammen mit dem Kohlenstoffatom, an das sie gebunden sind, einen Cyclopropyl-Ring bilden,
R⁸ für Hydroxycarbonyl, Methoxycarbonyl, Ethoxycarbonyl, Aminocarbonyl, Methyl-aminocarbonyl, Ethylaminocarbonyl, Dimethylaminocarbonyl oder Diethylamino-carbonyl steht,
R⁹ für Hydroxy, Trifluormethoxy, Methoxy, Ethoxy, Hydroxycarbonyl, Methoxy-carbonyl, Ethoxycarbonyl, Aminocarbonyl, Methylaminocarbonyl, Ethylamino-carbonyl, Dimethylaminocarbonyl oder Diethylaminocarbonyl steht,
- R⁴: für Phenyl steht,
wobei Phenyl mit 1 bis 3 Substituenten unabhängig voneinander ausgewählt aus der Gruppe Fluor, Chlor, Cyano, Methyl, Ethyl, Difluormethyl, Trifluormethyl, Methoxy, Difluor-methoxy und Trifluormethoxy substituiert ist,
- R⁵: für Wasserstoff oder Methyl steht,
sowie ihre Salze, Solvate und Solvate der Salze.

Besonders bevorzugt sind im Rahmen der vorliegenden Erfindung Verbindungen der Formel (I), in welcher
- A: für eine Bindung oder -C(R^{6A}R^{6B})- steht, wobei
R^{6A} für Wasserstoff steht,
R^{6B} für Wasserstoff steht,
- R¹: für (C₂-C₄)-Alkyl, (C₂-C₄)-Alkenyl oder Cyclopropyl steht,
wobei (C₂-C₄)-Alkyl und (C₂-C₄)-Alkenyl mit 1 oder 2 Substituenten unabhängig voneinander ausgewählt aus der Gruppe Fluor, Oxo, Hydroxy und Trifluormethyl substituiert sein können,
- R²: für Phenyl steht,
wobei Phenyl mit einem Substituenten ausgewählt aus der Gruppe Fluor oder Chlor substituiert ist,
- R³: für eine Gruppe der Formel 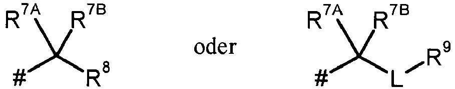 steht, wobei
# für die Anknüpfstelle an das Stickstoffatom steht,
L für Methylen steht,
R^{7A} für Wasserstoff oder Methyl steht,
R^{7B} für Wasserstoff oder Methyl steht,
oder
R^{7B} und R^{7B} zusammen mit dem Kohlenstoffatom, an das sie gebunden sind, einen Cyclopropyl-Ring bilden,
R⁸ für Hydroxycarbonyl, Methoxycarbonyl, Ethoxycarbonyl oder Aminocarbonyl steht,
R⁹ für Hydroxy, Methoxy, Hydroxycarbonyl, Methoxycarbonyl, Ethoxycarbonyl oder Aminocarbonyl steht,
- R⁴: für eine Gruppe der Formel 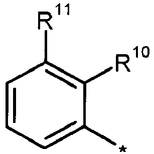 steht, wobei
* für die Anknüpfstelle an die Gruppe -C(R⁵)(AC(=O)NHR³)- steht,
R¹⁰ für Wasserstoff, Chlor, Trifluormethyl, Trifluormethoxy oder Methoxy steht,
R¹¹ für Wasserstoff, Fluor, Chlor, Trifluormethyl, Trifluormethoxy oder Methoxy steht,
wobei mindestens einer der Reste R¹⁰ und R¹¹ von Wasserstoff verschieden ist,
- R⁵: für Wasserstoff oder Methyl steht,
sowie ihre Salze, Solvate und Solvate der Salze.

Bevorzugt sind im Rahmen der vorliegenden Erfindung auch Verbindungen der Formel (I), in welcher R² für p-Chlorphenyl steht, sowie ihre Salze, Solvate und Solvate der Salze.

Bevorzugt sind im Rahmen der vorliegenden Erfindung auch Verbindungen der Formel (I), in welcher R¹ für 3,3,3-Trifluorprop-1-en-1-yl steht, sowie ihre Salze, Solvate und Solvate der Salze.

Bevorzugt sind im Rahmen der vorliegenden Erfindung auch Verbindungen der Formel (I), in welcher R¹ für 3,3,3-Trifluorpropyl steht, sowie ihre Salze, Solvate und Solvate der Salze.

Bevorzugt sind im Rahmen der vorliegenden Erfindung auch Verbindungen der Formel (I), in welcher R¹ für 1,1,1-Trifluorpropan-2-ol-3-yl steht, sowie ihre Salze, Solvate und Solvate der Salze.

Bevorzugt sind im Rahmen der vorliegenden Erfindung auch Verbindungen der Formel (I), in welcher
- R¹: für (C₂-C₄)-Alkyl oder (C₂-C₄)-Alkenyl steht,
wobei (C₂-C₄)-Alkyl und (C₂-C₄)-Alkenyl mit 1 oder 2 Substituenten unabhängig voneinander ausgewählt aus der Gruppe Fluor, Hydroxy, Oxo und Trifluormethyl substituiert sind
sowie ihre Salze, Solvate und Solvate der Salze.

Bevorzugt sind im Rahmen der vorliegenden Erfindung auch Verbindungen der Formel (I), in welcher R¹ für Cyclopropyl steht, sowie ihre Salze, Solvate und Solvate der Salze.

Bevorzugt sind im Rahmen der vorliegenden Erfindung auch Verbindungen der Formel (I), in welcher
- A: für -C(R^{6A}R^{6B})- steht,
wobei
R^{6A} für Wasserstoff steht,
R^{6B} für Wasserstoff steht,
sowie ihre Salze, Solvate und Solvate der Salze.

Bevorzugt sind im Rahmen der vorliegenden Erfindung auch Verbindungen der Formel (I), in welcher
- A: für -C(R^{6A}R^{6B})- steht,
wobei
R^{6A} für Wasserstoff steht,
R^{6B} für Wasserstoff steht,
- R³: für eine Gruppe der Formel 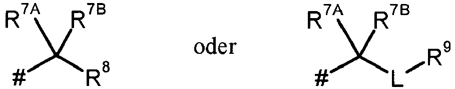 steht, wobei
# für die Anknüpfstelle an das Stickstoffatom steht,
L für Methylen steht,
R^{7A} für Wasserstoff oder Methyl steht,
R^{7B} für Wasserstoff oder Methyl steht,
oder
R^{7B} und R^{7B} zusammen mit dem Kohlenstoffatom, an das sie gebunden sind, einen Cyclopropyl-Ring bilden,
R⁸ für Hydroxycarbonyl, Methoxycarbonyl, Ethoxycarbonyl oder Aminocarbonyl steht,
R⁹ für Hydroxy, Methoxy, Hydroxycarbonyl, Methoxycarbonyl, Ethoxycarbonyl oder Aminocarbonyl steht,
sowie ihre Salze, Solvate und Solvate der Salze.

Bevorzugt sind im Rahmen der vorliegenden Erfindung auch Verbindungen der Formel (I), in welcher
- R⁴: für eine Gruppe der Formel 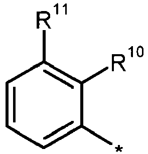 steht, wobei
* für die Anknüpfstelle an die Gruppe -C(R⁵)(AC(=O)NHR³)- steht,
R¹⁰ für Wasserstoff, Trifluormethoxy, Chlor, Trifluormethyl und Methoxy steht,
R¹¹ für Wasserstoff, Trifluormethoxy, Fluor, Chlor, Trifluormethyl und Methoxy steht, wobei mindestens einer der Reste R¹⁰ und R¹¹ von Wasserstoff verschieden ist,
sowie ihre Salze, Solvate und Solvate der Salze.

Bevorzugt sind im Rahmen der vorliegenden Erfindung auch folgende Verbindungen der Formel (I):
Ethyl-1-({[({3-(4-chlorphenyl)-5-oxo-4-[(*2S*)-3,3,3-trifluor-2-hydroxypropyl]-4,5-dihydro-1H-1,2,4-triazol-1-yl}acetyl)amino][3-(trifluormethyl)phenyl]acetyl}amino)cyclopropancarboxylat *(Diastereomerengemisch)*
1-({[({3-(4-Chlorphenyl)-5-oxo-4-[(2*S*)-3,3,3-trifluor-2-hydroxypropyl]-4,5-dihydro-1H-1,2,4-triazol-1-yl}acetyl)amino][3-(trifluormethyl)phenyl]acetyl}amino)cyclopropancarbonsäure *(Diastereomerengemisch)*
1-({[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino][3-(trifluormethyl)phenyl]acetyl}amino)cyclopropancarbonsäure *(Diastereomer 2)*
Methyl-N-{[({3-(4-chlorphenyl)-5-oxo-4-[(*2S*)-3,3,3-trifluor-2-hydroxypropyl]-4,5-dihydro-1H-1,2,4-triazol-1-yl} acetyl)amino] [3-(trifluormethyl)phenyl]acetyl} -beta-alaninat *(Diastereomerengemisch)*
N-{[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino][3-(trifluormethyl)phenyl]acetyl}-beta-alanin *(Diastereomerengemisch)*
N³-{[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino][3-(trifluormethyl)phenyl]acetyl}-beta-alaninamid *(Diastereomer 2)*
N³-{(*2R*)-2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-2-[3-(trifluormethyl)phenyl]propanoyl}-beta-alaninamid
Methyl-N-{(*2R*)-2-[(-{3-(4-chlorphenyl)-5-oxo-4-[(*2S*)-3,3,3-trifluor-2-hydroxypropyl]-4,5-dihydro-1H-1,2,4-triazol-1-yl}acetyl)amino]-2-[3-(trifluormethyl)phenyl]propanoyl}-2-methylalaninat
N-{(*2R*)-2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-2-[3-(trifluormethyl)phenyl]propanoyl}-2-methylalanin
N²-{(*2R*)-2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-2-[3-(trifluormethyl)phenyl]propanoyl}-2-methylalaninamid
Methyl-N-{[({3-(4-chlorphenyl)-5-oxo-4-[(*2S*)-3,3,3-trifluor-2-hydroxypropyl]-4,5-dihydro-1H-1,2,4-triazol-1-yl}}acetyl)amino][3-(trifluormethyl)phenyl]acetyl}-2-methylalaninat *(Diastereomerengemisch)*
N-{[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino][3-(trifluormethyl)phenyl]acetyl}-2-methylalanin *(Diastereomerengemisch)*
N-{[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino][3-(trifluormethyl)phenyl]acetyl}-2-methylalanin *(Diastereomer 2)*
N²-{[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino][3-(trifluormethyl)phenyl]acetyl}-2-methylalaninamid *(Diastereomerengemisch)*
N²-{[({3-(4-Chlorphenyl)-5-oxo-4-[(2*S*)-3,3,3-trifluor-2-hydroxypropyl]-4,5-dihydro-1H-1,2,4-triazol-1-yl}acetyl)amino][3-(trifluormethyl)phenyl]acetyl}-2-methylalaninamid *(Diastereomer 2)*
Methyl-N-{(*3S*)-3-[({3-(4-chlorphenyl)-5-oxo-4-[(*2S*)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalaninat
N-{(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalanin
N²-{(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalaninamid
2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(1-hydroxy-2-methylpropan-2-yl)-2-[3-(trifluormethyl)phenyl]acetamid *(Diastereomer 1)*
2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-2-[2-(trifluormethyl)phenyl]acetamid *(Diastereomerengemisch)*
2-[({3-(4-Chlorphenyl)-5-oxo-4-[(*2S*)-3,3,3-trofluor-2-hydroxypropyl]-4,5-dihydro-1H-1,2,4-triazol-1-yl}acetyl)amino]-N-(2-hydroxyethyl)-2-[2-(trifluormethyl)phenyl]acetamid *(Diastereomer 2)*
2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-2-[3-(trifluormethyl)phenyl]acetamid *(Diastereomerengemisch)*
2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-2-[3-(trifluormethyl)phenyl]acetamid *(Diastereomer 2)*
2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-2-[3-(trifluormethoxy)phenyl]acetamid *(Diastereomerengemisch)*
(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-3-[2-(trifluormethyl)phenyl]propanamid
(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-3-[3-(trifluormethyl)phenyl]propanamid
(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-methoxyethyl)-3-[3-(trifluormethyl)phenyl]propanamid
2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-[2-(trifluormethoxy)ethyl]-2-[3-(trifluormethyl)phenyl]acetamid *(Diastereomer 2).*

Besonders bevorzugt sind im Rahmen der vorliegenden Erfindung auch folgende Verbindungen der Formel (I):
N³-{[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino][3-(trifluormethyl)phenyl]acetyl}-beta-alaninamid *(Diastereomer 2)*
N³-{(*2R*)-2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-2-[3-(trifluormethyl)phenyl]propanoyl}-beta-alaninamid
Methyl-N-{(*2R*)-2-[({3-(4-chlorphenyl)-5-oxo-4-[(*2S*)-3,3,3-trifluor-2-hydroxypropyl]-4,5-dihydro-1H-1,2,4-triazol-1-yl}acetyl)amino]-2-[3-(trifluormethyl)phenyl]propanoyl}-2-methylalaninat
N-{(*2R*)-2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-2-[3-(trifluormethyl)phenyl]propanoyl}-2-methylalanin
N²-{(*2R*)-2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-2-[3-(trifluormethyl)phenyl]propanoyl}-2-methylalaninamid
N-{[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino][3-(trifluormethyl)phenyl]acetyl}-2-methylalanin *(Diastereomerengemisch)*
N-{[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino][3-(trifluormethyl)phenyl]acetyl}-2-methylalanin *(Diastereomer 2)*
N²-{[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino][3-(trifluormethyl)phenyl]acetyl}-2-methylalaninamid *(Diastereomer 2)*
N-{(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalanin
2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(1-hydroxy-2-methylpropan-2-yl)-2-[3-(trifluormethyl)phenyl]acetamid *(Diastereomer 1)*
2-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-2-[3-(trifluormethyl)phenyl]acetamid *(Diastereomerengemisch)*
(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-3-[3-(trifluormethyl)phenyl]propanamid
(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-methoxyethyl)-3-[3-(trifluormethyl)phenyl]propanamid.

Bevorzugt sind im Rahmen der vorliegenden Erfindung auch folgende Verbindungen der Formel (I):
Methyl-N-{(*3S*)-3-[({3-(4-chlorphenyl)-5-oxo-4-[(*2S*)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalaninat
N-{(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalanin
N²-{(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalaninamid
(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-3-[2-(trifluormethyl)phenyl]propanamid
(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-3-[3-(trifluormethyl)phenyl]propanamid
(*3S*)-3-({[3-(4-Chlorphenyl)-4-cyclopropyl-5-oxo-4,5-dihydro-1H-1,2,4-triazol-1-yl]acetyl}amino)-N-(2-hydroxyethyl)-3-[2-(trifluormethyl)phenyl]propanamid
(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-methoxyethyl)-3-[3-(trifluormethyl)phenyl]propanamid

Besonders bevorzugt sind im Rahmen der vorliegenden Erfindung auch folgende Verbindungen der Formel (I):
N-{(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalanin
N²-{(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalaninamid
(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-3-[2-(trifluormethyl)phenyl]propanamid
(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-hydroxyethyl)-3-[3-(trifluormethyl)phenyl]propanamid
(*3S*)-3-[({3-(4-Chlorphenyl)-5-oxo-4-[(*2S*)-3,3,3-trifluor-2-hydroxypropyl]-4,5-dihydro-1H-1,2,4-triazol-1-yl}acetyl)amino]-N-(2-methoxyethyl)-3 -[3 -(trifluormethyl)phenyl] propanamid

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II) 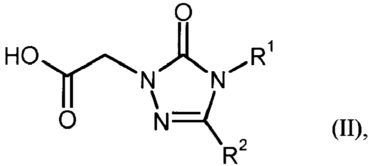 in welcher R¹ und R² jeweils die oben angegebenen Bedeutungen haben,
   in einem inerten Lösungsmittel unter Aktivierung der Carbonsäure-Funktion mit einer Verbindung der Formel (III) 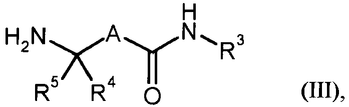 in welcher A, R³, R⁴ und R⁵ jeweils die oben angegebenen Bedeutungen haben,
   kuppelt,
   oder
[B] eine Verbindung der Formel (IV) 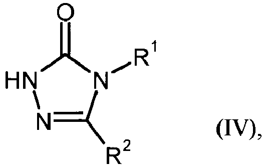 in welcher R¹ und R² jeweils die oben angegebenen Bedeutungen haben,
   in einem inerten Lösungsmittel in Gegenwart einer Base mit einer Verbindung der Formel (V) 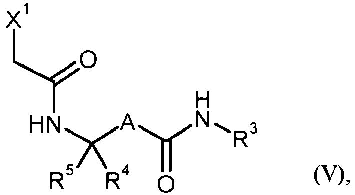 in welcher A, R³, R⁴ und R⁵ jeweils die oben angegebenen Bedeutungen haben
   und
   X¹ für eine Abgangsgruppe, wie beispielsweise Halogen, Mesylat oder Tosylat, steht,
   umsetzt,
   oder
[C] eine Verbindung der Formel (VI) 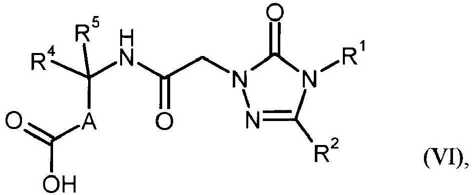 in welcher A, R¹, R², R⁴ und R⁵ jeweils die oben angegebenen Bedeutungen haben,
   in einem inerten Lösungsmittel unter Aktivierung der Carbonsäure-Funktion mit einer Verbindung der Formel (VII)

   H₂N-R³ (VII),

   in welcher R³ die oben angegebene Bedeutung hat,
   kuppelt,
und die resultierenden Verbindungen der Formel (I) gegebenenfalls mit den entsprechenden (i) Lösungsmitteln und/oder (ii) Basen oder Säuren in ihre Solvate, Salze und/oder Solvate der Salze überführt.

Inerte Lösungsmittel für die Verfahrensschritte (II) + (III) → (I) und (VI) + (VII) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N,N*-Dimethylformamid, *N,N'-*Dimethylpropylenharnstoff (DMPU) oder *N*-Methylpyrrolidon (NMP). Ebenso ist es möglich, Gemische der genannten Lösungsmittel zu verwenden. Bevorzugt sind Acetonitril, Dichlormethan, Tetrahydrofuran, Dimethylformamid oder Gemische dieser Lösungsmittel.

Als Kondensationsmittel für die Amidbildung in den Verfahrensschritten (II) + (III) → (I) und (VI) + (VII) → (I) eignen sich beispielsweise Carbodiimide wie *N,N'*-Diethyl-, *N,N'*-Dipropyl-, *N,N'*-Diisopropyl-, *N,N'*-Dicyclohexylcarbodiimid (DCC) oder *N*-(3-Dimethylaminoisopropyl)-*N*'-ethylcarbodiimid-Hydrochlorid (EDC), Phosgen-Derivate wie *N,N'*-Carbonyldiimidazol (CDI), 1,2-Oxazoliumverbindungen wie 2-Ethyl-5-phenyl-1,2-oxazolium-3-sulfat oder 2-tert..-Butyl-5-methyl-isoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat, Benzotriazol-1-yloxy-tris(pyrrolidino)phosphonium-hexafluorophosphat (PyBOP), *O*-(Benzotriazol-1-yl)-*N,N,N',N'*-tetramethyluronium-tetrafluoroborat (TBTU), *O*-(Benzotriazol-1-yl)-*N,N,N',N*'-tetramethyluronium-hexafluorophosphat (HBTU), 2-(2-Oxo-1-(2*H*)-pyridyl)-1,1,3,3-tetramethyluronium-tetrafluoroborat (TPTU), O-(7-Azabenzotriazol-1-y\)-*N,N,N',N'*-tetramethyluronium-hexafluorophosphat (HATU) oder O-(1*H*-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methylpiperidin oder *N,N-*Diisopropylethylamin.

Bevorzugt wird EDC in Kombination mit HOBt oder TBTU in Gegenwart von *N,N*-Diisopropylethylamin verwendet.

Die Kondensationen (II) + (III) → (I) und (VI) + (VII) → (I) werden im Allgemeinen in einem Temperaturbereich von -20°C bis +60°C, bevorzugt bei 0°C bis +40°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (IV) + (V) → (I) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Methylethylketon, Essigsäureethylester, Acetonitril, *N,N*-Dimethylformamid, Dimethylsulfoxid, *N,N'*-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Acetonitril, Aceton oder Dimethylformamid verwendet.

Als Basen für den Verfahrensschritt (IV) + (V) → (I) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie Triethylamin, *N*-Methylmorpholin, *N*-Methylpiperidin, *N,N*-Diisopropylethylamin, Pyridin,1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Kalium- oder Cäsiumcarbonat verwendet.

Die Base wird hierbei in einer Menge von 1 bis 5 Mol, bevorzugt in einer Menge von 1 bis 2.5 Mol, bezogen auf 1 Mol der Verbindung der Formel (IV), eingesetzt. Die Reaktion erfolgt im Allgemeinen in einem Temperaturbereich von 0°C bis +100°C, bevorzugt bei +20°C bis +80°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Die Herstellung der erfindungsgemäßen Verbindungen kann durch das folgende Syntheseschema veranschaulicht werden:

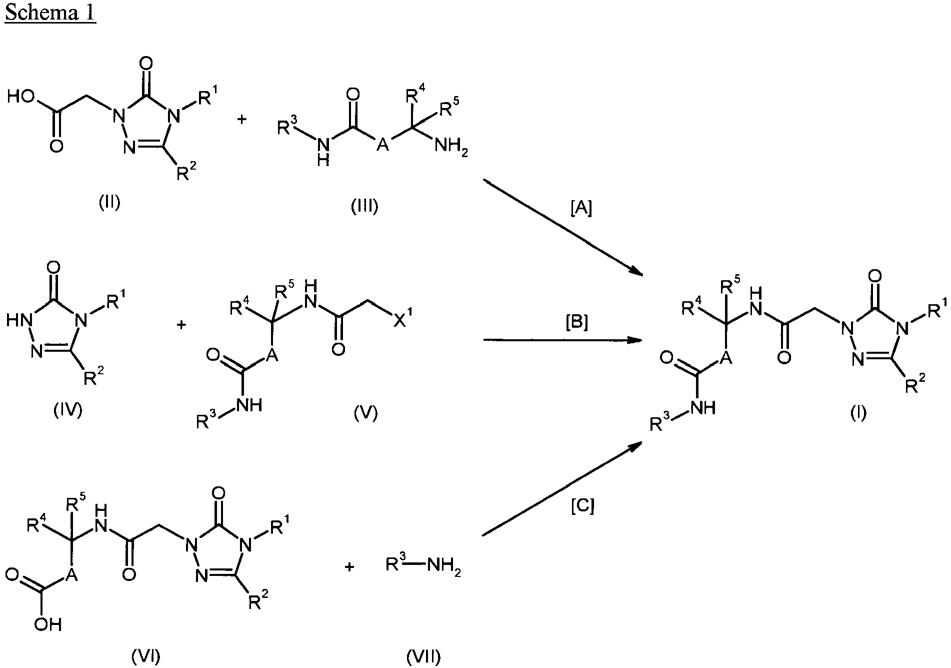

Die Verbindungen der Formel (II), (IV) und (VI) sind literaturbekannt (siehe z. B. WO 2007/134862), können in Analogie zu literaturbekannten Verfahren, oder wie im vorliegenden experimentellen Teil beschrieben, hergestellt werden.

Die Verbindungen der Formeln (III), (V) und (VII) sind kommerziell erhältlich, literaturbekannt, oder können in Analogie zu literaturbekannten Verfahren, oder wie im vorliegenden Experimentellen Teil beschrieben, hergestellt werden.

Weitere erfindungsgemäße Verbindungen können gegebenenfalls auch hergestellt werden durch Umwandlungen von funktionellen Gruppen einzelner Substituenten, insbesondere den unter R³ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, insbesondere Bildung von Carbonsäureamiden, sowie Einführung und Entfernung temporärer Schutzgruppen.

Die erfindungsgemäßen Verbindungen besitzen wertvolle pharmakologische Eigenschaften und können zur Vorbeugung und/oder Behandlung von verschiedenen Erkrankungen und krankheitsbedingten Zuständen bei Menschen und Tieren verwendet werden.

Bei den erfindungsgemäßen Verbindungen handelt es sich um potente, selektive duale V1a/V2-Rezeptor-Antagonisten, die die Vasopressin-Aktivität *in vitro* und *in vivo* inhibieren. Weiterhin weisen die erfindugsgemäßen Verbindungen eine erhöhte Löslichkeit in wässrigen Medien auf.

Die erfindungsgemäßen Verbindungen sind daher insbesondere für die Prophylaxe und/oder Behandlung von kardiovaskulären Erkrankungen geeignet. In diesem Zusammenhang seien als Zielindikationen beispielhaft und vorzugsweise genannt: akute und chronische Herzinsuffizienz, arterielle Hypertonie, koronare Herzerkrankung, stabile und instabile Angina pectoris, myokardiale Ischämie, Myokardinfarkt, Schock, Arteriosklerose, atriale und ventrikuläre Arrhythmien, transitorische und ischämische Attacken, Hirnschlag, entzündliche kardiovaskuläre Erkrankungen, periphere und kardiale Gefäßerkrankungen, periphere Durchblutungsstörungen, arterielle pulmonale Hypertonie, Spasmen der Koronararterien und peripherer Arterien, Thrombosen, thromboembolische Erkrankungen, Ödembildung wie zum Beispiel pulmonales Ödem, Hirnödem, renales Ödem oder Herzinsuffizienz-bedingtes Ödem, sowie Restenosen wie nach Thrombolysetherapien, percutan-transluminalen Angioplastien (PTA), transluminalen Koronarangioplastien (PTCA), Herztransplantationen und Bypass-Operationen.

Im Sinne der vorliegenden Erfindung umfasst der Begriff Herzinsuffizienz auch spezifischere oder verwandte Krankheitsformen wie Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Weiterhin eignen sich die erfindungsgemäßen Verbindungen zur Verwendung als Diuretikum für die Behandlung von Ödemen und bei Elektrolytstörungen, insbesondere bei der hypervolämischen und euvolämischen Hyponaträmie.

Die erfindungsgemäßen Verbindungen sind weiter geeignet für die Prophylaxe und/oder Behandlung der polyzystischen Nierenkrankheit (PCKD) und des Syndroms der inadäquaten ADH-Sekretion (SIADH).

Außerdem können die erfindungsgemäßen Verbindungen für die Prophylaxe und/oder Behandlung der Leberzirrhose, des Aszites, von Diabetes mellitus und diabetischen Folgeerkrankungen, wie z.B. Neuropathie und Nephropathie, von akutem und chronischem Nierenversagen sowie von chronischer Niereninsuffizienz verwendet werden.

Ferner eignen sich die erfindungsgemäßen Verbindungen für die Prophylaxe und/oder Behandlung zentralnervöser Störungen wie Angstzuständen und Depressionen, von Glaukom sowie von Krebs, insbesondere von Lungentumoren.

Darüber hinaus können die erfindungsgemäßen Verbindungen für die Prophylaxe und/oder Behandlung von entzündlichen Erkrankungen, asthmatischen Erkrankungen, chronisch-obstruktiven Atemwegserkrankungen (COPD), Schmerzzuständen, Prostatahypertrophien, Inkontinenz, Blasenentzündung, hyperaktiver Blase, Erkrankungen der Nebenniere wie zum Beispiel Phäochromozytom und Nebennierenapoplexie, Erkrankungen des Darms wie zum Beispiel Morbus Crohn und Diarrhoe, oder von Menstruationsstörungen wie zum Beispiel Dysmenorrhöen oder von Endometriose eingesetzt werden.

Weiterer Gegenstand der vorliegenden Erfindung sind die erfindungsgemäßen Verbindungen zur Verwendung in einem Verfahren zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, die mindestens eine der erfindungsgemäßen Verbindungen sowie einen oder mehrere weitere Wirkstoffe enthalten, insbesondere zur Behandlung und/oder Prophylaxe der zuvor genannten Erkrankungen. Als hierfür geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Diuretika, insbesondere Schleifendiuretika sowie Thiazide und Thiazid-ähnliche Diuretika;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Calcium-Sensitizer, wie beispielhaft und vorzugsweise Levosimendan;
- NO- und Häm-unabhängige Aktivatoren der Guanylatcyclase, wie insbesondere Cinaciguat sowie die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der Guanylatcyclase, wie insbesondere Riociguat sowie die in WO 00/06568, WO 00/06569, WO 02/42301 und WO 03/095451 beschriebenen Verbindungen;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib;
- den Energiestoffwechsel des Herzens beeinflussende Verbindungen, wie beispielhaft und vorzugsweise Etomoxir, Dichloracetat, Ranolazine oder Trimetazidine;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten und Rho-Kinase-Inhibitoren; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugs-weise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Vasopeptidase-Inhibitor bzw. Inhibitor der neutralen Endopeptidase (NEP), verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon, Canrenon oder Kalium-Canrenoat, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 10 mg/kg, vorzugsweise etwa 0.01 bis 1 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- BOC: *tert*.-Butoxycarbonyl
- CI: chemische Ionisation (bei MS)
- DCI: direkte chemische Ionisation (bei MS)
- DME: 1,2-Dimethoxyethan
- DMF: Dimethylformamid
- DMSO: Dimethylsulfoxid
- d. Th.: der Theorie (bei Ausbeute)
- EDC: *N*'-(3-Dimethylaminopropyl)-*N*-ethylcarbodiimid-Hydrochlorid
- eq.: Äquivalent(e)
- ESI: Elektrospray-Ionisation (bei MS)
- GC/MS: Gaschromatographie-gekoppelte Massenspektrometrie
- ges.: gesättigt
- h: Stunde(n)
- HOBt: 1-Hydroxy-1*H*-benzotriazol-Hydrat
- HPLC: Hochdruck-, Hochleistungsflüssigchromatographie
- HV: Hochvakuum
- konz.: konzentriert
- LC/MS: Flüssigchromatographie-gekoppelte Massenspektrometrie
- LDA: Lithiumdiisopropylamid
- LiHMDS: Lithiumhexamethyldisilazan
- min: Minute(n)
- MS: Massenspektrometrie
- MTBE: Methyl-tert.-butylether
- NMR: Kernresonanzspektrometrie
- rac: racemisch / Racemat
- R_{f}: Retentionsfaktor (bei Dünnschichtchromatographie auf Kieselgel)
- RT: Raumtemperatur
- Rₜ: Retentionszeit (bei HPLC)
- THF: Tetrahydrofuran
- TMOF: Trimethylorthoformiat
- UV: Ultraviolett-Spektrometrie
- v/v: Volumen zu Volumen-Verhältnis (einer Lösung)

### LC/MS-, HPLC- und GC/MS-Methoden:

Methode 1: Gerätetyp MS: Micromass ZQ; Gerätetyp HPLC: Waters Alliance 2795; Säule: Phenomenex Synergi 2.5 µ MAX-RP 100A Mercury 20 mm x 4mm; Eluent A: 11 Wasser + 0.5 ml 50%ige Ameisensäure, Eluent B: 11 Acetonitril + 0.5 ml 50%ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.
Methode 2: Gerätetyp MS: Waters (Micromass) Quattro Micro; Gerätetyp HPLC: Agilent 1100 Serie; Säule : Thermo Hypersil GOLD 3 µ 20 x 4 mm; Eluent A: 11 Wasser + 0.5 ml 50%ige Ameisensäure, Eluent B: 11 Acetonitril + 0.5 ml 50%ige Ameisensäure; Gradient: 0.0 min 100% A → 3.0 min 10% A → 4.0 min 10% A → 4.01 min 100% A (Flow 2.5 ml) → 5.00 min 100% A; Ofen: 50°C; Fluss: 2 ml/min; UV-Detektion: 210 nm.
Methode 3: Instrument: Micromass Quattro Premier mit Waters UPLC Acquity; Säule: Thermo Hypersil GOLD 1.9 µ 50 x 1 mm; Eluent A: 11 Wasser + 0.5 ml 50%ige Ameisensäure, Eluent B: 11 Acetonitril + 0.5 ml 50%ige Ameisensäure; Gradient: 0.0 min 90% A → 0.1 min 90% A → 1.5 min 10% A → 2.2 min 10% A Ofen: 50°C; Fluss: 0.33 ml/min; UV-Detektion: 210 nm.
Methode 4: Gerätetyp MS: Micromass ZQ; Gerätetyp HPLC: HP 1100 Series; UV DAD; Säule: Phenomenex Gemini 3 µ 30 mm x 3.00 mm; Eluent A: 11 Wasser + 0.5 ml 50%ige Ameisensäure, Eluent B: 11 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min. 2 ml/min; Ofen: 50°C; UV-Detektion: 210 nm.
Methode 5: Instrument: Waters ACQUITY SQD UPLC System; Säule: Waters Acquity UPLC HSS T3 1.8 µ 50 x 1 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50 °C; Fluss: 0.40 ml/min; UV-Detektion: 210 - 400 nm.
Methode 6 (präparative HPLC): Säule: Grom-Sil 120 ODS-4HE, 10 µm, SNr. 3331, 250 mm x 30 mm. Eluent A: Ameisensäure 0.1% in Wasser, Eluent B: Acetonitril; Fluss: 50 ml/min Programm: 0-6 min: 10% B; 6-27 min: Gradient bis 95% B; 27-38 min: 95% B; 38 - 45 min: 10% B.
Methode 7 (präparative HPLC): Säule: Reprosil C18, 10 µm, 250 mm x 30 mm. Eluent A: Ameisensäure 0.1 % in Wasser, Eluent B: Methanol; Fluss: 50 ml/min. Programm: 0 bis 4.25 min: 60%A /40% B; 4.25 bis 4.50 min: Gradient bis 60% B; 4.50 min bis 17 min Gradient bis 100%B; 17 min bis 19.50 min 100%B; 19.50 min bis 19.75 min Gradient bis 40 %B; 19.75 bis 22 min (Ende): 60%A/ 40%B.
Methode 8 (präparative HPLC): Säule: Reprosil C18, 10 µm, 250 mm x 30 mm. Eluent A: Ameisensäure 0.1% in Wasser, Eluent B: Acetonitril; Fluss: 50 ml/min. Programm: 0 bis 6 min: 90%A /10% B; 6 min bis 27 min: Gradient bis 95% B; 27 min bis 38 min 95%B; 38 min bis 39 min Gradient bis 10 %B; 39 min bis 43 min (Ende): 60%A/ 40%B.
Methode 9 (präparative HPLC): Säule: Grom-Sil 120 ODS-4HE, 10 µm, SNr. 3331, 250 mm x 30 mm. Eluent A: Ameisensäure 0.1% in Wasser, Eluent B: Acetonitril; Fluss: 50 ml/min Programm: 0-6 min: 5% B; 6-34 min: Gradient bis 95% B; 34-38 min: 95% B; 38-45 min: 5% B.
Methode 10 (chirale präparative HPLC): Chirale stationäre Kieselgel-Phase basierend auf dem Selektor Poly(*N*-methacryloyl-D-Leucin-dicyclopropylmethylamid); Säule: 670 mm x 40 mm; Elutionsmittel: 100 % Essigsäureethylester; Fluss: 80 ml/min, Temperatur: 24 °C; UV-Detektor 260 nM.
Methode 11 (chirale analytische HPLC): Chirale stationäre Kieselgel-Phase basierend auf dem Selektor Poly(*N*-methacryloyl-D-Leucin-dicyclopropylmethylamid); Säule: 250 mm x 4.6 mm, Elutionsmittel Essigsäureethylester 100%, Fluss: 2 ml/min, Temperatur: 24 °C; UV-Detektor 265 nM.
Methode 12 (chirale präparative HPLC): Chirale stationäre Vinyl-Kieselgel-Phase basierend auf dem Selektor Poly(*N*-methacryloyl-L-Leucin-*tert*-butylamid) ; Säule: 670 mm x 40 mm ; Elutionsmittel: iso-Hexan / Essigsäureethylester 2:1.; Fluss: 80 ml/min., Temperatur: 24 °C; UV-Detektor 265 nM.
Methode 13 (chirale analytische HPLC): Chirale stationäre Vinyl-Kieselgel-Phase basierend auf dem Selektor Poly(*N*-methacryloyl-D-Leucin-*tert*-butylamid); Säule: 250 mm x 4.6 mm, Elutionsmittel: iso-Hexan / Essigsäureethylester 2:1.; Fluss 1 ml/min., Temperatur 24°C; UV-Detector 265 nM.
Methode 14 (chirale präparative HPLC): Stationäre Phase Daicel Chiralpak AD-H, 10 µm, Säule: 250 mm x 20 mm; Temperatur: RT; UV-Detektion: 230 nm; Fluss: 20 ml/min.
   Methode 14a: Elutionsmittel: *iso*-Hexan / Isopropanol 70: 30 (v/v).
   Methode 14b: Elutionsmittel: *iso*-Hexan / Isopropanol 50: 50 (v/v).
Methode 15 (chirale analytische HPLC): Stationäre Phase Daicel Chiralpak AD-H, 5 µm, Säule: 250 mm x 4.6 mm; Temperatur: 30°C; UV-Detektion: 230 nm; Fluss: 1.0 ml/min. Verschiedene Elutionsmittel:
   Methode 15a: Elutionsmittel: *iso*-Hexan / *iso*-Propanol 70 : 30 (v/v).
   Methode 15b: Elutionsmittel: *iso*-Hexan / Isopropanol 50: 50 (v/v).

### Ausgangsverbindungen und Intermediate:

### Beispiel 1A

### Ethyl-N-({2-[(4-chlorphenyl)carbonyl]hydrazinyl}carbonyl)glycinat

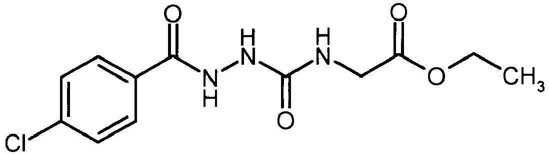

Eine Suspension von 12.95 g (75.9 mmol) 4-Chlorbenzhydrazid in 50 ml trockenem THF wurde bei 50 °C vorgelegt und tropfenweise mit einer Lösung von 10.0 g (77.5 mmol) Ethyl-2-isocyanatoacetat in 100 ml trockenem THF versetzt. Zunächst bildete sich eine Lösung, dann fiel ein Niederschlag aus. Nach Ende der Zugabe wurde die Mischung noch 2 h auf 50°C weiter gerührt, dann über Nacht bei RT stehen lassen. Die Kristalle wurden durch Filtration isoliert, mit wenig Diethylether gewaschen und am HV getrocknet. Es wurden 21.43 g (89% d. Th) der Titelverbindung erhalten.

LC/MS [Methode 1]: Rₜ = 1.13 min; m/z = 300 (M+H)⁺

¹H NMR (DMSO-d₆) δ = 10.29 (s, 1H), 8.21□□, 400MHz): δ = (s, 1H), 7.91 (d, 2H), 7.57 (d, 2H), 6.88 (br.s, 1H), 4.09 (q, 2H), 3.77 (d, 2H), 1.19 (t, 3H)

### Beispiel 2A

### [3-(4-Chlorphenyl)-5-oxo-1,5-dihydro-4H-1,2,4-triazol-4-yl]essigsäure

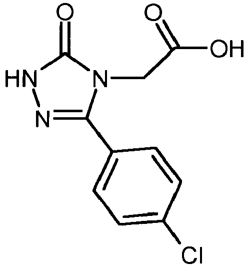

21.43 g (67.93 mmol) der Verbindung aus Beispiel 1A wurden mit 91 ml einer 3N Natronlauge versetzt und über Nacht zum Rückfluss erhitzt. Nach Abkühlen auf RT wurde die Mischung durch langsame Zugabe ca. 20%-iger Salzsäure auf pH 1 eingestellt. Der ausgefallene Feststoff wurde durch Filtration isoliert, mit Wasser gewaschen und bei 60°C in Vakuum getrocknet. Ausbeute: 17.55 g (90% d. Th., ca. 88%ige Reinheit).

LC/MS [Methode 1]: Rₜ = 0.94 min; m/z = 254 (M+H)⁺

¹H NMR (DMSO-d₆, 400MHz): δ = 13.25 (br.s, 1H), 12.09 (s, 1H), 7.65 - 7.56 (m, 4H), 4.45 (s, 2H).

### Beispiel 3A

### 5-(4-Chlorphenyl)-4-(3,3,3-trifluor-2-oxopropyl)-2,4-dihydro-3H-1,2,4-triazol-3-on (oder als Hydrat: 5-(4-Chlorphenyl)-4-(3,3,3-trifluor-2,2-dihydroxypropyl)-2,4-dihydro-3H-1,2,4-triazol-3-on)

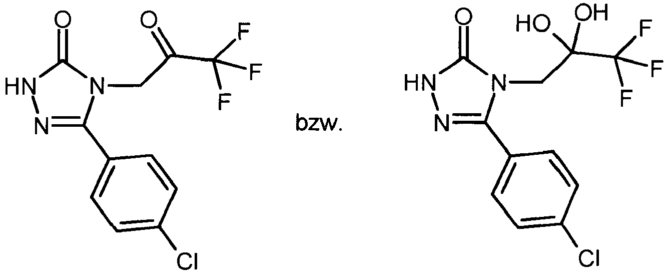

5 g (16.36 mmol) der Verbindung aus Beispiel 2A wurden unter Argon in 200 ml Pyridin gelöst, dann mit 17.18 g (81.8 mmol) Trifluoressigsäureanhydrid versetzt. Dabei stieg die Temperatur auf ca. 35°C. Nach 30 min wurde das Pyridin am Rotationsverdampfer entfernt und der Rückstand mit 1.5 L 0.5N Salzsäure verdünnt. Diese Mischung wurde auf 70°C erwärmt dann warm filtriert. Der Feststoff wurde mit etwas Wasser gewaschen. Das gesamte Filtrat wurde dreimal mit Essigsäureethylester extrahiert. Die vereinigten organischen Phasen wurden mit Wasser, dann einer gesättigten wässrigen Natriumhydrogencarbonat-Lösung, dann mit einer gesättigten wässrigen Natriumchloridlösung gewaschen, über Natriumsulfat getrocknet und am Rotationsverdampfer vom Lösungsmittel befreit. Der Rückstand wurde am HV getrocknet. Ausbeute: 3.56 g (68% d. Th.) der Titelverbindung als Hydrat.

LC/MS [Methode 1]: Rₜ = 1.51 min; m/z = 306 (M+H)⁺ und 324 (M+H)⁺(Keton bzw. Hydrat)

¹H NMR (DMSO-d₆, 400MHz): δ = □□12.44 (s, 1H), 7.72 (d, 2H), 7.68 (br.s, 2H), 7.61 (d, 2H), 3.98 (s, 2H).

### Beispiel 4A

### 5-(4-Chlorphenyl)-4-(3,3,3-trifluor-2-hydroxypropyl)-2,4-dihydro-3H-1,2,4-triazol-3-on

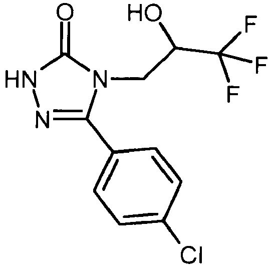

3.56 g (11 mmol) der Verbindung aus Beispiel 3A wurden in 100 ml Methanol gelöst und unter Eiskühlung mit 3.75 g (99 mmol) Natriumborhydrid versetzt (Gasentwicklung). Nach 1.5 h wurden langsam 200 ml 1M Salzsäure zugegeben. Das Methanol wurde am Rotationsverdampfer entfernt, der Rückstand mit 500 ml Wasser verdünnt und dreimal mit Essigsäureethylester extrahiert. Die vereinigten organischen Phasen wurden mit einer gesättigten wässrigen Natriumhydrogencarbonat-Lösung, dann mit einer gesättigten wässrigen Natriumchloridlösung gewaschen, über Natriumsulfat getrocknet und am Rotationsverdampfer vom Lösungsmittel befreit. Der Rückstand wurde am HV getrocknet. 3.04 g (90% d. Th.) der Titelverbindung wurden erhalten.

LC/MS [Methode 2]: Rₜ = 1.80 min; m/z = 308 (M+H)⁺.

¹H NMR (DMSO-d₆, 400MHz): δ = 12.11 (s, 1H), 7.75□□, (d, 2H), 7.62 (d, 2H), 6.85 (d, 1H), 4.34 - 4.23 (m, 1H), 3.92 (dd, 1H), 3.77 (dd, 1H).

### Beispiel 5A

### Methyl-[3-(4-chlorphenyl)-5-oxo-4-(3,3,3-trifluor-2-hydroxypropyl)-4,5-dihydro-1H-1,2,4-triazol-1-yl]acetat

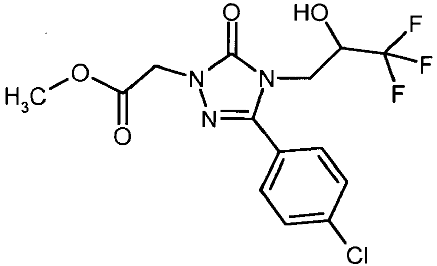

3.04 g (9.9 mmol) der Verbindung aus Beispiel 4A wurden in 100 ml Acetonitril gelöst und mit 1.07 g (9.9 mmol) Chloressigsäuremethylester, 2.73 g (19.8 mmol) Kaliumcarbonat und einer kleinen Spatelspitze Kaliumiodid versetzt. Die Reaktionsmischung wurde auf Rückfluss für 1 h erhitzt, auf RT abkühlen lassen und filtriert. Das Filtrat wurde am Rotationsverdampfer von den flüchtigen Komponenten befreit und der Rückstand am HV getrocknet. Ausbeute: 3.70 g (89% d. Th.) der Titelverbindung in ca. 90%iger Reinheit.

LC/MS [Methode 3]: Rₜ = 1.10 min; m/z = 380 (M+H)⁺.

¹H NMR (DMSO-d₆, 400MHz): δ = □□7.78 (d, 2H), 7.64 (d, 2H), 6.91 (d, 1H), 4.72 (s, 2H), 4.16 - 4.35 (m, 1H), 3.99 (dd, 1H), 3.84 (dd, 1H), 3.70 (s, 3H).

Die racemische Verbindung aus Beispiel 5A wurde, wie in WO 2007/134862 beschrieben, durch präparative HPLC an einer chiralen Phase in die Enantiomere Beispiel 6A und Beispiel 7A getrennt.

Säule: Chirale Kieselgel-Phase basierend auf dem Selektor Poly(*N*-methacryloyl-L-isoleucin-3-pentylamid, 430 mm x 40 mm; Eluent: Stufengradient *iso*-Hexan/Essigsäureethylester 1:1 (v/v) → Essigsäureethylester → *iso*-Hexan/Essigsäureethylester 1:1 (v/v); Fluss: 50 ml/min; Temperatur: 24°C; UV-Detektion: 260 nm.

Man erhielt auf diese Weise aus 3.6 g racemischer Verbindung aus Beispiel 5A (in 27 ml Essigsäureethylester und 27 ml I *iso*-Hexan gelöst und in drei Portionen durch die Säule getrennt) 1.6 g des zuerst eluierenden Enantiomers 1 (Beispiel 6A) sowie 1.6 g des später eluierenden Enantiomers 2 (Beispiel 7A).

### Beispiel 6A

### {3-(4-Chlorphenyl)-5-oxo-4-[(2S)-3,3,3-trifluor-2-hydroxypropyl]-4,5-dihydro-1H-1,2,4-triazol-1-yl}-essigsäuremethylester) (Enantiomer 1)

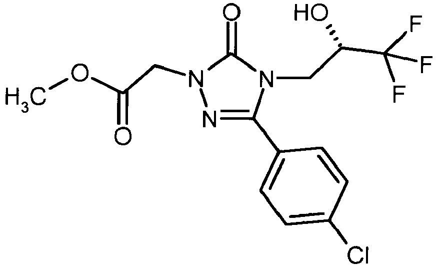

Zuerst eluierendes Enantiomer aus der Racemat-Trennung von Beispiel 5A.

Rₜ = 3.21 min [Säule: Chirale Kieselgel-Phase basierend auf dem Selektor Poly(*N*-methacryloyl-L-isoleucin-3-pentylamid, 250 mm x 4.6 mm; Eluent: *iso*-Hexan/Essigsäureethylester 1:1; Fluss: 1 ml/min; UV-Detektion: 260 nm].

### Beispiel 7A

### {3-(4-Chlorphenyl)-5-oxo-4-[(2R)-3,3,3-trifluor-2-hydroxypropyl]-4,5-dihydro-1H-1,2,4-triazol-1-yl}-essigsäuremethylester (Enantiomer 2)

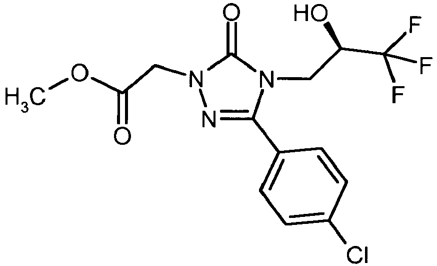

Zuletzt eluierendes Enantiomer aus der Racemat-Trennung von Beispiel 5A.

Rₜ = 4.48 min [Säule: Chirale Kieselgel-Phase basierend auf dem Selektor Poly(*N*-methacryloyl-L-isoleucin-3-pentylamid, 250 mm x 4.6 mm; Eluent: *iso*-Hexan/Essigsäureethylester 1:1; Fluss: 1 ml/min; UV-Detektion: 260 nm].

### Beispiel 8A

### {3-(4-Chlorphenyl)-5-oxo-4-[(2S)-3,3,3-trifluor-2-hydroxypropyl]-4,5-dihydro-1H-1,2,4-triazol-1-yl}-essigsäure

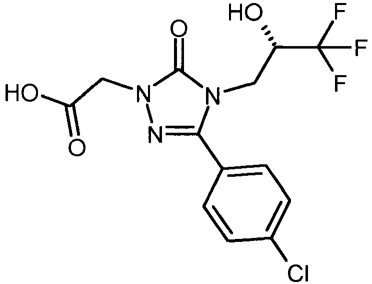

Der enantiomerenreine Ester aus Beispiel 6A (1.6g, 4.21 mmol) wurde in 77 ml Methanol gelöst und mit 17 ml einer 1M Lösung von Lithiumhydroxid in Wasser versetzt. Die Mischung wurde 1 h bei RT gerührt und dann am Rotationsverdampfer eingeengt. Der Rückstand wurde mit 100 ml Wasser verdünnt und mit 1 N Salzsäure auf pH 1 - 2 angesäuert. Das ausgefallene Produkt wurde abfiltriert, mit Wasser und Cyclohexan nacheinander gewaschen und trocken gesaugt. Nach weiterer Trocknung am HV wurde die Titelverbindung (1.1 g, 71% d. Th.) erhalten.

[α]_{D}²⁰ = +3.4° (Methanol, c = 0.37 g/100 ml)

LC/MS [Methode 1]: R, = 1.51 min; m/z = 366 (M+H)⁺

¹H-NMR (400 MHz, DMSO-d₆): δ = 3.84 (dd, 1H), 4.00 (dd, 1H), 4.25 (m, 1H), 4.58 (s, 2H), 6.91 (d, 1H), 7.63 (d, 2H), 7.78 (d, 2H), 13.20 (br. s, 1H).

### Beispiel 9A

### {3-(4-Chlorphenyl)-5-oxo-4-[(2R)-3,3,3-trifluor-2-hydroxypropyl]-4,5-dihydro-1H-1,2,4-triazol-1-yl}-essigsäure

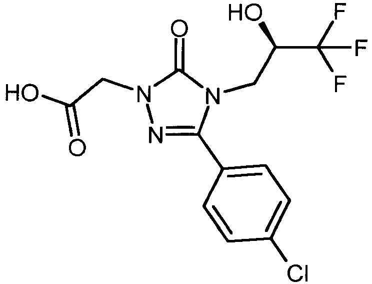

Analog zu Beispiel 8A wurde aus Beispiel 7A die Titelverbindung erhalten.

[α]_{D}²⁰ = -4.6° (Methanol, c = 0.44 g/100 ml)

LC/MS [Methode 1]: Rₜ = 1.53 min; m/z = 366 (M+H)⁺

¹H-NMR (400 MHz, DMSO-d₆): δ = 3.84 (dd, 1H), 4.00 (dd, 1H), 4.25 (m, 1H), 4.58 (s, 2H), 6.91 (d, 1H), 7.63 (d, 2H), 7.78 (d, 2H), 13.20 (br. s, 1H).

### Beispiel 10A

### Methyl-{3-(4-chlorphenyl)-5-oxo-4-[(1E)-3,3,3-trifluorprop-1-en-1-yl]-4,5-dihydro-1H-1,2,4-triazol-1-yl}acetat

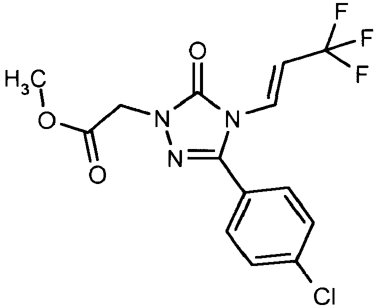

280 mg (0.74 mmol) der Verbindung aus Beispiel 7A wurden bei RT zusammen mit 108 mg (0.89 mmol) 4-Dimethylaminopyridin in 5.3 ml Pyridin vorgelegt, portionsweise mit 0.31 ml Trifluormethansulfonsäureanhydrid (1.84 mmol) versetzt und 12 h gerührt. Das Pyridin wurde am Rotationsverdampfer entfernt. Der Rückstand wurde in Acetonitril und 1N Salzsäure aufgenommen und über die präparative HLPC [Methode 9] gereinigt. Erhalten wurden 230 mg (86 % d. Th.) der Titelverbindung.

LC/MS [Methode 4]: Rₜ = 1.14 min; m/z = 362 (M+H)⁺

¹H-NMR (400MHz, DMSO-d₆): δ [ppm]= 3.72 (s, 3H), 4.78 (s, 2H), 6.85 (dd, 1H), 7.18 (d, 1H), 7.68 (s, 4H).

### Beispiel 11A

### {3-(4-Chlorphenyl)-5-oxo-4-[(1E)-3,3,3-trifluorprop-1-en-1-yl]-4,5-dihydro-1H-1,2,4-triazol-1-yl}essigsäure

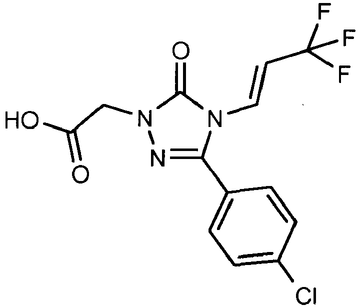

260 mg (0.72 mmol) der Verbindung aus Beispiel 10A wurden in 5 ml Methanol gelöst und mit 2.87 ml (2.87 mmol) einer 1M wässrigen Lithiumhydroxidlösung versetzt. Die Mischung wurde 1 h bei RT gerührt, dann mit 1N Salzsäure angesäuert und mit DMSO verdünnt. Die gesamte Lösung wurde per präparativer HLPC [Methode 9] gereinigt. Erhalten wurden 215 mg (86 % d. Th.) der Titelverbindung.

LC/MS [Methode 4]: Rₜ = 1.03 min.; m/z = 348 (M+H)⁺

¹H-NMR (400MHz, DMSO-d₆): δ [ppm]= 4.64 (s, 2H), 6.79 - 6.92 (m, 1H), 7.19 (dd, 1H), 7.68 (s, 4H), 13.31 (br. s, 1H).

### Beispiel 12A

### 2-[(5-Chlor-2-thienyl)carbonyl]-N-(2-methoxyethyl)hydrazincarboxamid

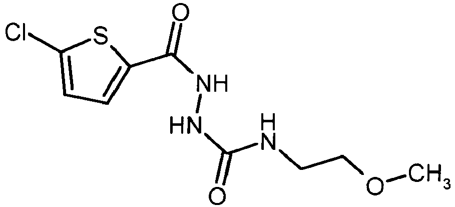

3.1 g (17.55 mmol) 5-Chlorthiophen-2-carbohydrazid wurden in 30 ml trockenem THF bei 50°C fein suspendiert. Anschließend wurden 1.81 g (17.90 mmol) 1-Isocyanato-2-methoxyethan in 30ml THF gelöst zugetropft. Es wurde 2.5 h bei 50°C gerührt. Nach dem Abkühlen auf RT wurde das Lösungsmittel am Rotationsverdampfer entfernt und der Rückstand mit Diethylether versetzt. Die Kristalle wurden abgesaugt, mit Diethylether nachgewaschen und im Hochvakuum getrocknet. Es wurden 4.87 g (100 % d. Th.) der Titelverbindung erhalten.

¹H-NMR (400MHz, DMSO-d₆): δ = 3.14 - 3.21 (m, 2H), 3.28 - 3.36 (m, 5H), 6.52 (br. s, 1H), 7.22 (d, 1H), 7.70 (d, 1H), 7.97 (s, 1H), 10.24 (s, 1H).

### Beispiel 13A

### 5-(5-Chlor-2-thienyl)-4-(2-methoxyethyl)-2,4-dihydro-3H-1,2,4-triazol-3-on

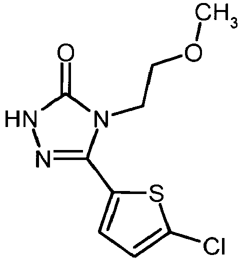

4.85 g (17.46) mmol der Verbindung aus Beispiel 12A wurden in 17 ml (52.39 mmol) 3M Natronlauge gelöst und 168 h unter Rückfluss erhitzt. Dabei wurden nach 16, 40, 64 und 88 h je 1.05 g (26.19 mmol, insgesamt 104.76 mmol) festes Natriumhydroxid zugegeben. Der Ansatz wurde mit 1M Salzsäure auf pH 10 gestellt, und das Gemisch zweimal mit je 30 ml Essigsäureethylester extrahiert. Die vereinigten organischen Phasen wurden über Natriumsulfat getrocknet, filtriert und am Rotationsverdampfer vom Lösemittel befreit und im Hochvakuum getrocknet. Es wurden 2.44 g (54 % d. Th.) der Titelverbindung erhalten.

¹H-NMR (400MHz, DMSO-d₆): δ = 3.20 (s, 3H), 3.53 (t, 2H), 3.92 (t, 2H), 7.24 (d, 1H), 7.51 (d, 1H), 12.04 (s, 1H).

### Beispiel 14A

### Ethyl-[3-(5-chlor-2-thienyl)-4-(2-methoxyethyl)-5-oxo-4,5-dihydro-1H-1,2,4-triazol-1-yl]acetat

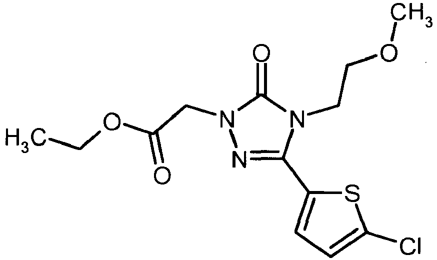

2.4 g (9.24 mmol) der Verbindung aus Beispiel 13A wurden mit 2.55 g (18.48 mmol) Kaliumcarbonat in 48 ml Acetonitril suspendiert. Anschließend wurde mit 1.08 ml (10.17 mmol) Chloressigsäureethylester versetzt und 4.5 h bei 80°C unter Rückfluss erhitzt. Es wurden nochmals 113 mg (0.92 mmol) Chloressigsäureethylester zugegeben und 2 h bei 80°C gerührt. Die Suspension wurde über eine Schicht Kieselgel filtriert, mit Essigsäureethylester nachgewaschen, am Rotationsverdampfer eingedampft und am HV getrocknet. Erhalten wurden 3.24 g (100 % d. Th.) der Titelverbindung.

LC/MS [Methode 22]: Rₜ = 2.42 min; m/z = 346 (M+H)⁺.

¹H-NMR (400MHz, DMSO-d₆): δ = 1.21 (t, 3H), 3.30 (s, 3H), 3.55 (t, 2H), 3.99 (t, 2H), 4.15 (q, 2H), 4.65 (s, 2H), 7.27 (d, 1H), 7.58 (d, 1H).

### Beispiel 15A

### [3-(5-Chlor-2-thienyl)-4-(2-methoxyethyl)-5-oxo-4,5-dihydro-1H-1,2,4-triazol-1-yl]essigsäure

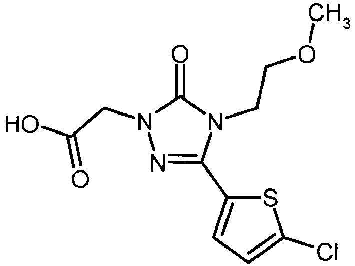

3.2 g (9.25 mmol) der Verbindung aus Beispiel 14A wurden in 28 ml Methanol gelöst. Anschließend wurden 2.82 ml 20%ige wässrige Kaliumhydroxidlösung zugegeben. Es wurde 2h bei RT gerührt. Der Methanolanteil wurde am Rotationsverdampfer auf die Hälfte reduziert. Anschließend wurde mit Wasser verdünnt und einmal mit 15 ml Essigsäureethylester extrahiert. Die wässrige Phase wurde mit 920 µl konzentrierter Salzsäure angesäuert und zweimal mit je 15 ml Essigsäureethylester extrahiert. Die vereinigten organischen Phasen wurden über Natriumsulfat getrocknet, filtriert und am Rotationsverdampfer eingeengt. Nach Trocknen im Hochvakuum erhielt man 2.34 g (80 % d. Th.) der Titelverbindung.

LC/MS [Methode 22]: Rₜ = 2.05 min; m/z = 318 (M+H)⁺.

¹H-NMR (400MHz, DMSO-d₆): δ = 3.20 (s, 3H), 3.55 (t, 2H), 3.99 (t, 2H), 4.53 (s, 2H), 7.27 (d, 1H), 7.58 (d, 1H), 13.14 (br. s, 1H).

### Beispiel 16A

### (2R)-2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propansäure

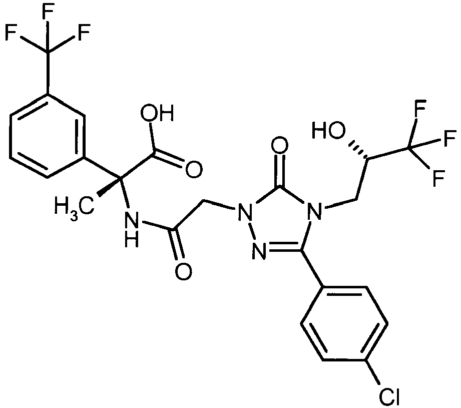

250 mg der Verbindung aus Beispiel 8A (0.68 mmol) und 92 mg (0.68 mmol) HOBt wurden in 5 ml DMF vorgelegt und mit 131 mg (0.68 mmol) EDC versetzt. Die Mischung wurde 20 min bei RT gerührt dann auf eine Lösung von 221 mg (0.82 mmol) (2R)-2-Amino-2-[3-(trifluormethyl)phenyl]propionsäure-Hydrochlorid und 119 µl (0.68 mmol) N,N-Diisopropylethylamin in 2 ml DMF zugetropft. Die Reaktionsmischung wurde 20 min bei RT gerührt, dann mit 1 ml 1N Salzsäure versetzt und per präparativer HPLC (Methode 10) gereinigt. Die geeignete Fraktion wurde am Rotationsverdampfer eingeengt und der Rückstand am HV getrocknet. Es wurden 260 mg (65% d. Th.) der Titelverbindung erhalten.

LC-MS [Methode 3]: Rₜ = 1.23 min; MS [ESIpos]: m/z = 581 (M+H)⁺

¹H-NMR (400MHz, DMSO-d₆): δ [ppm]= 1.85 (s, 3H), 3.76 - 3.88 (m, 1H), 3.90 - 4.01 (m, 1H), 4.26 (br. s., 1H), 4.51 - 4.67 (m, 2H), 6.92 (d, 1H), 7.55 - 7.71 (m, 4H), 7.71 - 7.83 (m, 4H), 8.80 (s, 1H), 13.10 (s, 1H).

### Beispiel 17A

### Ethyl-1-({[(tert.-butoxycarbonyl)amino][3-(trifluormethyl)phenyl]acetyl} amino)cyclopropan-carboxylat

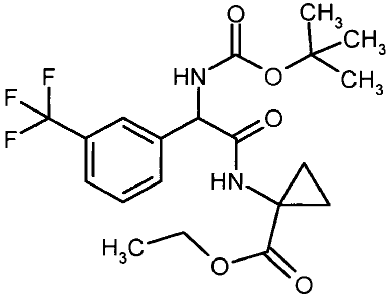

Eine Mischung aus 500 mg (1.57 mmol) [(*tert*.-Butoxycarbonyl)amino][3-(trifluormethyl)phenyl]essigsäure, 243 mg (1.88 mmol) 1-Aminocyclopropancarbonsäure Ethylester, 450 mg (2.35 mmol) EDC und 317 mg (2.35 mmol) HOBt in 10 ml DMF wurde über Nacht bei RT gerührt. Die Reaktionsmischung wurde mit Essigsäureethylester verdünnt und zweimal mit 1N Salzsäure und einmal mit einer gesättigten wässrigen Natriumhydrogencarbonatlösung gewaschen. Die organische Phase wurde über Natriumsulfat getrocknet dann am Rotationsverdampfer vom Lösungsmittel befreit. Der Rückstand wurde im Hochvakuum getrocknet. Man erhielt 650 mg (96% d. Th.) der Titelverbindung.

LC-MS [Methode 1]: Rₜ = 2.11 min; MS [ESIpos]: m/z = 431 (M+H)⁺

¹H-NMR (400MHz, DMSO-d₆): δ [ppm]= 0.92 (t, 3H), 0.95 - 1.04 (m, 2H), 1.24 - 1.33 (m, 2H), 1.37 (s, 9H), 3.89 (q, 2H), 5.23 (d, 1H), 7.50 (br. d, 1H), 7.59 (t, 1H), 7.66 (d, 1H), 7.73 (d, 1H), 7.80 (s, 1H), 8.91 (s, 1H).

### Beispiel 18A

### Methyl-N-{[(tert.-butoxycarbonyl)amino][3-(trifluormethyl)phenyl]acetyl} -2-methylalaninat

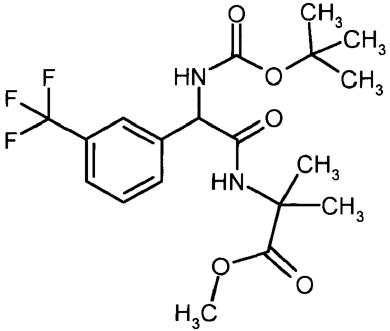

Eine Mischung aus 500 mg (1.57 mmol) [(*tert*.-Butoxycarbonyl)amino][3-(trifluormethyl)phenyl]essigsäure und 317 mg (2.35 mmol) HOBt in 10 ml DMF wurde mit 450 mg (2.35 mmol) EDC versetzt und 20 min bei RT gerührt. 313 mg (2.04 mmol) Methyl-2-methylalaninat-Hydrochlorid und 382 µl (2.19 mmol) N,N'-Diisopropylethylamin wurden zugegeben und die Mischung über Nacht gerührt. Zur Reinigung wurde die gesamte Reaktionsmischung per präparativer HPLC [Methode 6] getrennt. Die geeignete Fraktion wurde am Rotationsverdampfer eingeengt und der Rückstand im Hochvakuum getrocknet. Man erhielt 502 mg (77% d. Th.) der Titelverbindung.

LC-MS [Methode 3]: Rₜ = 1.28 min; MS [ESIpos]: m/z = 319 (M+H-BOC)⁺; [ESIneg]: m/z = 417 (M-H)⁻.

¹H-NMR (400MHz, DMSO-d₆): δ [ppm]= 1.26 (s, 3H), 1.38 (d, 12H), 3.44 (s, 3H), 5.31 (d, 1H), 7.45 (br. d, 1H), 7.56 - 7.62 (m, 1H), 7.63 - 7.71 (m, 2H), 7.75 (br. s, 1H), 8.61 - 8.69 (m, 1H).

### Beispiel 19A

### Methyl-N-{[(tert.-butoxycarbonyl)amino][3-(trifluormethyl)phenyl]acetyl}-beta-alaninat

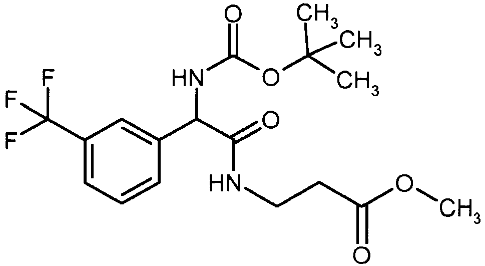

Analog zu Beispiel 18A wurden aus 250 mg (0.78 mmol) [(*tert*.-Butoxycarbonyl)amino][3-(trifluormethyl)phenyl]essigsäure und 142 mg (1.02 mmol) Methyl-beta-alaninat-Hydrochlorid 256 mg (81% d. Th.) der Titelverbindung erhalten.

LC-MS [Methode 1]: Rₜ = 1.90 min; MS [ESIpos]: m/z = 405(M+H)⁺.

¹H-NMR (400MHz, DMSO-d₆): δ [ppm]= 1.37 (s, 9H), 2.42 (t, 2H), 3.20- 3.33 (m, 2H), 3.51 (s, 3H), 5.23 (d, 1H), 7.51 (br. d, 1H), 7.54 - 7.60 (m, 1H), 7.61 - 7.71 (m, 2H), 7.76 (br. s, 1H), 8.36 (t, 1H).

### Beispiel 20A

### tert.-Butyl-{2-[(3-amino-3-oxopropyl)amino]-2-oxo-1-[3-(trifluormethyl)phenyl]ethyl} carbamat (Racemat)

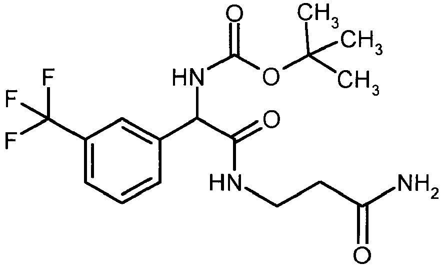

Eine Mischung aus 400 mg (1.25 mmol) [(*tert.*-Butoxycarbonyl)amino][3-(trifluormethyl)-phenyl]essigsäure und 254 mg (1.88 mmol) HOBt in 12 ml DMF wurde mit 360 mg (1.88 mmol) EDC versetzt und 30 min bei RT gerührt. 234 mg (1.88 mmol) Beta-alaninamid-Hydrochlorid und 436 µl (2.50 mmol) N,N'-Diisopropylethylamin wurden zugegeben und die Mischung 1h nachgerührt. Sie wurde mit Essigsäureethylester verdünnt und viermal mit Wasser gewaschen. Die organische Phase wurde über Natriumsulfat getrocknet und am Rotationsverdampfer vom Lösungsmittel befreit. Der Rückstand wurde im Hochvakuum getrocknet. Es wurden 446 mg (91 % d. Th.) der Titelverbindung erhalten.

LC-MS [Methode 1]: Rₜ = 1.56 min; MS [ESIpos]: m/z = 390 (M+H)⁺.

¹H-NMR (400MHz, DMSO-d₆): δ [ppm]= 1.37 (s, 9H), 2.11 - 2.26 (m, 2H), 3.15 - 3.28 (m, 2H), 5.25 (d, 1H), 6.81 (br. s., 1H), 7.29 (br. s., 1H), 7.50 (d, 1H), 7.53 - 7.60 (m, 1H), 7.64 (d, 1H), 7.69 (d, 1H), 7.77 (s, 1H), 8.30 (t, 1H).

Durch Chromatographie an einer chiralen Phase [Methode 14a] wurde die Titelverbindung in ihren Enantiomere getrennt: siehe Beispiele 21A und 22A.

### Beispiel 21A

### tert.-Butyl-{2-[(3-amino-3-oxopropyl)amino]-2-oxo-1-[3-(trifluormethyl)phenyl]ethyl}carbamat (Enantiomer 1)

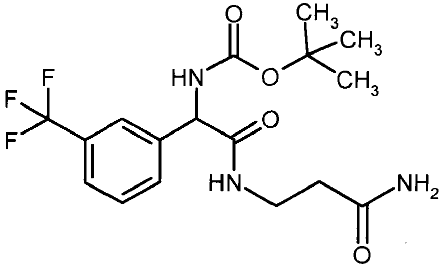

Zuerst eluierendes Enantiomer (205 mg) aus der chromatographischen Enantiomerentrennung der Verbindung aus Beispiel 20A nach Methode 14a.

Chirale analytische HPLC [Methode 15b]: Rₜ = 3.29 min.

### Beispiel 22A

### tert.-Butyl-{2-[(3-amino-3-oxopropyl)amino]-2-oxo-1-[3-(trifluormethyl)phenyl]ethyl}carbamat (Enantiomer 2)

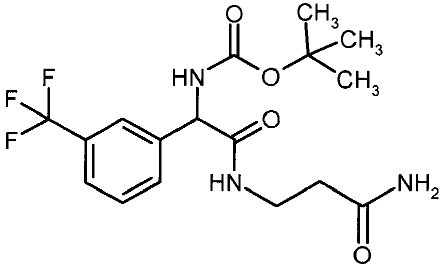

Zuletzt eluierendes Enantiomer (208 mg) aus der chromatographischen Enantiomerentrennung der Verbindung aus Beispiel 20A nach Methode 14a.

Chirale analytische HPLC [Methode 15b]: Rₜ = 4.15 min.

### Beispiel 23A

### tert. -Butyl-{2-[(1-amino-2-methyl-1-oxopropan-2-yl)amino] -2-oxo-1-[3-(trifluor-methyl)phenyl]ethyl} -carbamat

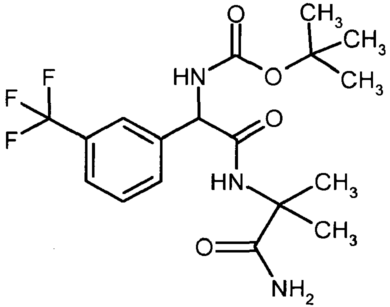

Analog zu Beispiel 18A wurden aus 222 mg (0.69 mmol) [(*tert.*-Butoxycarbonyl)amino][3-(trifluormethyl)-phenyl]essigsäure und 115 mg (0.77 mmol) 2-Methylalaninamid-Hydrochlorid 237 mg (85%d. Th.) der Titelverbindung erhalten.

LC-MS [Methode 4]: Rₜ = 2.04 min; MS [ESIpos]: m/z = 404 (M+H)⁺.

¹H-NMR (400MHz, DMSO-d₆): δ [ppm]= 1.30 (s, 3H), 1.33 (s, 3H), 1.38 (s, 9H), 5.23 (d, 1H), 6.96 (br. s, 1H), 6.99 (br. s, 1H), 7.50 - 7.61 (m, 2H), 7.64 (d, 1H), 7.69 (d, 1H), 7.77 (s, 1H), 8.28 (br. s., 1H).

### Beispiel 24A

### tert. -Butyl-{2-[(1-amino-2-methyl-1-oxopropan-2-yl)amino]-2-oxo-1-[2-(trifluor-methyl)phenyl] ethyl}carbamat

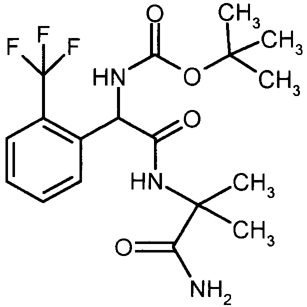

Analog zu Beispiel 18A wurden aus 250 mg (0.78 mmol) [(*tert*.-Butoxycarbonyl)amino][2-(trifluormethyl)-phenyl]essigsäure und 119 mg (0.86 mmol) 2-Methylalaninamid-Hydrochlorid 220 mg (70%d. Th.) der Titelverbindung erhalten.

LC-MS [Methode 3]: Rₜ = 1.07 min; MS [ESIpos]: m/z = 404 (M+H)⁺.

¹H-NMR (400MHz, DMSO-d₆): δ [ppm]= 1.38 (br. s., 9H), 1.39 (br. s., 3H), 1.41 (s, 3H), 5.36 (d, 1H), 7.12 (s, 1H), 7.22 (br. s., 1H), 7.50 (t, 1H), 7.57 (d, 1H), 7.63 - 7.73 (m, 2H), 7.90 (d, 1H), 8.04 (s, 1H).

### Beispiel 25A

### tert.-Butyl-{2-[(1-amino-2-methyl-1-oxopropan-2-yl)amino]-1-(2-chlorphenyl)-2-oxoethyl}carbamat

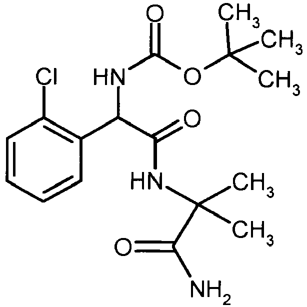

Analog zu Beispiel 18A wurden aus 250 mg (0.88 mmol) [(*tert*.-Butoxycarbonyl)amino](2-chlorophenyl)essigsäure und 133 mg (0.96 mmol) 2-Methylalaninamid-Hydrochlorid 263 mg (81%d. Th.) der Titelverbindung erhalten.

LC-MS [Methode 5]: Rₜ = 0.81 min; MS [ESIpos]: m/z = 370 (M+H)⁺.

¹H-NMR (400MHz, DMSO-d₆): δ [ppm]= 1.38 (br. s., 3H), 1.39 (br. s., 9H), 1.41 (s, 3H), 5.40 (d, 1H), 7.08 (br. s., 1H), 7.11 (br. s., 1H), 7.29 - 7.35 (m, 2H), 7.37 - 7.48 (m, 2H), 7.69 (d, 1H), 8.06 (s, 1H).

### Beispiel 26A

### [(tert. -Butoxycarbonyl)amino] (2,3 -dichlorphenyl)essigsäure

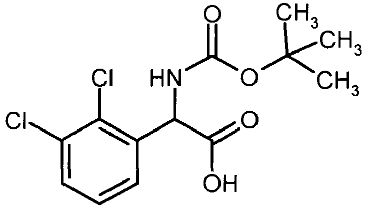

Eine Lösung von 500 mg (2.27 mmol) Amino-(2,3-dichlorphenyl)essigsäure in 25ml 5-%iger wässriger Natriumhydrogencarbonatlösung wurde mit 25 ml Dioxan verdünnt und mit 532 ml (2.32 mmol) Di-tert.-butyldicarbonat versetzt. Die Reaktionsmischung wurde über Nacht bei RT gerührt. Zur Aufarbeitung wurden 150 ml 1N Salzsäure hinzugefügt und das Produkt dreimal mit Essigsäureethylester ausextrahiert. Die vereinigten organischen Phasen wurden über Natriumsulfat getrocknet und am Rotationsverdampfer vom Lösungsmittel befreit. Der Rückstand wurde im Hochvakuum getrocknet. Man erhielt 750 mg (quant. Ausbeute) der Titelverbindung.

LC-MS [Methode 5]: Rₜ = 1.00 min; MS [ESIpos]: m/z = 320 (M+H)⁺.

¹H-NMR (400MHz, DMSO-d₆): δ [ppm]= 1.38 (s, 9H), 5.62 (d, 1H), 7.35 - 7.45 (m, 2H), 7.61 (dd, 1H), 7.83 (d, 1H), 13.12 (br. s., 1H).

### Beispiel 27A

### tert.-Butyl-{2-[(1-amino-2-methyl-1-oxopropan-2-yl)amino]-1-(2,3-dichlorphenyl)-2-oxoethyl} carbamat

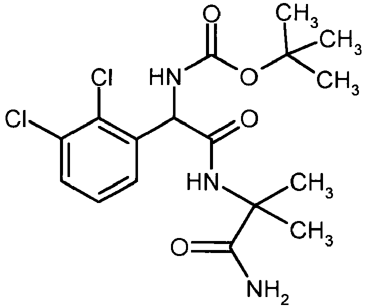

Analog zu Beispiel 18A wurden aus 250 mg (0.78 mmol) der Verbindung aus Beispiel 26A und 119 mg (0.86 mmol) 2-Methylalaninamid-Hydrochlorid 250 mg (77%d. Th.) der Titelverbindung erhalten.

LC-MS [Methode 3]: Rₜ = 1.11 min; MS [ESIpos]: m/z = 404 (M+H)⁺.

¹H-NMR (400MHz, DMSO-d₆): δ [ppm]= 1.34 - 1.43 (m, 15H), 5.45 (d, 1H), 7.05 (br. s., 1H), 7.10 (br. s., 1H), 7.32 - 7.39 (m, 2H), 7.56 - 7.61 (m, 1H), 7.81 (d, 1H), 8.15 (s, 1H).

### Beispiel 28A

### Methyl-N-{(3S)-3-[(tert.-butoxycarbonyl)amino]-3-[3-(trifluormethyl)phenyl]propanoyl}-2-methylalaninat

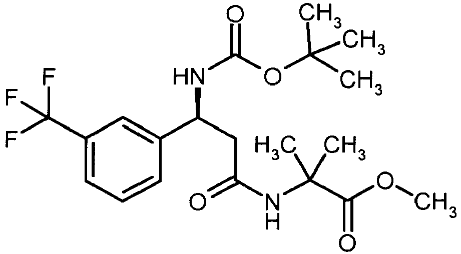

Analog zu Beispiel 18A wurden aus 125 mg (0.38 mmol) (3*S*)-3-[(*tert*.-Butoxycarbonyl)aminol-3-[3-(trifluormethyl)phenyl]propansäure und 86 mg (0.56 mmol) Methyl-2-methylalaninat-Hydrochlorid 130 mg (80% d. Th.) der Titelverbindung erhalten.

LC-MS [Methode 3]: Rₜ = 1.26 min; MS [ESIpos]: m/z = 433 (M+H)⁺.

### Beispiel 29A

### tert.-Butyl-{ 2-[(1-hydroxy-2-methylpropan-2-yl)amino]-2-oxo-1-[3-(trifluormethyl)phenyl]ethyl}-carbamat (Racemat)

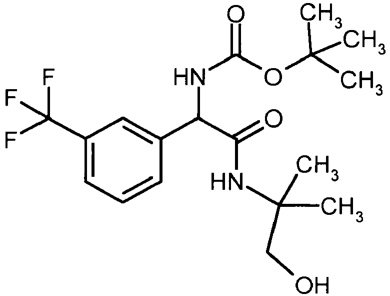

Eine Mischung aus 400 mg (1.25 mmol) *[(tert.* -Butoxycarbonyl)amino] [3-(trifluormethyl)phenyl]essigsäure und 254 mg (1.88 mmol) HOBt in 10 ml DMF wurde mit 360 mg (1.88 mmol) EDC versetzt und 20 min bei RT gerührt. Die entstandene Lösung wurde zu einer Lösung von 168 mg (1.88 mmol) 2-Amino-2-methyl-1-propanol in 2 ml DMF zugetropft. Die Reaktionsmischung wurde bei RT 20 min gerührt. Zur Reinigung wurde 1 ml 1N Salzsäure hinzugefügt und die gesamte Reaktionsmischung über präparative HPLC [Methode 6] getrennt. Die geeignete Fraktion wurde am Rotationsverdampfer eingeengt und der Rückstand im Hochvakuum getrocknet. Man erhielt 364 mg (74% d. Th.) der Titelverbindung.

LC-MS [Methode 3]: Rₜ = 1.21 min; MS [ESIpos]: m/z = 391 (M+H)⁺

¹H-NMR(400MHz, DMSO-d₆): δ [ppm]= 1.13 (s, 3H), 1.14 (s, 3H), 1.38 (s, 9H), 3.26 - 3.32 (m, 1H), 3.41 (dd, 1H), 4.79 (t, 1H), 5.29 (d, 1H), 7.35 (d, 1H), 7.57 (t, 1H), 7.63 (d, 1H), 7.67 - 7.79 (m, 3H).

Durch Chromatographie an einer chiralen Phase [Methode 12] wurde die Titelverbindung in ihre Enantiomere getrennt: siehe Beispiele 30A und 31A.

### Beispiel 30A

### tert. -Butyl-{2-[(-hydroxy-2-methylpropan-2-yl)amino]-2-oxo-1-[3-(trifluormethyl)phenyl] ethyl}-carbamat (Enantiomer 1)

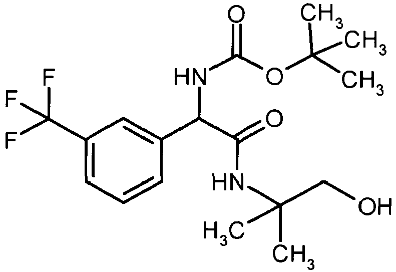

Zuerst eluierendes Enantiomer aus der chromatographischen Enantiomerentrennung der Verbindung aus Beispiel 29A nach Methode 12.

Chirale analytische HPLC [Methode 13]: Rₜ = 5.61 min.

### Beispiel 31A

### tert.-Butyl-{2-[(1-hydroxy-2-methylpropan-2-yl)amino]-2-oxo-1-[3-(trifluormethyl)phenyl]ethyl}-carbamat (Enantiomer 2)

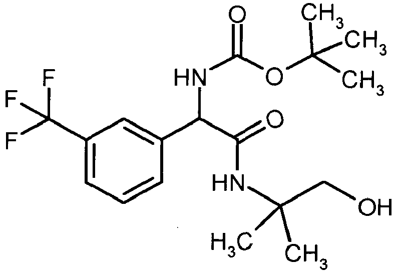

Zuletzt eluierendes Enantiomer aus der chromatographischen Enantiomerentrennung der Verbindung aus Beispiel 29A nach Methode 12.

Chirale analytische HPLC [Methode 13]: Rₜ = 4.85 min.

### Beispiel 32A

### tert.-Butyl-{1-(2-chlorphenyl)-2-[(2-methoxyethyl)amino]-2-oxoethyl}carbamat

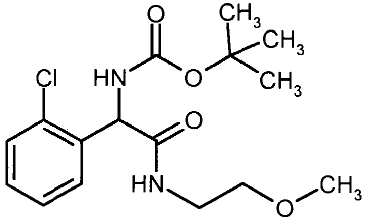

Eine Mischung aus 250 mg (0.88 mmol) [(*tert*.-Butoxycarbonyl)amino](2-chlorophenyl)essigsäure, 177 mg (1.31 mmol) HOBt, 252 mg (1.31 mmol) EDC und 72 mg (0.96 mmol) 2-Methoxyethanamin in 6.3 ml DMF wurde 2 h bei RT gerührt. Zur Reinigung wurde 1 ml 1N Salzsäure zugegeben und die gesamte Reaktionsmischung über präparative HPLC [Methode 6] getrennt. Die geeignete Fraktion wurde am Rotationsverdampfer eingeengt und der Rückstand im Hochvakuum getrocknet. Man erhielt 269 mg (90% d. Th.) der Titelverbindung.

LC-MS [Methode 5]: Rₜ = 0.88 min; MS [ESIpos]: m/z = 343 (M+H)⁺

¹H-NMR (400MHz, DMSO-d₆): δ [ppm]= 1.38 (s, 9H), 3.18 - 3.29 (m, 2H), 3.22 (s, 3H), 3.29 - 3.37 (m, 2H), 5.45 (d, 1H), 7.28 - 7.34 (m, 2H), 7.35 - 7.46 (m, 2H), 7.49 (d, 1H), 8.06 (br. t, 1H).

### Beispiel 33A

### tert.-Butyl-{2-[(2-hydroxyethyl)amino]-2-oxo-1-[2-(trifluormethyl)phenyl] ethyl}carbamat

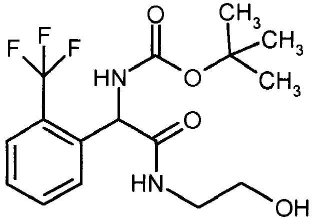

Analog zu Beispiel 18A, aber ohne N,N'-Diisopropylethylamin, wurden aus 500 mg (1.57 mmol) [(*tert*.-Butoxycarbonyl)amino][2-(trifluormethyl)phenyl]essigsäure und 287 mg (4.70 mmol) 2-Ethanolamin 540 mg (95% d. Th.) der Titelverbindung erhalten.

LC-MS [Methode 4]: Rₜ = 2.04 min; MS [ESIpos]: m/z = 363 (M+H)⁺

¹H-NMR (400MHz, DMSO-d₆): δ [ppm]= 1.37 (br. s., 9H), 3.08 - 3.22 (m, 2H), 3.35 - 3.46 (m, 2H), 4.65 (t, 1H), 5.43 (d, 1H), 7.50 (t, 1H), 7.58 (d, 1H), 7.62 - 7.73 (m, 3H), 7.81 - 7.91 (m, 1H).

### Beispiel 34A

### tert.-Butyl-{2-[(2-methoxyethyl)amino]-2-oxo-1-[2-(trifluormethyl)phenyl]ethyl}carbamat

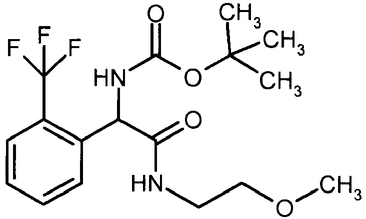

Analog zu Beispiel 18A, aber ohne N,N'-Diisopropylethylamin, wurden aus 500 mg (1.57 mmol) [(*tert*.-Butoxycarbonyl)amino][2-(trifluormethyl)phenyl]essigsäure und 176 mg (2.35 mmol) 2-Methoxyethanamin 510 mg (87% d. Th.) der Titelverbindung erhalten.

LC-MS [Methode 4]: Rₜ = 2.28 min; MS [ESIpos]: m/z = 377 (M+H)⁺

¹H-NMR (400MHz, DMSO-d₆): δ [ppm]= 1.37 (br. s., 9H), 3.19 - 3.29 (m, 2H), 3.23 (s, 3H), 3.28 - 3.41 (m, 2H), 5.43 (d, 1H), 7.46 - 7.55 (m, 1H), 7.56 - 7.61 (m, 1H), 7.62 - 7.75 (m, 3H), 7.91 - 8.01 (m, 1H).

### Beispiel 35A

### tert.-Butyl-{2-[(2-hydroxyethyl)amino]-2-oxo-1-[3-(trifluormethyl)phenyl]ethyl} carbamat

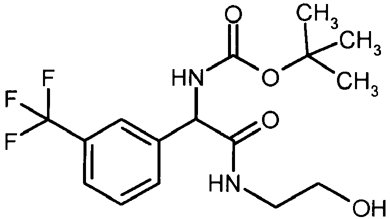

Analog zu Beispiel 18A, aber ohne N,N'-Diisopropylethylamin, wurden aus 319 mg (1.0 mmol) [(*tert*.-Butoxycarbonyl)amino][3-(trifluormethyl)phenyl]essigsäure und 183 mg (3.0 mmol) 2-Ethanolamin 210 mg (58% d. Th.) der Titelverbindung erhalten.

LC-MS [Methode 4]: Rₜ = 1.05 min; MS [ESIpos]: m/z = 363 (M+H)⁺

¹H-NMR(400MHz, DMSO-d₆): δ [ppm]= 1.37 (d, 9H), 3.01 - 3.22 (m, 2H), 3.34 - 3.43 (m, 2H), 4.68 (t, 1H), 5.29 (d, 1H), 7.49 (d, 1H), 7.57 (t, 1H), 7.64 (d, 1H), 7.71 (d, 1H), 7.78 (s, 1H), 8.29 (t, 1H).

### Beispiel 36A

### [(tert.-Butoxycarbonyl)amino][3-(trifluormethoxy)phenyl]essigsäure

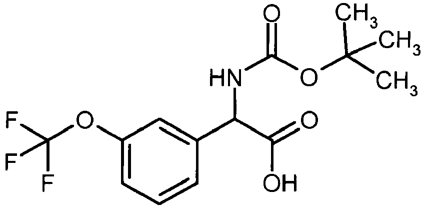

Analog zu Beispiel 26A wurden 2.0 g (8.5 mmol) 3-(Trifluormethoxy)-DL-phenylalanin mit 1.89 g (8.68 mmol) Di-*tert*.-butyldicarbonat umgesetzt. Man erhielt 3.0 g (quant.) der Titelverbindung.

LC-MS [Methode 4]: Rₜ = 1.05 min; MS [ESIpos]: m/z = 236 (M+H-BOC)⁺; [ESIneg]: m/z = 334 (M-H)⁻.

¹H-NMR(400MHz, DMSO-d₆): δ [ppm]= 1.39 (s, 9H), 5.22 (d, 1H), 7.31 (d, 1H), 7.38 - 7.55 (m, 3H), 7.75 (d, 1H), 12.96 (br. s., 1H).

### Beispiel 37A

### tert.-Butyl-{2-[(2-hydroxyethyl)amino]-2-oxo-1-[3-(trifluormethoxy)phenyl]ethyl}carbamat

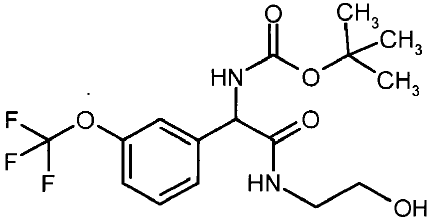

Analog zu Beispiel 18A, aber ohne N,N'-Diisopropylethylamin, wurden aus 250 mg (0.75 mmol) [(*tert*.-Butoxycarbonyl)amino][2-(trifluormethoxy)phenyl]essigsäure und 55 mg (0.90 mmol) 2-Ethanolamin 258 mg (91% d. Th.) der Titelverbindung erhalten.

LC-MS [Methode 3]: Rₜ = 1.10 min; MS [ESIpos]: m/z = 279 (M+H-BOC)⁺; [ESIneg]: m/z = 377 (M-H)⁻.

¹H-NMR (400MHz, DMSO-d₆): δ [ppm]= 1.37 (s, 9H), 3.02 - 3.22 (m, 2H), 3.33 - 3.43 (m, 2H), 4.68 (t, 1H), 5.24 (d, 1H), 7.27 (d, 1H), 7.37 - 7.50 (m, 4H), 8.27 (t, 1H).

### Beispiel 38A

### tert.-Butyl-{(1S)-3-[(2-hydroxyethyl)amino]-3-oxo-1-[2-(trifluormethyl)phenyl]propyl}carbamat

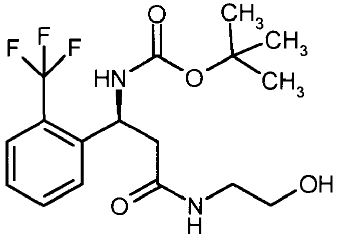

Analog zu Beispiel 32A aber mit 15 h Reaktionszeit wurden aus 150 mg (0.45 mmol) (*3S*)-3-[(*tert*.-Butoxycarbonyl)amino]-3-[2-(trifluormethyl)phenyl]propansäure und 33 mg (0.54 mmol) 2-Aminoethanol 146 mg (86% d. Th.) der Titelverbindung erhalten.

LC-MS [Methode 1]: Rₜ = 1.53 min; MS [ESIpos]: m/z = 377 (M+H)⁺

¹H-NMR (400MHz, DMSO-d₆): δ [ppm]= 1.32 (s, 9H), 2.24 - 2.34 (m, 2H), 3.02 - 3.19 (m, 2H), 3.34 - 3.41 (m, 2H), 4.64 (t, 1H), 5.20 - 5.30 (m, 1H), 7.39 - 7.55 (m, 2H), 7.61 - 7.73 (m, 5H).

### Beispiel 39A

### tert. -Butyl-{(1S)-3-[(2-hydroxyethyl)amino]-3-oxo-1-[3-(trifluormethyl)phenyl]propyl carbamat

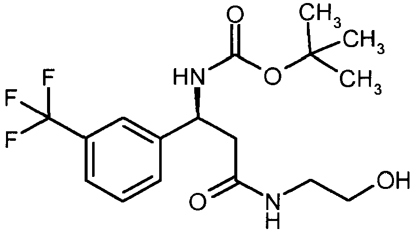

Analog zu Beispiel 18A, aber ohne N,N'-Diisopropylethylamin, wurden aus 166 mg (0.50 mmol) (*3S*)-3-[(*tert*.-Butoxycarbonyl)amino]-3-[3-(trifluormethyl)phenyl]propansäure und 76 mg (1.24 mmol) 2-Aminoethanol 179 mg (94% d. Th.) der Titelverbindung erhalten.

LC-MS [Methode 4]: Rₜ = 2.00 min; MS [ESIpos]: m/z = 377 (M+H)⁺

¹H-NMR (400MHz, DMSO-d₆): δ [ppm]= 1.35 (s, 9H), 3.03 (q, 2H), 3.21 - 3.31 (m, 2H), 4.58 (t, 1H), 4.98 (q, 1H), 7.50 - 7.64 (m, 5H), 7.84 (t, 1H).

### Beispiel 40A

### tert.-Butyl-{(1S)-3-[(2-methoxyethyl)amino]-3-oxo-1-[2-(trifluormethyl)phenyl]propyl}carbamat

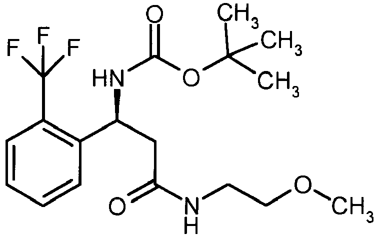

Analog zu Beispiel 32A aber mit 15 h Reaktionszeit wurden aus 142 mg (0.43 mmol) (*3S*)-3-[(*tert*.-Butoxycarbonyl)amino]-3-[2-(trifluormethyl)phenyl]propansäure und 38 mg (0.51 mmol) 2-Methoxyethanamin 133 mg (80% d. Th.) der Titelverbindung erhalten.

LC-MS [Methode 1]: Rₜ = 1.76 min; MS [ESIpos]: m/z = 391 (M+H)⁺

¹H-NMR (400MHz, DMSO-d₆): δ [ppm]= 1.32 (s, 9H), 2.23 - 2.35 (m, 2H), 3.13 - 3.25 (m, 2H), 3.23 (s, 3H), 3.30 (t, 2H), 5.19 - 5.30 (m, 1H), 7.40 - 7.52 (m, 2H), 7.62 - 7.70 (m, 3H), 7.76 (br. t, 1H).

### Beispiel 41A

### tert.-Butyl-{(1S)-3-[(2-methoxyethyl)amino]-3-oxo-1-[3-(trifluormethyl)phenyl]propyl carbamat

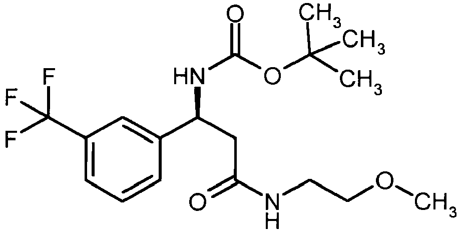

Analog zu Beispiel 18A, aber ohne N,N'-Diisopropylethylamin, wurden aus 150 mg (0.45 mmol) (*3S*)-3-[(*tert*.-Butoxycarbonyl)amino]-3-[3-(trifluormethyl)phenyl]propansäure und 41 mg (0.54 mmol) 2-Methoxyethanamin 167 mg (95% d. Th.) der Titelverbindung erhalten.

LC-MS [Methode 4]: Rₜ = 2.20 min; MS [ESIpos]: m/z = 391 (M+H)⁺

¹H-NMR (400MHz, DMSO-d₆): δ [ppm]= 1.35 (s, 9H), ca. 2.52 (m, 2H, teilweise unter dem DMSO-Signal), 3.04 - 3.26 (m, 4H), 3.16 (s, 3H), 4.97 (br. q, 1H), 7.46 - 7.66 (m, 5H), 7.93 (t, 1H).

### Beispiel 42A

### tert.-Butyl-(2-oxo-2-{[2-(trifluormethoxy)ethyl]amino}-1-[3-(trifluormethyl)phenyl]ethyl)carbamat (Racemat)

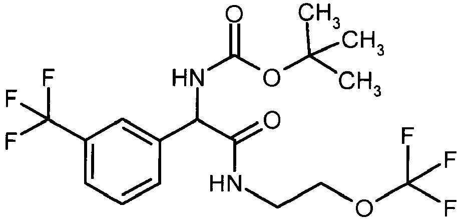

Analog zu Beispiel 18A wurden aus 250 mg (0.78 mmol) [(*tert*.-Butoxycarbonyl)amino][3-(trifluormethyl)phenyl]essigsäure und 156 mg (0.94 mmol) 2-(Trifluormethoxy)-ethanamin-Hydrochlorid 320 mg (95% d. Th.) der Titelverbindung erhalten.

LC-MS [Methode 4]: Rₜ = 2.52 min; MS [ESIpos]: m/z = 431 (M+H)⁺

¹H-NMR(400MHz, DMSO-d₆): δ [ppm]= 1.38 (s, 9H), 3.27 - 3.48 (m, 2H), 3.94 - 4.07 (m, 2H), 5.29 (d, 1H), 7.53 - 7.60 (m, 2H), 7.62 - 7.67 (m, 1H), 7.70 (d, 1H), 7.78 (s, 1H), 8.53 (t, 1H).

Durch Chromatographie an einer chiralen Phase [Methode 14a] wurde die Titelverbindung in ihren beiden Enantiomere getrennt: siehe Beispiele 43A und 44A.

### Beispiel 43A

### tert.-Butyl-(2-oxo-2-{[2-(trifluormethoxy)ethyl]amino}-1-[3-(trifluormethyl)phenyl]ethyl)carbamat (Enantiomer 1)

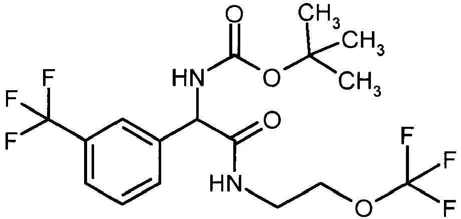

Zuerst eluierendes Enantiomer (150 mg) aus der chromatographischen Enantiomerentrennung der Verbindung aus Beispiel 42A nach Methode 14a.

Chirale analytische HPLC [Methode 15a]: Rₜ = 3.56 min.

### Beispiel 44A

### tert.-Butyl-(2-oxo-2-{[2-(trifluormethoxy)ethyl]amino}-1-[3-(trifluormethyl)phenyl] ethyl)carbamat (Enantiomer 2)

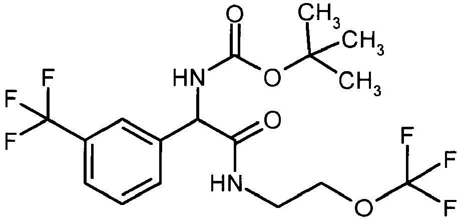

Zuletzt eluierendes Enantiomer (128 mg) aus der chromatographischen Enantiomerentrennung der Verbindung aus Beispiel 42A nach Methode 14a.

Chirale analytische HPLC [Methode 15a]: Rₜ = 3.89 min.

### Beispiel 45A

### Ethyl-1-({amino[3-(trifluormethyl)phenyl]acetyl}amino)cyclopropancarboxylat-Hydrochlorid

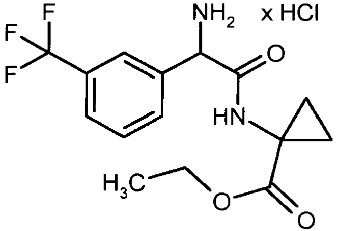

262 mg (0.61 mmol) der Verbindung aus Beispiel 17A wurden in 4 ml Dichlormethan gelöst und mit 4 ml einer 4N Lösung von Chlorwasserstoff in Dioxan versetzt. Die Lösung wurde 3 h bei RT gerührt, dann am Rotationsversampfer von den flüchtigen Bestandteilen befreit. Der Rückstand wurde im Hochvakuum getrocknet. Man erhielt 223 mg (quant.) der Titelverbindung.

LC-MS [Methode 3]: Rt = 0.79 min; MS [ESIpos]: m/z = 331 (M+H)+

¹H-NMR (400MHz, DMSO-d₆): δ [ppm]= 0.95 (t, 3H), 0.97 - 1.08 (m, 2H), 1.27 - 1.34 (m, 1H), 1.46 - 1.53 (m, 1H), 3.85 - 3.99 (m, 2H), 5.08 (s, 1H), 7.73 (t, 1H), 7.81 - 7.87 (m, 2H), 7.95 (s, 1H), 8.73 (br. s., 3H), 9.24 (s, 1H).

Nach der gleichen Methode wurden die Beispiele 46A bis 61A in Tabelle 1 hergestellt. Die Reaktionszeiten bis zur vollständigen Umsetzung lagen zwischen 0.5 h und 16 h. Die Ausbeuten sind größer als 90% d. Th.

**Tabelle 1:**

| **Beispiel Nr.** | **Struktur** | **Edukt** | **Beispielname und Analytik** |
|---|---|---|---|
| **46A** | 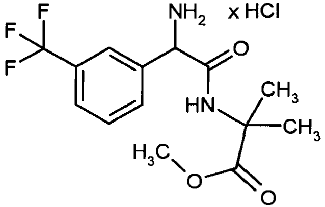 | 18A | Methyl-N-{amino[3-(trifluormethyl)phenyl]acetyl -2-methylalaninat-Hydrochlorid |
| | | | LC-MS [Methode 2]: Rₜ = 1.32 min; MS [ESIpos]: m/z = 319 (M+H)⁺ |
| **47A** | 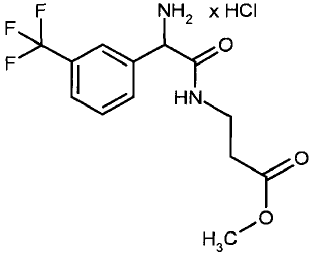 | 19A | Methyl-N-{amino[3-(trifluormethyl)phenyl]acetyl}-beta-alaninat-Hydrochlorid |
| | | | LC-MS [Methode 3]: Rₜ = 0.66 min; MS [ESIpos]: m/z = 305 (M+H)⁺ |
| **48A** | 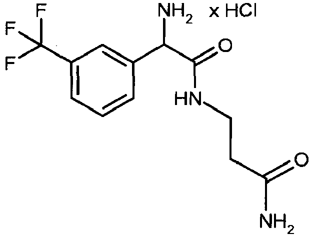 | 21A | N3-{Amino[3-(trifluormethyl)phenyl]-acetyl}-beta-alaninamid-Hydrochlorid (*Enantiomer 1*) |
| | | | LC-MS [Methode 3]: Rₜ = 0.28 min; MS [ESIpos]: m/z = 290 (M+H)⁺ |
| **49A** | 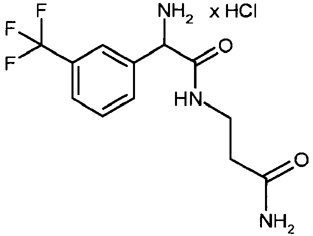 | 22A | N3-{Amino[3-(trifluormethyl)phenyl]-acetyl}-beta-alaninamid-Hydrochlorid (*Enantiomer 2*) |
| | | | LC-MS [Methode 2]: Rₜ = 0.86 min; MS [ESIpos]: m/z = 290 (M+H)⁺ |
| **50A** | 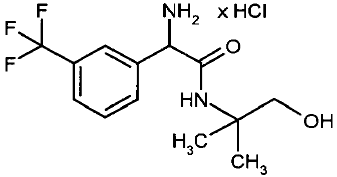 | 30A | 2-Amino-N-(1-hydroxy-2-methylpropan-2-yl)-2-[3-(trifluormethyl)phenyl] acetamid-Hydrochlorid (*Enantiomer 1*) |
| | | | LC-MS [Methode 2]: Rₜ = 1.22 min; MS [ESIpos]: m/z = 291 (M+H)⁺ |
| **51A** | 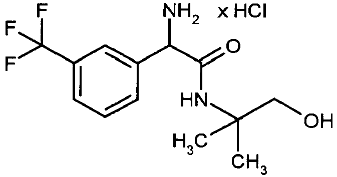 | 31A | 2-Amino-N-(1-hydroxy-2-methylpropan-2-yl)-2-[3-(trifluormethyl)phenyl]acetamid-Hydrochlorid (*Enantiomere 2*) |
| | | | LC-MS [Methode 2]: Rₜ = 1.22 min; MS [ESIpos]: m/z = 291 (M+H)⁺ |
| **52A** | 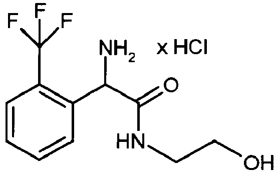 | 33A | 2-Amino-N-(2-hydroxyethyl)-2-[2-(trifluormethyl)phenyl] acetamid-Hydrochlorid (Reinheit 75%) |
| | | | LC-MS [Methode 2]: Rₜ = 0.22 min; MS [ESIpos]: m/z = 263 (M+H)⁺ |
| **53A** | 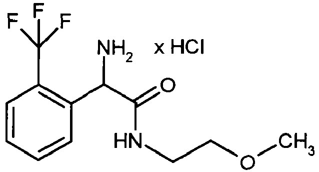 | 34A | 2-Amino-N-(2-methoxyethyl)-2-[2-(trifluormethyl)phenyl] acetamid-Hydrochlorid |
| | | | LC-MS [Methode 2]: Rₜ = 0.93 min; MS [ESIpos]: m/z = 277 (M+H)⁺ |
| **54A** | 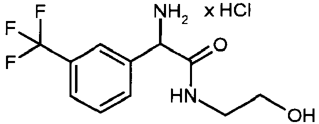 | 35A | 2-Amino-N-(2-hydroxyethyl)-2-[3-(trifluormethyl)phenyl]acetamid-Hydrochlorid |
| | | | LC-MS [Methode 2]: Rₜ = 0.97 min; MS [ESIpos]: m/z = 263 (M+H)⁺ |
| **55A** | 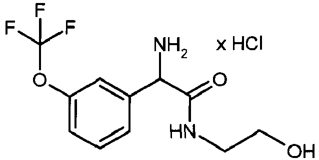 | 37A | 2-Amino-N-(2-hydroxyethyl)-2-[3-(trifluormethoxy)phenyl] acetamid-Hydrochlorid |
| | | | LC-MS [Methode 2]: Rₜ = 1.01 min; MS [ESIpos]: m/z = 279 (M+H)⁺ |
| **56A** | 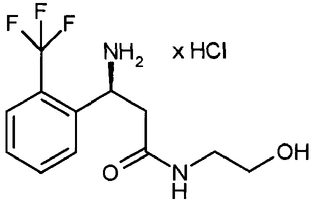 | 38A | (*3S*)-3-Amino-N-(2-hydroxyethyl)-3-[2-(trifluormethyl)phenyl]propanamid-Hydrochlorid |
| | | | LC-MS [Methode 3]: Rₜ = 0.30 min; MS [ESIpos]: m/z = 277 (M+H)⁺ |
| **57A** | 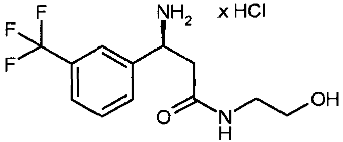 | 39A | (3*S*)-3-Amino-N-(2-hydroxyethyl)-3-[3-(trifluormethyl)phenyl]propanamid-Hydrochlorid |
| | | | LC-MS [Methode 2]: Rₜ = 0.90 min; MS [ESIpos]: m/z = 277 (M+H)⁺ |
| **58A** | 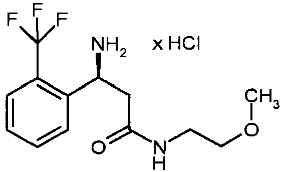 | 40A | (*3S*)-3-Amino-N-(2-methoxyethyl)-3-[2-(trifluormethyl)phenyl]propanamid-Hydrochlorid |
| | | | LC-MS [Methode 3]: Rₜ = 0.52 min; MS [ESIpos]: m/z = 291 (M+H)⁺ |
| **59A** | 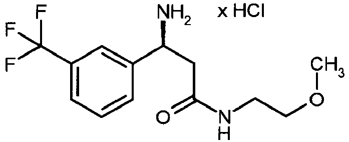 | 41A | (*3S)*-3-Amino-N-(2-methoxyethyl)-3-[3-(trifluormethyl)phenyl]propanamid - Hydrochlorid |
| | | | LC-MS [Methode 3]: Rₜ = 0.62 min; MS [ESIpos]: m/z = 291 (M+H)⁺ |
| **60A** | 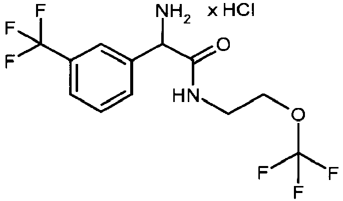 | 43A | 2-Amino-N-[2-(trifluormethoxy)ethyl]-2-[3-(trifluormethyl)phenyl]acetamid-Hydrochlorid (*Enantiomer 1*) |
| | | | LC-MS [Methode 3]: Rₜ = 0.81 min; MS [ESIpos]: m/z = 331 (M+H)⁺ |
| **61A** | 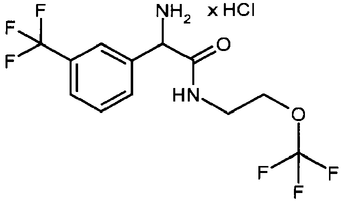 | 44A | 2-Amino-N-[2-(trifluormethoxy)ethyl]-2-[3-(trifluormethyl)phenyl]acetamid-Hydrochlorid (*Enantiomer 2*) |
| | | | LC-MS [Methode 3]: Rₜ = 0.81 min; MS [ESIpos]: m/z = 331 (M+H)⁺ |

### Beispiel 62A

### N²-{Amino[2-(trifluormethyl)phenyl]acetyl}-2-methylalaninamid-Hydrochlorid

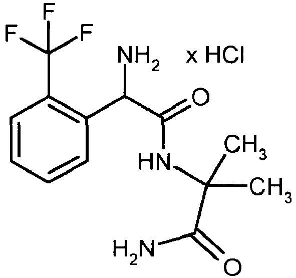

922 mg (0.48 mmol) der Verbindung aus Beispiel 24A wurden mit 4.45 ml einer 4N Lösung von Chlorwasserstoff in Dioxan versetzt. Die Lösung wurde 1 h bei RT gerührt, dann am Rotationsversampfer von den flüchtigen Bestandteilen befreit. Der Rückstand wurde mit 10 ml Diethylether gerührt. Der Feststoff wurde durch Filtration isoliert und im Hochvakuum getrocknet. Man erhielt 167 mg (96% d. Th.) der Titelverbindung.

LC-MS [Methode 5]: Rₜ = 0.36 min; MS [ESIpos]: m/z = 304 (M+H)⁺.

¹H-NMR (400MHz, DMSO-d₆): δ [ppm]= 1.34 (s, 3H), 1.40 (s, 3H), 5.14 (s, 1H), 7.13 (s, 1H), 7.37 (s, 1H), 7.68 (t, 1H), 7.75 - 7.89 (m, 3H), 8.21 (br. s., 1H), 8.94 (br. s., 3H).

In Analogie zu Beispiel 62A wurden die Beispiele 63A bis 66A in Tabelle 2 hergestellt. Die Ausbeute sind jeweils größer als 90% d. Th..

**Tabelle 2:**

| **Beispiel Nr.** | **Struktur** | **Edukt** | **Beispielname und Analytik** |
|---|---|---|---|
| **63A** | 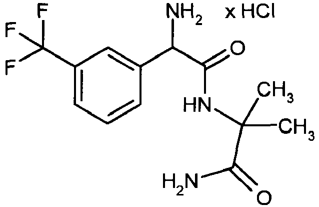 | 23A | N²-{Amino[3-(trifluormethyl)phenyl]acetyl}-2-methylalaninamid-Hydrochlorid |
| | | | LC-MS [Methode 2]: Rₜ = 1.05 min; MS [ESIpos]: m/z = 304 (M+H)⁺ |
| **64A** | 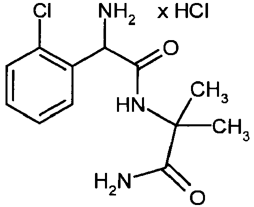 | 25A | N²-[Amino(2-chlorphenyl)acetyl]-2-methylalaninamid-Hydrochlorid |
| | | | LC-MS [Methode 5]: Rₜ = 0.26 min; MS [ESIpos]: m/z = 270 (M+H)⁺ |
| **65A** | 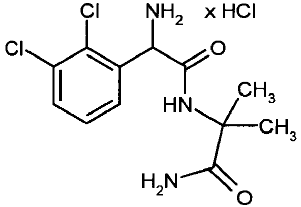 | 27A | N²-[Amino(2,3-dichlorphenyl)acetyl]-2-methylalaninamid-Hydrochlorid |
| | | | LC-MS [Methode 5]: Rₜ = 0.44 min; MS [ESIpos]: m/z = 304 (M+H)⁺ |
| **66A** | 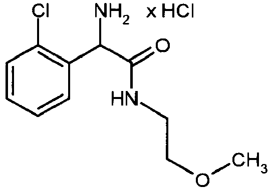 | 32A | 2-Amino-2-(2-chlorphenyl)-N-(2-methoxyethyl)acetamid-Hydrochlorid |
| | | | LC-MS [Methode 5]: Rₜ = 0.29 min; MS [ESIpos]: m/z = 243 (M+H)⁺ |

### Beispiel 67A

### Methyl-N-{(3S)-3-amino-3-[3-(trifluormethyl)phenyl]propanoyl}-2-methylalaninat

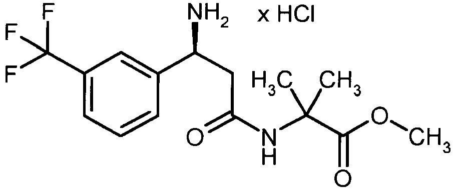

130 mg (0.30 mmol) der Verbindung aus Beispiel 28A wurden in 2 ml Acetonitril gelöst und mit 2 ml einer 4N-Chlorwasserstofflösung in Dioxan versetzt. Die Lösung wurde 3 h bei RT gerührt, dann am Rotationsversampfer von den flüchtigen Bestandteilen befreit. Der Rückstand wurde im Hochvakuum getrocknet. Man erhielt 108 mg (97% d. Th.) der Titelverbindung.

LC-MS [Methode 3]: Rt = 0.75 min; MS [ESIpos]: m/z = 333 (M+H)+.

¹H-NMR(400MHz, DMSO-d₆): δ [ppm]= 1.16 (s, 3H), 1.24 (s, 3H), 2.78 - 2.91 (m, 2H), 3.41 (s, 3H), 4.66 - 4.76 (m, 1H), 7.63 - 7.70 (m, 1H), 7.73 - 7.81 (m, 2H), 7.87 (s, 1H), 8.48 - 8.68 (m, 4H).

### Ausführungsbeispiele:

### Beispiel 1

### Ethyl-1-({[({3-(4-chlorphenyl)-5-oxo-4-[(2S)-3,3,3-trifluor-2-hydroxypropyl]-4,5-dihydro-1H-1,2,4-triazol-1-yl}acetyl)amino][3-(trifluormethyl)phenyl]acetyl}amino)cyclopropancarboxylat (Diastereomerengemisch)

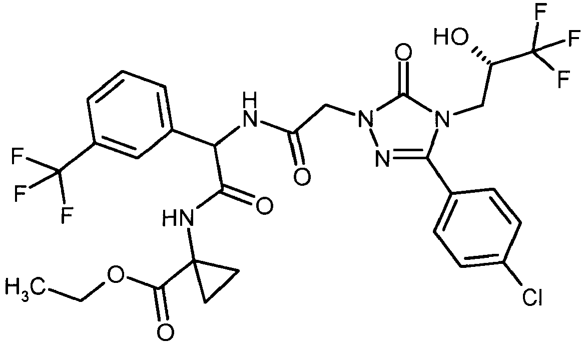

Eine Mischung aus 50 mg (0.14 mmol) der Verbindung aus Beispiel 8A, 55 mg (0.15 mmol) der Verbindung aus Beispiel 45A, 39 mg (0.21 mmol) EDC, 28 mg (0.21 mmol) HOBt und 29 µl (0.16 mmol) N,N'-Diisopropylethylamin in 1.4 ml DMF wurde 3 h bei RT gerührt, dann per präparativer HPLC [Methode 6] getrennt. Die geeignete Fraktion wurde am Rotationsverdampfer eingeengt und der Rückstand am Hochvakuum getrocknet. Man erhielt 82 mg (88% d. Th.) der Titelverbindung als Diastereomerengemisch.

LC-MS [Methode 3]: Rₜ = 1.31 min; MS [ESIpos]: m/z = 678 (M+H)⁺

¹H-NMR (400MHz, DMSO-d₆): δ [ppm]= 0.91 (td interpretiert als je 1 t pro Diastereomer, 3H), 0.95 - 1.08 (m, 2H), 1.25 - 1.33 (m, 1H), 1.38 - 1.46 (m, 1H), 3.78 - 4.01 (m, 4H), 4.18 - 4.33 (m, 1H), 4.52 - 4.65 (m, 2H), 5.54 (d, 1H), 6.89 (dd interpretiert als je 1 d pro Diastereomer, 1H), 7.58 - 7.65 (m, 3H), 7.66 - 7.71 (m, 1H), 7.71 - 7.77 (m, 3H), 7.82 (s, 1H), 9.07 (d, 1H), 9.09 (s, 1H).

### Beispiel 2

### 1-({[({3-(4-Chlorphenyl)-5-oxo-4-[(2S)-3,3,3-trifluor-2-hydroxypropyl]-4,5-dihydro-1H-1,2,4-triazol-1-yl}acetyl)amino][3-(trifluormethyl)phenyl]acetyl}amino)cyclopropancarbonsäure (Diastereomerengemisch)

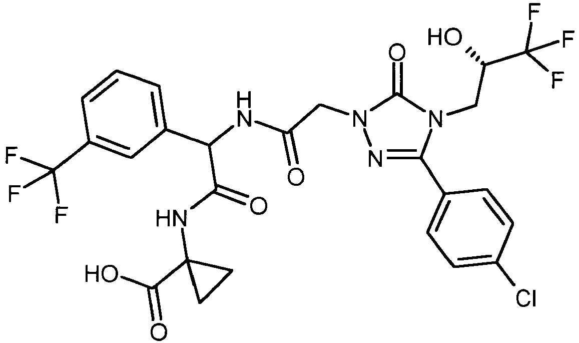

78 mg (0.115 mmol) der Verbindung aus Beispiel 1 wurden in 2.2 ml Methanol gelöst und mit 460 µl einer 1M wässrigen Lithiumhydroxidlösung (0.46 mmol) versetzt. Die Mischung wurde über Nacht bei RT gerührt, dann durch Zugabe 1N Salzsäure auf pH 2 gestellt, mit wenig DMSO verdünnt und per präparativer HPLC [Methode 6] getrennt. Man erhielt 64 mg (86 % d. Th.) der Titelverbindung.

LC-MS [Methode 3]: Rₜ = 1.19 min; MS [ESIpos]: m/z = 650 (M+H)⁺

Durch präparative HPLC an einer chiralen Phase [Methode 14a] wurden die beiden Diastereomere getrennt: siehe Beispiel 3 und Beispiel 4.

### Beispiel 3

### 1-({[({3-(4-Chlorphenyl)-5-oxo-4-[(2S)-3,3,3-trifluor-2-hydroxypropyl]-4,5-dihydro-1H-1,2,4-triazol-1-yl}acetyl)amino] [3-(trifluormethyl)phenyl]acetyl} amino)cyclopropancarbonsäure (Diastereomer 1)

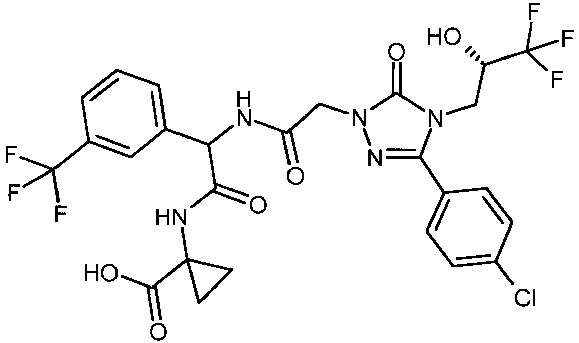

Zuerst eluierendes Diastereomer aus der Diastereomerentrennung von 64 mg der Verbindung aus Beispiel 2 nach Methode 14a. Nach der Chromatographie an der chiralen Phase wurde das erhaltene Produkt noch von Lösungsmittelkontaminanten durch präparative HPLC [Methode 6] gereinigt. Man erhielt 14 mg der Titelverbindung.

Chirale analytische HPLC [Methode 15a]: Rₜ = 4.60 min.

LC-MS [Methode 1]: Rₜ = 1. 92 min; MS [ESIpos]: m/z = 650 (M+H)⁺

¹H-NMR (400MHz, DMSO-d₆): δ [ppm]= 0.84 - 0.93 (m, 1H), 0.93 - 1.02 (m, 1H), 1.25 - 1.42 (m, 2H), 3.82 (dd, 1H), 3.96 (dd, 1H), 4.20 - 4.34 (m, 1H), 4.52 - 4.65 (m [AB], 2H), 5.55 (d, 1H), 6.91 (br. d, 1H), 7.55 - 7.78 (m, 6H), 7.82 (s, 1H), 8.99 - 9.07 (m, 2H), 12.43 (s, 1H).

### Beispiel 4

### 1-({[({3-(4-Chlorphenyl)-5-oxo-4-[(2S)-3,3,3-trifluor-2-hydroxypropyl]-4,5-dihydro-1H-1,2,4-triazol-1-yl}acetyl)amino][3-(trifluormethyl)phenyl]acetyl}amino)cyclopropancarbonsäure (Diastereomer 2)

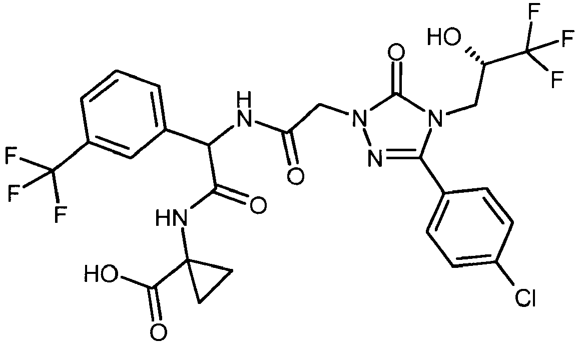

Zuletzt eluierendes Diastereomer aus der Diastereomerentrennung von 64 mg der Verbindung aus Beispiel 2 nach Methode 14a. Nach der Chromatographie an der chiralen Phase wurde das erhaltene Produkt noch von Lösungsmittelkontaminanten durch präparative HPLC [Methode 6] gereinigt. Man erhielt 16 mg der Titelverbindung.

Chirale analytische HPLC [Methode 15a]: Rₜ = 5.85 min.

LC-MS [Methode 1]: Rₜ = 1. 91 min; MS [ESIpos]: m/z = 650 (M+H)⁺

¹H-NMR (400MHz, DMSO-d₆): δ [ppm]= 0.83 - 0.92 (m, 1H), 0.93 - 1.02 (m, 1H), 1.25 - 1.42 (m, 2H), 3.82 (dd, 1H), 3.96 (dd, 1H), 4.20 - 4.34 (m, 1H), 4.52 - 4.67 (m [AB], 2H), 5.55 (d, 1H), 6.89 (d, 1H), 7.55 - 7.78 (m, 7H), 7.83 (s, 1H), 8.89 - 9.14 (m, 2H), 12.43 (s, 1H).

### Beispiel 5

### Ethyl-1-({({[3-(4-Chlorphenyl)-4-cyclopropyl-5-oxo-4,5-dihydro-1H-1,2,4-triazol-1-yl]acetyl}amino)[3-(trifluormethyl)phenyl]acetyl}amino)cyclopropancarboxylat (Racemat)

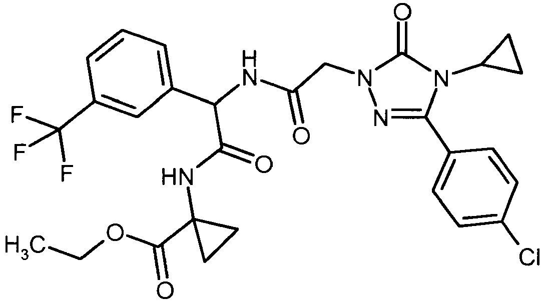

Analog zu Beispiel 1 wurde aus 50 mg (0.17 mmol) [3-(4-Chlorphenyl)-4-cyclopropyl-5-oxo-4,5-dihydro-1*H*-1,2,4-triazol-1-yl]-essigsäure (Herstellung siehe WO 2007/134862, Beispiel 88A) und 69 mg (0.19 mmol) der Verbindung aus Beispiel 45A die Titelverbindung hergestellt. Man erhielt 83 mg (80% d. Th.) der Titelverbindung.

LC-MS [Methode 3]: Rₜ = 1.27 min; MS [ESIpos]: m/z = 606 (M+H)⁺

¹H-NMR (400MHz, DMSO-d₆): δ [ppm]= 0.51 - 0.60 (m, 2H), 0.85 - 0.92 (m, 2H), 0.91 (t, 3H), 0.94 - 1.08 (m, 2H), 1.24 - 1.33 (m, 1H), 1.38 - 1.46 (m, 1H), 3.17 (tt, 1H), 3.82 - 3.98 (m, 2H), 4.45 - 4.59 (m [AB], 2H), 5.52 (d, 1H), 7.54 - 7.65 (m, 3H), 7.65 - 7.71 (m, 1H), 7.71 - 7.84 (m, 4H), 9.03 (d, 1H), 9.08 (s, 1H).

### Beispiel 6

### 1-({({[3-(4-Chlorphenyl)-4-cyclopropyl-5-oxo-4,5-dihydro-1H-1,2,4-triazol-1-yl]acetyl}amino)[3-(trifluormethyl)phenyl]acetyl}amino)cyclopropancarbonsäure (Racemat)

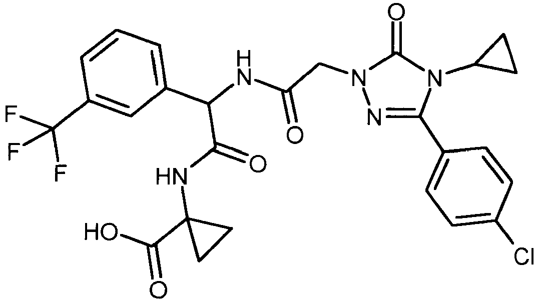

64 mg (0.106 mmol) der Verbindung aus Beispiel 5 wurden in 3 ml Methanol gelöst und mit 422 µl einer 1M wässrigen Lithiumhydroxidlösung (0.42 mmol) versetzt. Die Mischung wurde über Nacht bei RT gerührt. Da die Umsetzung sehr langsam war, wurden noch 210 µl (0.21 mmol) einer 1M Natriumhydroxidlösung zugeben und die Mischung weiter 5 Tage bei RT gerührt. Durch Zugabe 1N Salzsäure wurde die Mischung auf pH 2 gestellt, mit wenig DMSO verdünnt und per präparativer HPLC [Methode 6] getrennt. Man erhielt 37 mg (61 % d. Th.) der Titelverbindung.

LC-MS [Methode 3]: Rₜ = 1.15 min; MS [ESIpos]: m/z = 578 (M+H)⁺

¹H-NMR (400MHz, DMSO-d₆): δ [ppm]= 0.51 - 0.61 (m, 2H), 0.83 - 0.93 (m, 3H), 0.94 - 1.02 (m, 1H), 1.25 - 1.41 (m, 2H), 3.17 (tt, 1H), 4.47 - 4.58 (m [AB], 2H), 5.54 (d, 1H), 7.55 - 7.62 (m, 3H), 7.64 - 7.68 (m, 1H), 7.70 (d, 1H), 7.76 - 7.84 (m, 3H), 8.98 (d, 1H), 9.01 (s, 1H), 12.43 (br. s., 1H).

### Beispiel 7

### Ethyl-1-({({[3-(5-chlor-2-thienyl)-4-(2-methoxyethyl)-5-oxo-4,5-dihydro-1H-1,2,4-triazol-1-yl]acetyl}amino)[3-(trifluormethyl)phenyl]acetyl}amino)cyclopropancarboxylat (Racemat)

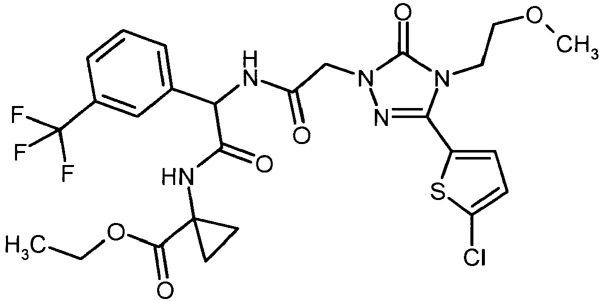

Analog zu Beispiel 1 wurden 25 mg (79 µmol) der Verbindung aus Beispiel 15A und 32 mg (87 µmol) der Verbindung aus Beispiel 45A eingesetzt. Nach HPLC-Reinigung [Methode 7] erhielt man 47 mg (95% d. Th.) der Titelverbindung.

LC-MS [Methode 5]: Rₜ = 1.05 min; MS [ESIpos]: m/z = 630 (M+H)⁺

¹H-NMR (400MHz, DMSO-d₆): δ [ppm]= 0.91 (t, 3H), 0.95 - 1.09 (m, 2H), 1.24 - 1.32 (m, 1H), 1.38 - 1.46 (m, 1H), 3.19 (s, 3H), 3.53 (t, 2H), 3.84 - 3.94 (m, 2H), 3.96 (t, 2H), 4.49 - 4.60 (m [AB], 2H), 5.53 (d, 1H), 7.26 (d, 1H), 7.55 (d, 1H), 7.62 (t, 1H), 7.69 (d, 1H), 7.74 (d, 1H), 7.81 (s, 1H), 9.07 (d, 1H), 9.09 (s, 1H).

### Beispiel 8

### 1-({({[3-(5-Chlor-2-thienyl)-4-(2-methoxyethyl)-5-oxo-4,5-dihydro-1H-1,2,4-triazol-1-yl]acetyl}amino)[3-(trifluormethyl)phenyl]acetyl}amino)cyclopropancarbonsäure (Racemat)

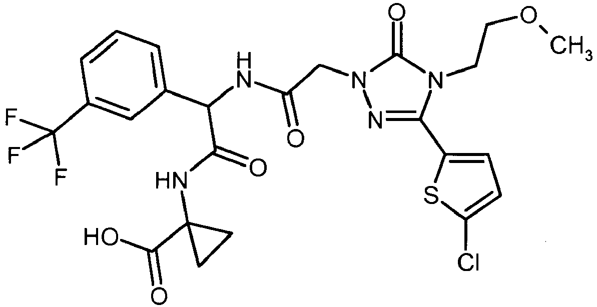

43 mg (68 µmol) der Verbindung aus Beispiel 7 wurden in 3 ml Methanol gelöst und mit 273 µl einer 1M wässrigen Lithiumhydroxidlösung (0.27 mmol) versetzt. Die Mischung wurde über Nacht bei RT gerührt, dann durch Zugabe 1N Salzsäure sauer gestellt und mit Wasser verdünnt. Der ausgefallene Feststoff wurde abgesaugt, mit wenig Wasser gewaschen und im Vakuumtrockenschrank bei 50°C getrocknet. Man erhielt 32 mg (74 % d. Th.) der Titelverbindung.

LC-MS [Methode 3]: Rₜ = 1.16 min; MS [ESIpos]: m/z = 602 (M+H)⁺

¹H-NMR (400MHz, DMSO-d₆): δ [ppm]= 0.84 - 0.93 (m, 1H), 0.94 - 1.04 (m, 1H), 1.25 - 1.43 (m, 2H), 3.19 (s, 3H), 3.50 - 3.60 (m, 2H), 3.97 (t, 2H), 4.46 - 4.64 (m, 2H), 5.54 (d, 1H), 7.26 (d, 1H), 7.53 - 7.63 (m, 2H), 7.69 (dd, 2H), 7.82 (s, 1H), 8.98 - 9.08 (m, 2H), 12.44 (br. s., 1H).

### Beispiel 9

### Methyl-N-{({[3-(5-chlor-2-thienyl)-4-(2-methoxyethyl)-5 -oxo-4,5-dihydro-1H-1,2,4-triazol-1-yl]acetyl}amino)[3-(trifluormethyl)phenyl]acetyl}-2-methylalaninat (Racemat)

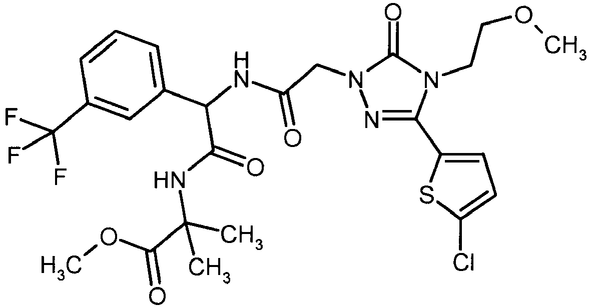

Analog zu Beispiel 1 wurden 25 mg (79 µmol) der Verbindung aus Beispiel 15A und 31 mg (87 µmol) der Verbindung aus Beispiel 46A eingesetzt. Nach HPLC-Reinigung [Methode 7] erhielt man 44 mg (90% d. Th.) der Titelverbindung.

LC-MS [Methode 5]: Rₜ = 1.05 min; MS [ESIpos]: m/z = 618 (M+H)⁺

¹H-NMR (400MHz, DMSO-d₆): δ [ppm]= 1.27 (s, 3H), 1.40 (s, 3H), 3.20 (s, 3H), 3.44 (s, 3H), 3.54 (t, 2H), 3.97 (t, 2H), 4.49 - 4.61 (m, 2H), 5.63 (d, 1H), 7.26 (d, 1H), 7.56 (d, 1H), 7.59 - 7.65 (m, 1H), 7.66 - 7.72 (m, 2H), 7.76 (s, 1H), 8.84 (s, 1H), 9.02 (d, 1H).

### Beispiel 10

### N-{({[3-(5-Chlor-2-thienyl)-4-(2-methoxyethyl)-5-oxo-4,5-dihydro-1H-1,2,4-triazol-1-yl]acetyl}amino)[3-(trifluormethyl)phenyl]acetyl}-2-methylalanin (Racemat)

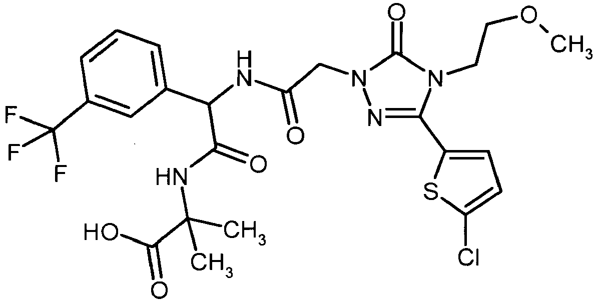

Analog zu Beispiel 8 wurden 40 mg (65 µmol) der Verbindung aus Beispiel 9 verseift. Man erhielt 31 mg (75 % d. Th.) der Titelverbindung.

LC-MS [Methode 3]: Rₜ = 1.20 min; MS [ESIpos]: m/z = 604 (M+H)⁺

¹H-NMR(400MHz, DMSO-d₆): δ [ppm]= 1.27 (s, 3H), 1.38 (s, 3H), 3.20 (s, 3H), 3.54 (t, 2H), 3.97 (t, 2H), 4.49 - 4.61 (m, 2H), 5.65 (d, 1H), 7.26 (d, 1H), 7.54 - 7.62 (m, 2H), 7.63 - 7.73 (m, 2H), 7.79 (s, 1H), 8.68 (s, 1H), 9.00 (d, 1H), 12.28 (br. s., 1H).

### Beispiel 11

### Methyl-N-{({[3-(4-chlorphenyl)-4-cyclopropyl-5-oxo-4,5-dihydro-1H-1,2,4-triazol-1-yl]acetyl}amino)[3-(trifluormethyl)phenyl]acetyl}-2-methylalaninat (Racemat)

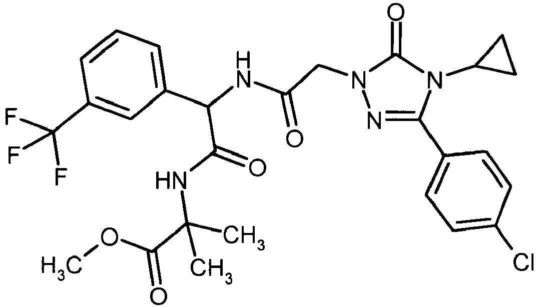

Analog zu Beispiel 1 wurde aus 50 mg (0.17 mmol) [3-(4-Chlorphenyl)-4-cyclopropyl-5-oxo-4,5-dihydro-1*H*-1,2,4-triazol-1-yl]-essigsäure (Herstellung siehe WO 2007/134862, Beispiel 88A) und 72 mg (0.19 mmol) der Verbindung aus Beispiel 46A die Titelverbindung hergestellt. Man erhielt 89 mg (88% d. Th.) der Titelverbindung.

LC-MS [Methode 3]: Rₜ = 1.28 min; MS [ESIpos]: m/z = 594 (M+H)⁺

¹H-NMR (400MHz, DMSO-d₆): δ [ppm]= 0.40 - 0.68 (m, 2H), 0.79 - 1.00 (m, 2H), 1.27 (s, 3H), 1.40 (s, 3H), 3.18 (dt, 1H), 3.44 (s, 3H), 4.46 - 4.58 (m [AB], 2H), 5.62 (d, 1H), 7.55 - 7.64 (m, 3H), 7.65 - 7.72 (m, 2H), 7.74 - 7.83 (m, 3H), 8.83 (s, 1H), 8.98 (d, 1H).

### Beispiel 12

### N-{({[3-(4-Chlorphenyl)-4-cyclopropyl-5-oxo-4,5-dihydro-1H-1,2,4-triazol-1-yl]acetyl}amino)[3-(trifluormethyl)phenyl]acetyl}-2-methylalanin (Racemat)

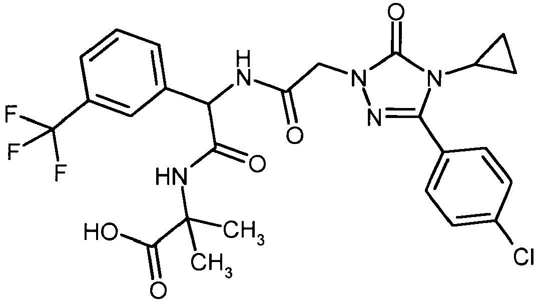

Analog zu Beispiel 6 wurden 68 mg (114 µmol) der Verbindung aus Beispiel 9 verseift. Man erhielt 55 mg (83 % d. Th.) der Titelverbindung.

LC-MS [Methode 3]: Rₜ = 1.19 min; MS [ESIpos]: m/z = 580 (M+H)⁺

¹H-NMR (400MHz, DMSO-d₆): δ [ppm]= 0.51 - 0.62 (m, 2H), 0.84 - 0.94 (m, 2H), 1.27 (s, 3H), 1.38 (s, 3H), 3.18 (tt, 1H), 4.52 (s, 2H), 5.64 (d, 1H), 7.54 - 7.61 (m, 3H), 7.63 - 7.68 (m, 1H), 7.70 (d, 1H), 7.76 - 7.84 (m, 3H), 8.67 (s, 1H), 8.96 (d, 1H), 12.27 (s, 1H).

### Beispiel 13

### Methyl-N-{[({3-(4-ehlorphenyl)-5-oxo-4-[(2S)--3,3,3-trifluor-2-hydroxypropyl]-4,5-dihydro-1H-1,2,4-triazol-1-yl}acetyl)amino][3-(trifluormethyl)phenyl]acetyl}-beta-alaninat (Diastereomerengemisch)

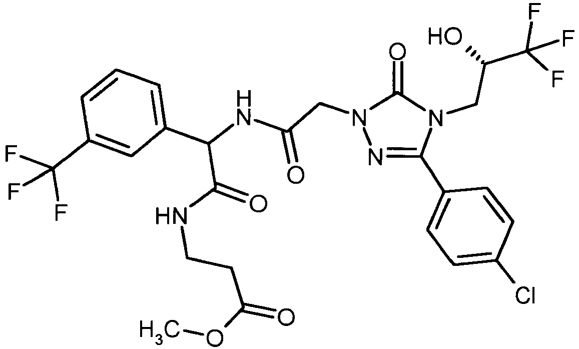

Analog zu Beispiel 1 wurde aus 50 mg (0.14 mmol) der Verbindung aus Beispiel 8A und 51 mg (0.15 mmol) der Verbindung aus Beispiel 47A die Titelverbindung hergestellt. Man erhielt 88 mg (99% d. Th.) der Titelverbindung als Diastereomerengemisch.

LC-MS [Methode 3]: Rₜ = 1.25 min; MS [ESIpos]: m/z = 652 (M+H)⁺

¹H-NMR (400MHz, DMSO-d₆): δ [ppm]= 2.44 (t, 2H), 3.25 - 3.31 (m, 2H), 3.52 (s, 3H), 3.82 (dd, 1H), 3.91 - 4.00 (m, 1H), 4.19 - 4.33 (m, 1H), 4.52 - 4.66 (m, 2H), 5.55 (d, 1H), 6.89 (t interpretiert als je 1 d pro Diastereomer, 1H), 7.55 - 7.80 (m, 8H), 8.60 (t interpretiert als je 1 d pro Diastereomer, 1H), 9.05 (d, 1H).

### Beispiel 14

### N-{[({3-(4-Chlorphenyl)-5-oxo-4-[(2S)-3,3,3-trifluor-2-hydroxypropyl]-4,5-dihydro-1H-1,2,4-triazol-1-yl}acetyl)amino][3-(trifluormethyl)phenyl]acetyl}-beta-alanin (Diastereomerengemisch)

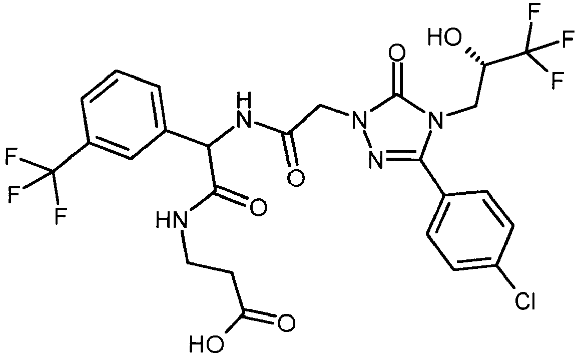

78 mg (0.12 mmol) der Verbindung aus Beispiel 13 wurden in 2.3 ml Methanol gelöst und mit 360 µl einer 1M wässrigen Lithiumhydroxidlösung (0.36 mmol) versetzt. Die Mischung wurde 1h bei RT gerührt, dann durch Zugabe 1N Salzsäure auf pH 2 gestellt, mit wenig DMSO verdünnt und per präparativer HPLC [Methode 6] getrennt. Man erhielt 67 mg (88 % d. Th.) der Titelverbindung.

LC-MS [Methode 3]: Rₜ = 1.17 min; MS [ESIpos]: m/z = 638 (M+H)⁺

¹H-NMR (400MHz, DMSO-d₆): δ [ppm]= 2.36 (t, 2H), 3.17 - 3.30 (m, 2H), 3.82 (dd, 1H), 3.96 (br. d, 1H), 4.17 - 4.35 (m, 1H), 4.50 - 4.68 (m, 2H), 5.57 (d, 1H), 6.89 (t interpretiert als je 1 d pro Diastereomer, 1H), 7.52 - 7.82 (m, 8H), 8.59 (t interpretiert als je 1 d pro Diastereomer, 1H), 9.04 (d, 1H), 12.23 (br. s., 1H).

### Beispiel 15

### Methyl-N-{({[3-(4-chlorphenyl)-4-cyclopropyl-5-oxo-4,5-dihydro-1H-1,2,4-triazol-1-yl]acetyl}amino)[3-(trifluormethyl)phenyl]acetyl}-beta-alaninat (Racemat)

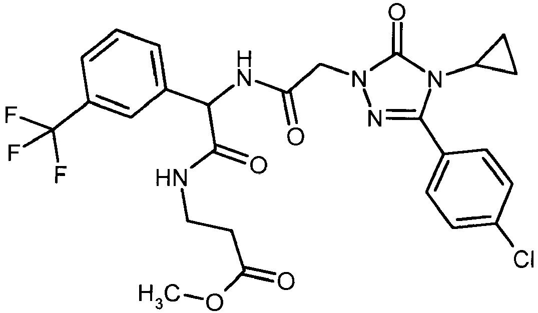

Eine Mischung aus 50 mg (0.14 mmol) [3-(4-Chlorphenyl)-4-cyclopropyl-5-oxo-4,5-dihydro-1*H-*1,2,4-triazol-1-yl]-essigsäure (Herstellung siehe WO 2007/134862, Beispiel 88A), 49 mg (0.26 mmol) EDC und 35 mg (0.26 mmol) HOBt in 1.7 ml DMF wurde 20 min bei RT gerührt. 64 mg (0.19 mmol) der Verbindung aus Beispiel 47A und 36 µl (0.20 mmol) N,N'-Diisopropylethylamin wurden hinzugefügt und die Mischung weiter über Nacht bei RT gerührt, dann per präparativer HPLC [Methode 6] getrennt. Die geeignete Fraktion wurde am Rotationsverdampfer eingeengt und der Rückstand am Hochvakuum getrocknet. Man erhielt 80 mg (81% d. Th.) der Titelverbindung.

LC-MS [Methode 3]: Rₜ = 1.21 min; MS [ESIpos]: m/z = 580 (M+H)⁺

¹H-NMR (400MHz, DMSO-d₆): δ [ppm]= 0.50 - 0.60 (m, 2H), 0.84 - 0.94 (m, 2H), 2.41 - 2.47 (m, 2H), 3.17 (tt, 1H), 3.25 - 3.31 (m, 2H), 3.52 (s, 3H), 4.44 - 4.59 (m [AB], 2H), 5.55 (s, 1H), 7.53 - 7.63 (m, 3H), 7.63 - 7.72 (m, 2H), 7.72 - 7.83 (m, 3H), 8.59 (t, 1H), 9.01 (d, 1H).

### Beispiel 16

### N-{({[3-(4-Chlorphenyl)-4-cyclopropyl-5-oxo-4,5-dihydro-1H-1,2,4-triazol-1-yl]acetyl}amino)[3-(trifluormethyl)phenyl]acetyl}-beta-alanin (Racemat)

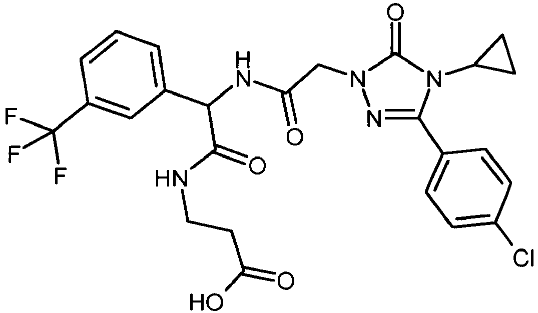

Analog zu Beispiel 2 wurden 52 mg (90 µmol) der Verbindung aus Beispiel 15 verseift und gereinigt. Man erhielt 33 mg (65 % d. Th.) der Titelverbindung.

LC-MS [Methode 3]: Pₜ = 1.13 min; MS [ESIpos]: m/z = 566 (M+H)⁺

¹H-NMR (400MHz, DMSO-d₆): δ [ppm]= 0.52 - 0.61 (m, 2H), 0.82 - 0.95 (m, 2H), 2.36 (t, 2H), 3.12 - 3.30 (m, 3H), 4.46 - 4.59 (m, 2H), 5.56 (d, 1H), 7.52 - 7.62 (m, 3H), 7.69 (t, 2H), 7.74 - 7.82 (m, 3H), 8.58 (t, 1H), 9.01 (d, 1H), 12.25 (br. s., 1H).

### Beispiel 17

### N³-{[({3-(4-Chlorphenyl)-5-oxo-4-[(2S)-3,3,3-trifluor-2-hydroxypropyl]-4,5-dihydro-1H-1,2,4-triazol-1-yl}acetyl)amino][3-(trifluormethyl)phenyl]acetyl}-beta-alaninamid (Diastereomer 1)

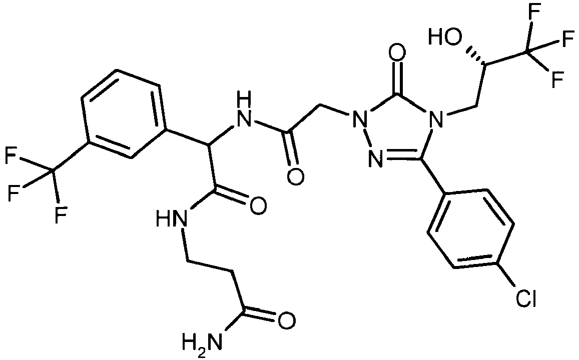

Eine Mischung aus 19 mg (51 µmol) der Verbindung aus Beispiel 8A, 20 mg (61 µmol) der Verbindung aus Beispiel 48A, 15 mg (77 µmol) EDC, 10 mg (77 µmol) HOBt und 13 µl (77 µmol) N,N'-Dilsopropylethylamin in 1 ml DMF wurde 20 min bei RT gerührt, dann mit 1ml 1N Salzsäure versetzt und per präparativer HPLC [Methode 6] getrennt. Die geeignete Fraktion wurde am Rotationsverdampfer eingeengt und der Rückstand am Hochvakuum getrocknet. Man erhielt 30 mg (92% d. Th.) der Titelverbindung.

LC-MS [Methode 1]: Rₜ = 1.79 min; MS [ESIpos]: m/z = 637 (M+H)⁺

¹H-NMR (400MHz, DMSO-d₆): δ [ppm]= 2.15 - 2.25 (m, 2H), 3.16 - 3.30 (m, 2H), 3.82 (dd, 1H), 3.96 (dd, 1H), 4.18 - 4.34 (m, 1H), 4.59 (s, 2H), 5.58 (d, 1H), 6.82 (br. s., 1H), 6.90 (d, 1H), 7.29 (br. s., 1H), 7.55 - 7.68 (m, 4H), 7.69 - 7.77 (m, 3H), 7.78 (s, 1H), 8.55 (t, 1H), 9.04 (d, 1H).

### Beispiel 18

### N³-{[({3-(4-Chlorphenyl)-5-oxo-4-[(2S)-3,3,3-trifluor-2-hydroxypropyl]-4,5-dihydro-1H-1,2,4-triazol-1-yl}acetyl)amino][3-(trifluormethyl)phenyl]acetyl}-beta-alaninamid (Diastereomer 2)

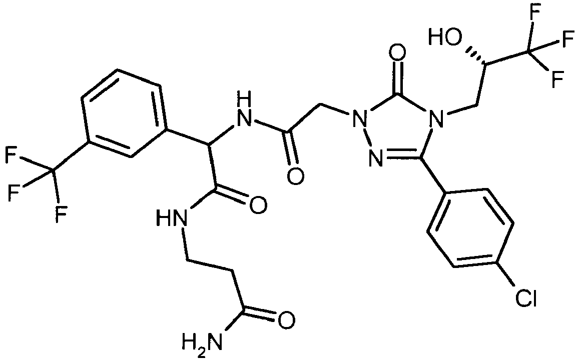

Analog zu Beispiel 17 wurde aus 19 mg (51 µmol) der Verbindung aus Beispiel 8A und 25 mg (61 µmol) der Verbindung aus Beispiel 49A die Titelverbindung erhalten. Ausbeute: 19 mg (58% d. Th.).

LC-MS [Methode 1]: Rₜ = 1.77 min; MS [ESIpos]: m/z = 637 (M+H)⁺

¹H-NMR (400MHz, DMSO-d₆): δ [ppm]= 2.13 - 2.26 (m, 2H), 3.16 - 3.32 (m, 2H), 3.82 (dd, 1H), 3.96 (dd, 1H), 4.19 - 4.33 (m, 1H), 4.53 - 4.65 (m [AB], 2H), 5.57 (d, 1H), 6.82 (br. s., 1H), 6.89 (d, 1H), 7.29 (br. s., 1H), 7.62 (d, 4H), 7.73 (s, 3H), 7.77 - 7.81 (m, 1H), 8.55 (t, 1H), 9.04 (d, 1H).

### Beispiel 19

### N³-{(2R)-2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propanoyl}-beta-alaninamid

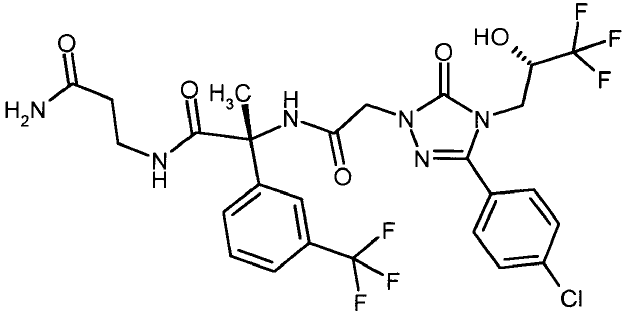

Analog zu Beispiel 17 wurde aus 40 mg (69 µmol) der Verbindung aus Beispiel 16A und 25 mg (103 µmol) beta-Alaninamid-Hydrochlorid die Titelverbindung erhalten. Ausbeute: 38 mg (85% d. Th.).

LC-MS [Methode 1]: Fₜ = 1.83 min; MS [ESIpos]: m/z = 651 (M+H)⁺

¹H-NMR (400MHz, DMSO-d₆): δ [ppm]= 1.84 (s, 3H), 2.09 - 2.22 (m, 2H), 3.13 - 3.28 (m, 2H), 3.82 (dd, 1H), 3.96 (dd, 1H), 4.19 - 4.36 (m, 1H), 4.58 (s, 2H), 6.76 (br. s., 1H), 6.90 (d, 1H), 7.25 (br. s., 1H), 7.51 - 7.58 (m, 1H), 7.60 - 7.66 (m, 3H), 7.69 (d, 1H), 7.75 (d, 3H), 7.90 (t, 1H), 8.69 (s, 1H).

### Beispiel 20

### Methyl-N-{(2R)-2-[({3-(4-chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propanoyl}-2-methylalaninat

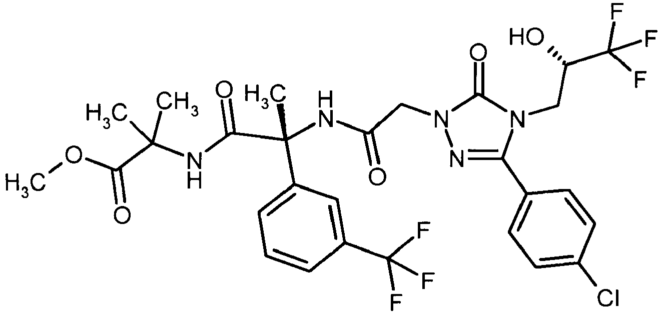

Analog zu Beispiel 17 wurde aus 40 mg (69 µmol) der Verbindung aus Beispiel 16A und 16 mg (103 µmol) Methyl-2-methylalaninat-Hydrochlorid die Titelverbindung erhalten. Ausbeute: 36 mg (77% d. Th.).

LC-MS [Methode 1]: Rₜ = 2.24 min; MS [ESIpos]: m/z = 680 (M+H)⁺

¹H-NMR (400MHz, DMSO-d₆): δ [ppm]= 1.26 (s, 3H), 1.29 (s, 3H), 1.82 (s, 3H), 3.52 (s, 3H), 3.82 (dd, 1H), 3.95 (dd, 1H), 4.17 - 4.32 (m, 1H), 4.52 - 4.63 (m [AB], 2H), 6.89 (d, 1H), 7.56 - 7.67 (m, 4H), 7.68 - 7.79 (m, 4H), 7.98 (s, 1H), 8.69 (s, 1H).

### Beispiel 21

### N-{(2R)-2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propanoyl}-2-methylalanin

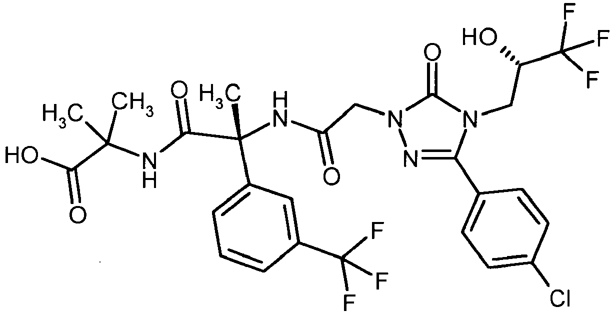

32 mg (47 µmol) der Verbindung aus Beispiel 20 wurden in 1.6 ml Acetonitril gelöst und mit 235 µl (235 µmol) einer 1M wässrigen Lithiumhydroxidlösung versetzt. Die Mischung wurde über Nacht bei RT gerührt, dann mit 1 ml 1N Salzsäure versetzt und per präparativer HPLC [Methode 6] getrennt. Man erhielt 31 mg (99 % d. Th.) der Titelverbindung.

¹H-NMR (400MHz, DMSO-d₆): δ [ppm]= 1.27 (s, 3H), 1.29 (s, 3H), 1.80 (s, 3H), 3.82 (dd, 1H), 3.95 (dd, 1H), 4.19 - 4.33 (m, 1H), 4.51 - 4.64 (m [AB], 2H), 6.90 (d, 1H), 7.52 - 7.58 (m, 1H), 7.59 - 7.66 (m, 3H), 7.69 - 7.78 (m, 4H), 7.82 (s, 1H), 8.71 (s, 1H), 12.29 (s, 1H).

### Beispiel 22

### N²-{(2R)-2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propanoyl}-2-methylalaninamid

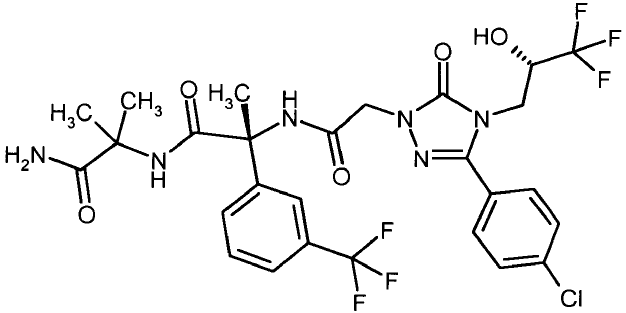

Eine Mischung aus 31 mg (47 µmol) der Verbindung aus Beispiel 21, 13 mg (70 µmol) EDC und 9.4 mg (70 µmol) HOBt in 2 ml DMF wurde 20 min bei RT gerührt, dann in 2 ml Ammoniak (35%-ige Lösung in Wasser) gegossen. Das Reaktionsgemisch wurde 30 min bei RT gerührt, dann kurz am Rotationsverdampfer eingeengt, mit 1 N Salzsäure sauer gestellt und per präparativer HPLC [Methode 6] getrennt. Die geeignete Fraktion wurde am Rotationsverdampfer eingeengt und der Rückstand am Hochvakuum getrocknet. Man erhielt 29 mg (92% d. Th.) der Titelverbindung.

LC-MS [Methode 3]: Rₜ = 1.21 min; MS [ESIpos]: m/z = 665 (M+H)⁺

¹H-NMR (400MHz, DMSO-d₆): δ [ppm]= 1.27 (s, 3H), 1.30 (s, 3H), 1.78 (s, 3H), 3.83 (dd, 1H), 3.96 (dd, 1H), 4.20 - 4.36 (m, 1H), 4.56 - 4.71 (m [AB], 2H), 6.85 (s, 2H), 6.89 (d, 1H), 7.55 - 7.69 (m, 4H), 7.73 - 7.84 (m, 5H), 8.91 (s, 1H).

### Beispiel 23

### Methyl-N-{[({3-(4-chlorphenyl)-5-oxo-4-[(2S)-3,3,3-trifluor-2-hydroxypropyl]-4,5-dihydro-1H-1,2,4-triazol-1-yl}acetyl)amino][3-(trifluormethyl)phenyl]acetyl}-2-methylalaninat (Diastereomerengemisch)

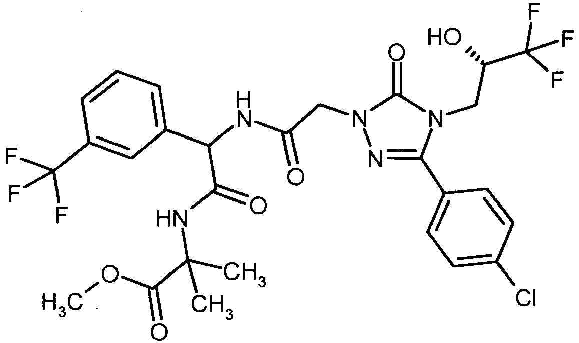

Analog zu Beispiel 1 wurde aus 398 mg (1.09 mmol) der Verbindung aus Beispiel 8A und 457 mg (1.20 mmol) der Verbindung aus Beispiel 46A die Titelverbindung hergestellt. Man erhielt 664 mg (91% d. Th.) der Titelverbindung als Diastereomerengemisch.

LC-MS [Methode 3]: Rₜ = 1.32 min; MS [ESIpos]: m/z = 666 (M+H)⁺

¹H-NMR (400MHz, DMSO-d₆): δ [ppm]= 1.27 (s, 3H), 1.40 (s, 3H), 3.44 (d, interpretiert als 3.440 und 3.444, je 1 s pro Diastereomer, insgesamt 3H), 3.83 (dd, 1H), 3.96 (br. dd, 1H), 4.20 - 4.33 (m, 1H), 4.52 - 4.65 (m, 2H), 5.64 (d, 1H), 6.86 - 6.92 (dd, interpretiert als 6.89 und 6.90, je 1 d pro Diastereomer, 1H), 7.58 - 7.80 (m, 7H), 8.84 (s, 1H), 9.02 (d, 1H).

### Beispiel 24

### N-{[({3-(4-Chlorphenyl)-5-oxo-4-[(2S)-3,3,3-trifluor-2-hydroxypropyl]-4,5-dihydro-1H-1,2,4-triazol-1-yl}acetyl)amino] [3-(trifluormethyl)phenyl]acetyl}-2-methylalanin (Diastereomerengemisch)

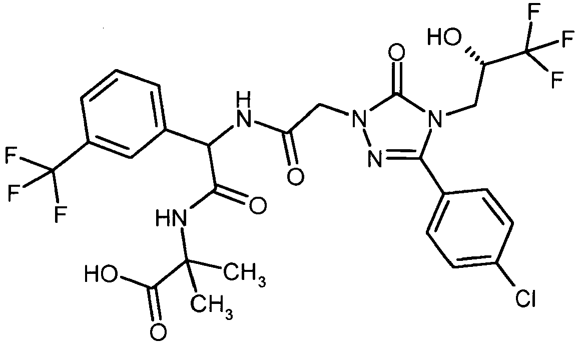

Analog zu Beispiel 2 wurden 69 mg (0.104 mmol) der Verbindung aus Beispiel 23 verseift und gereinigt. Man erhielt 63 mg (93% d. Th.) der Titelverbindung als Diastereomerengemisch.

LC-MS [Methode 3]: Rₜ = 1.22 min; MS [ESIpos]: m/z = 652 (M+H)⁺

Durch präparative HPLC an einer chiralen Phase [Methode 14a] wurden die beiden Diastereomere getrennt: siehe Beispiel 25 und Beispiel 26.

### Beispiel 25

### N-{[({3-(4-Chlorphenyl)-5-oxo-4-[(2S)-3,3,3-trifluor-2-hydroxypropyl]-4,5-dihydro-1H-1,2,4-triazol-1-yl}acetyl)amino][3-(trifluormethyl)phenyl]acetyl}-2-methylalanin (Diastereomer 1)

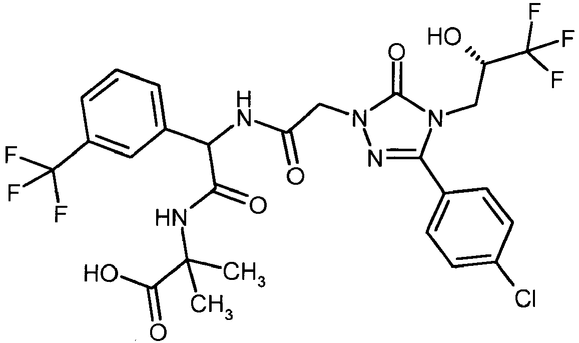

Zuerst eluierendes Diastereomer aus der Diastereomerentrennung von 63 mg der Verbindung aus Beispiel 24 nach Methode 14a. Nach der Chromatographie an der chiralen Phase wurde das erhaltene Produkt noch von Lösungsmittelkontaminanten durch präparative HPLC [Methode 6] gereinigt. Man erhielt 15 mg der Titelverbindung.

Chirale analytische HPLC [Methode 15a]: Rₜ = 5.02 min.

LC-MS [Methode 1]: Rₜ = 1.99 min; MS [ESIpos]: m/z = 652 (M+H)⁺

¹H-NMR (400MHz, DMSO-d₆): δ [ppm]= 1.28 (s, 3H), 1.38 (s, 3H), 3.83 (dd, 1H), 3.96 (dd, 1H), 4.19 - 4.34 (m, 1H), 4.59 (s, 2H), 5.66 (d, 1H), 6.91 (d, 1H), 7.55 - 7.77 (m, 7H), 7.79 (s, 1H), 8.62 - 8.73 (m, 1H), 9.00 (d, 1H), 12.28 (s, 1H).

### Beispiel 26

### N-{[({3-(4-Chlorphenyl)-5-oxo-4-[(2S)-3,3,3-trifluor-2-hydroxypropyl]-4,5-dihydro-1H-1,2,4-triazol-1-yl}acetyl)amino][3-(trifluormethyl)phenyl]acetyl}-2-methylalanin (Diastereomer 2)

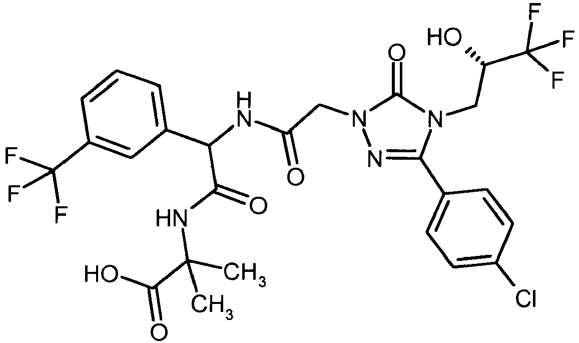

Zuletzt eluierendes Diastereomer aus der Diastereomerentrennung von 63 mg der Verbindung aus Beispiel 24 nach Methode 14a. Nach der Chromatographie an der chiralen Phase wurde das erhaltene Produkt noch von Lösungsmittelkontaminanten durch präparative HPLC [Methode 6] gereinigt. Man erhielt 12 mg der Titelverbindung.

Chirale analytische HPLC [Methode 15a]: Rₜ = 6.77 min.

LC-MS [Methode 1]: Rₜ = 1.99 min; MS [ESIpos]: m/z = 652 (M+H)⁺

¹H-NMR (400MHz, DMSO-d₆): δ [ppm]= 1.27 (s, 3H), 1.38 (s, 3H), 3.83 (dd, 1H), 3.97 (dd, 1H), 4.18 - 4.34 (m, 1H), 4.53 - 4.65 (m [AB], 2H), 5.66 (d, 1H), 6.89 (d, 1H), 7.56 - 7.77 (m, 7H), 7.79 (s, 1H), 8.67 (s, 1H), 9.00 (d, 1H), 12.27 (s, 1H).

### Beispiel 27

### N²-{[({3-(4-Chlorphenyl)-5-oxo-4-[(2S)-3,3,3-trifluor-2-hydroxypropyl]-4,5-dihydro-1H-1,2,4-triazol-1-yl}acetyl)amino] [3-(trifluormethyl)phenyl]acetyl}-2-methylalaninamid (Diastereomerengemisch)

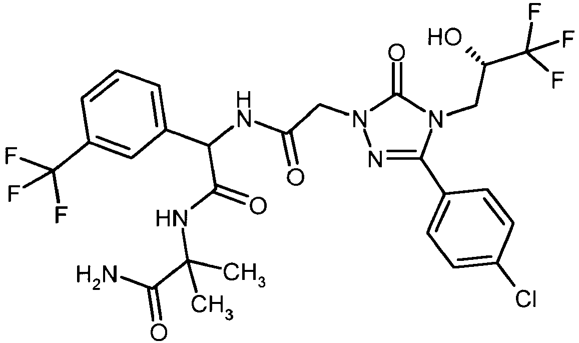

Analog zu Beispiel 1 wurde aus 195 mg (0.53 mmol) der Verbindung aus Beispiel 8A und 198 mg (0.58 mmol) der Verbindung aus Beispiel 63A die Titelverbindung als Diastereomerenmischung erhalten: 330 mg (95% d. Th.).

LC-MS [Methode 4]: Rₜ = 2.22 min; MS [ESIpos]: m/z = 651 (M+H)⁺

Durch präparative HPLC an einer chiralen Phase [Methode 14b] wurden die beiden Diastereomere getrennt: siehe Beispiel 28 und Beispiel 29.

### Beispiel 28

### N²-{[({3-(4-Chlorphenyl)-5-oxo-4-[(2S)-3,3,3-trifluor-2-hydroxypropyl]-4,5-dihydro-1H-1,2,4-triazol-1-yl}acetyl)amino][3-(trifluormethyl)phenyl]acetyl}-2-methylalaninamid (Diastereomer 1)

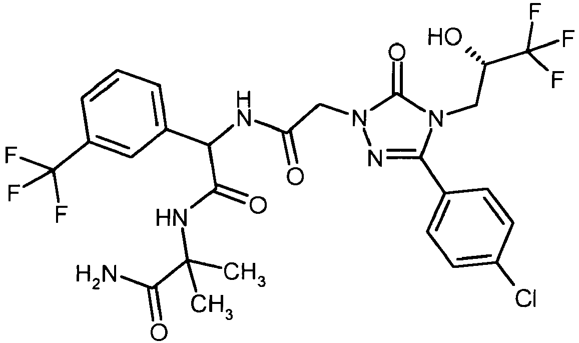

Zuerst eluierendes Diastereomer aus der Diastereomerentrennung von 320 mg der Verbindung aus Beispiel 27 nach Methode 14b. Nach der Chromatographie an der chiralen Phase wurde das erhaltene Produkt noch von Lösungsmittelkontaminanten durch präparative HPLC [Methode 6] gereinigt. Man erhielt 130 mg der Titelverbindung.

Chirale analytische HPLC [Methode 15b]: Rₜ = 3.52 min.

LC-MS [Methode 1]: Rₜ = 1.90 min; MS [ESIpos]: m/z = 651 (M+H)⁺

¹H-NMR (400MHz, DMSO-d₆): δ [ppm]= 1.28 (s, 3H), 1.33 (s, 3H), 3.83 (dd, 1H), 3.96 (dd, 1H), 4.19 - 4.34 (m, 1H), 4.59 (s, 2H), 5.60 (d, 1H), 6.89 (br. s, 1H), 6.91 (d, 1H), 6.97 (br. s., 1H), 7.56 - 7.68 (m, 4H), 7.71 - 7.77 (m, 3H), 7.81 (s, 1H), 8.43 (s, 1H), 8.97 (d, 1H).

### Beispiel 29

### N²-{[({3-(4-Chlorphenyl)-5-oxo-4-[(2S)-3,3,3-trifluor-2-hydroxypropyl]-4,5-dihydro-1H-1,2,4-triazol-1-yl}acetyl)amino][3-(trifluormethyl)phenyl]acetyl}-2-methylalaninamid (Diastereomer 2)

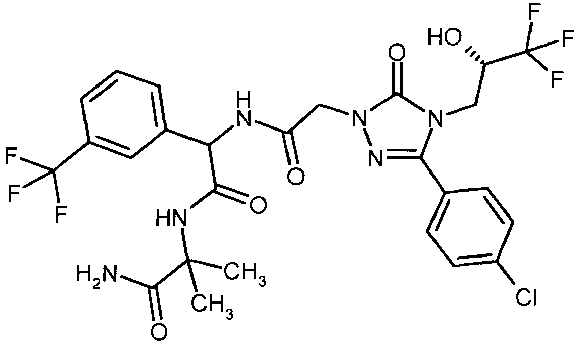

Zuletzt eluierendes Diastereomer aus der Diastereomerentrennung von 320 mg der Verbindung aus Beispiel 27 nach Methode 14b. Nach der Chromatographie an der chiralen Phase wurde das erhaltene Produkt noch von Lösungsmittelkontaminanten durch präparative HPLC [Methode 6] gereinigt. Man erhielt 141 mg der Titelverbindung.

Chirale analytische HPLC [Methode 15b]: Rₜ = 5.01 min.

LC-MS [Methode 1]: Rₜ = 1.89 min; MS [ESIpos]: m/z = 651 (M+H)⁺

¹H-NMR (400MHz, DMSO-d₆): δ [ppm]= 1.27 (s, 3H), 1.33 (s, 3H), 3.83 (dd, 1H), 3.97 (dd, 1H), 4.19 - 4.33 (m, 1H), 4.52 - 4.65 (m [AB], 2H), 5.61 (s, 1H), 6.89 (br. s, 2H), 6.97 (s, 1H), 7.55 - 7.77 (m, 7H), 7.81 (s, 1H), 8.44 (s, 1H), 8.96 (d, 1H).

### Beispiel 30

### N²-{({[3-(4-Chlorphenyl)-4-(2-fluorbenzyl)-5-oxo-4,5-dihydro-1H-1,2,4-triazol-1-yl]acetyl}amino)[2-(trifluormethyl)phenyl]acetyl}-2-methylalaninamid (Racemat)

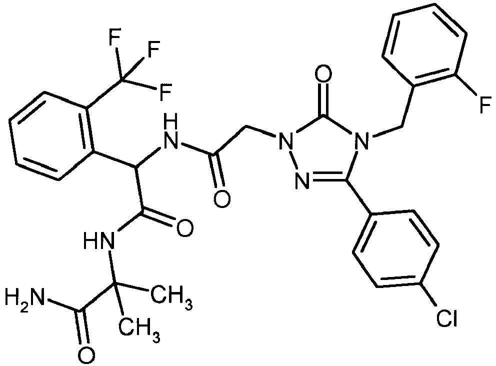

Analog zu Beispiel 1 wurden 25 mg (69 µmol) [3-(4-Chlorphenyl)-4-(2-fluorbenzyl)-5-oxo-4,5-dihydro-1H-1,2,4-triazol-1-yl]essigsäure (Herstellung siehe WO 2007/134862, Beispiel 156A) und 28 mg (76 µmol) der Verbindung aus Beispiel 62A eingesetzt. Nach HPLC-Reinigung [Methode 7] erhielt man 28 mg (63% d. Th.) der Titelverbindung.

LC-MS [Methode 5]: Rₜ = 1.01 min; MS [ESIpos]: m/z = 647 (M+H)⁺

¹H-NMR (400MHz, DMSO-d₆): δ [ppm]= 1.35 (s, 3H), 1.37 (s, 3H), 4.51 - 4.68 (m [AB], 2H), 5.02 (s, 2H), 5.65 (d, 1H), 7.00 - 7.18 (m, 5H), 7.26 - 7.34 (m, 1H), 7.49 - 7.59 (m, 5H), 7.66 - 7.77 (m, 3H), 8.03 (s, 1H), 9.15 (d, 1H).

### Beispiel 31

### N²-{[({3-(4-Chlorphenyl)-5-oxo-4-[(1E)-3,3,3-trifluorprop-1-en-1-yl]-4,5-dihydro-1H-1,2,4-triazol-1-yl}acetyl)amino][2-(trifluormethyl)phenyl]acetyl}-2-methylalaninamid (Racemat)

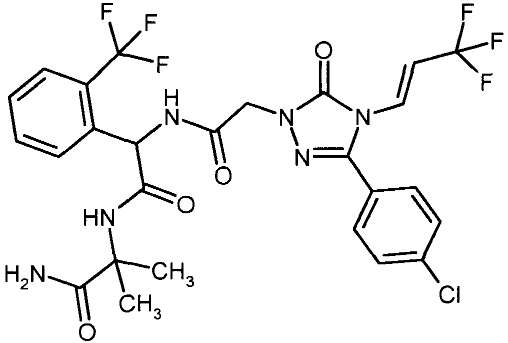

Analog zu Beispiel 1 wurden 25 mg (72 µmol) der Verbindung aus Beispiel 11A und 29 mg (79 µmol) der Verbindung aus Beispiel 62A eingesetzt. Nach HPLC-Reinigung [Methode 7] erhielt man 32 mg (70% d. Th.) der Titelverbindung.

LC-MS [Methode 5]: Rₜ = 1.04 min; MS [ESIpos]: m/z = 633 (M+H)⁺

¹H-NMR(400MHz, DMSO-d₆): δ [ppm]= 1.36 (s, 3H), 1.38 (s, 3H), 4.52 - 4.68 (m [AB], 2H), 5.65 (d, 1H), 6.85 (dq, 1H), 7.03 (br. s, 1H), 7.06 (br. s, 1H), 7.17 (dq, 1H), 7.55 (t, 1H), 7.61 - 7.78 (m, 7H), 8.02 (s, 1H), 9.16 (d, 1H).

### Beispiel 32

### N²-{({[3-(4-Chlorphenyl)-5-oxo-4-(3,3,3-trifluorpropyl)-4,5-dihydro-1H-1,2,4-triazol-1-yl]acetyl}amino)[2-(trifluormethyl)phenyl]acetyl}-2-methylalaninamid (Racemat)

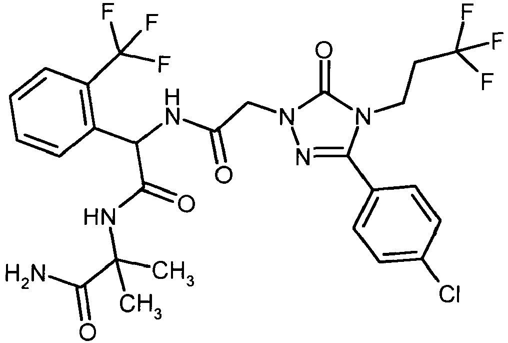

Eine Lösung von 25 mg (39 µmol) der Verbindung aus Beispiel 31 in 10 ml Methanol wurde in einem mit einer Platin-Kartusche (5% Pt/C) ausgestatteten H-Cube (Durchfluss-Hydrierapparat der Firma Thales Nano, Budapest, Modell HC-2-SS) bei einem Durchfluss von 1 ml / min, bei 60°C und Normaldruck Wasserstoff hydriert. Die resultierende Lösung wurde am Rotationsverdampfer vom Methanol befreit, der Rückstand in 2 ml Acetonitril gelöst und durch präparative HPLC [Methode 7] gereinigt. Es wurden 8 mg (32 % d. Th.) der Titelverbindung erhalten.

LC/MS [Methode 5]: Rₜ = 0.96 min; m/z = 635 (M+H)⁺

¹H-NMR (400MHz, DMSO-d₆): δ [ppm]= 1.35 (s, 3H), 1.38 (s, 3H), 2.56 - 2.66 (m, 2H), 3.98 (t, 2H), 4.46 - 4.62 (m [AB], 2H), 5.63 (d, 1H), 7.03 (br.s, 1H), 7.04 (br.s, 1H), 7.51 - 7.58 (m, 1H), 7.60 - 7.77 (m, 7H), 8.01 (s, 1H), 9.11 (d, 1H).

### Beispiel 33

### N²-[(2-Chlorphenyl)({[3-(4-chlorphenyl)-4-(2-fluorbenzyl)-5-oxo-4,5-dihydro-1H-1,2,4-triazol-1-yl]acetyl}amino)acetyl]-2-methylalaninamid (Racemat)

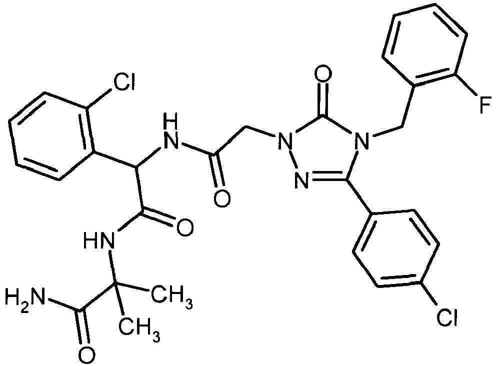

Analog zu Beispiel 1 wurden 25 mg (69 µmol) [3-(4-Chlorphenyl)-4-(2-fluorbenzyl)-5-oxo-4,5-dihydro-1H-1,2,4-triazol-1-yl]essigsäure (Herstellung siehe WO 2007/134862, Beispiel 156A) und 26 mg (76 µmol) der Verbindung aus Beispiel 64A eingesetzt. Nach HPLC-Reinigung [Methode 7] erhielt man 35 mg (83% d. Th.) der Titelverbindung.

LC-MS [Methode 5]: Rₜ = 0.97 min; MS [ESIpos]: m/z = 613 (M+H)⁺

¹H-NMR (400MHz, DMSO-d₆): δ [ppm]= 1.35 (s, 3H), 1.38 (s, 3H), 4.53 - 4.66 (m [AB], 2H), 5.03 (s, 2H), 5.70 (d, 1H), 6.97 (br. s., 1H), 7.01 (br. s., 1H), 7.02 - 7.18 (m, 3H), 7.26 - 7.39 (m, 3H), 7.44 - 7.51 (m, 2H), 7.53 (s, 4H), 8.12 (s, 1H), 9.01 (d, 1H).

### Beispiel 34

### N'-{(2-Chlorphenyl)[({3-(4-chlorphenyl)-5-oxo-4-[(1E)-3,3,3-trifluorprop-1-en-1-yl]-4,5-dihydro-1H-1,2,4-triazol-1-yl}acetyl)amino]acetyl}-2-methylalaninamid (Racemat)

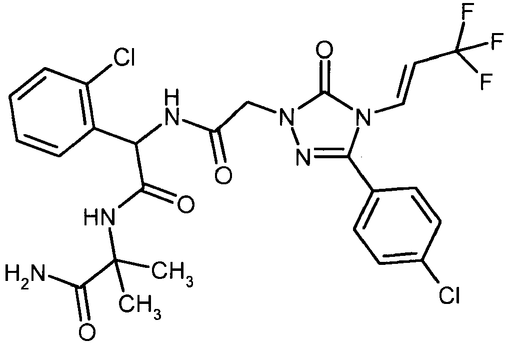

Analog zu Beispiel 1 wurden 25 mg (72 µmol) der Verbindung aus Beispiel 11A und 27 mg (79 µmol) der Verbindung aus Beispiel 64A eingesetzt. Nach HPLC-Reinigung [Methode 7] erhielt man 34 mg (79% d. Th.) der Titelverbindung.

LC-MS [Methode 5]: Rₜ = 1.01 min; MS [ESIpos]: m/z = 599 (M+H)⁺

¹H-NMR(400MHz, DMSO-d₆): δ [ppm]= 1.35 (s, 3H), 1.39 (s, 3H), 4.54 - 4.66 (m [AB], 2H), 5.69 (d, 1H), 6.85 (dq, 1H), 6.96 (br. s., 1H), 7.02 (br. s., 1H), 7.17 (dq, 1H), 7.32 - 7.39 (m, 2H), 7.44 - 7.51 (m, 2H), 7.61 - 7.70 (m, 4H), 8.12 (s, 1H), 9.02 (d, 1H).

### Beispiel 35

### N²-[(2-Chlorphenyl)({[3-(4-chlorphenyl)-5-oxo-4-(3,3,3-trifluorpropyl)-4,5-dihydro-1H-1,2,4-triazol-1-yl]acetyl}amino)acetyl]-2-methylalaninamid (Racemat)

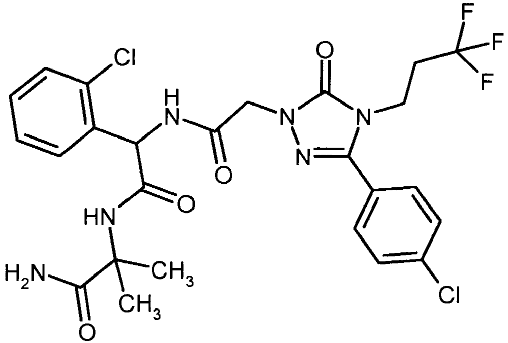

Analog zu Beispiel 32 wurden 20 mg (33 µmol) der Verbindung aus Beispiel 34 hydriert und gereinigt. Man erhielt 11 mg (54% d. Th.) der Titelverbindung.

LC-MS [Methode 5]: Rₜ = 0.98 min; MS [ESIpos]: m/z = 601 (M+H)⁺

¹H-NMR(400MHz, DMSO-d₆): δ [ppm]= 1.35 (s, 3H), 1.38 (s, 3H), 2.59 - 2.66 (m, 2H), 3.98 (t, 2H), 4.48 - 4.62 (m [AB], 2H), 5.68 (d, 1H), 6.96 (br. s, 1H), 7.01 (br. s, 1H), 7.31 - 7.40 (m, 2H), 7.42 - 7.52 (m, 2H), 7.65 (q, 4H), 8.10 (s, 1H), 8.97 (d, 1H).

### Beispiel 36

### N²-[({[3-(4-Chlorphenyl)-4-(2-fluorbenzyl)-5-oxo-4,5-dihydro-1H-1,2,4-triazol-1-yl]acetyl}amino)(2,3-dichlorphenyl)acetyl]-2-methylalaninamid (Racemat)

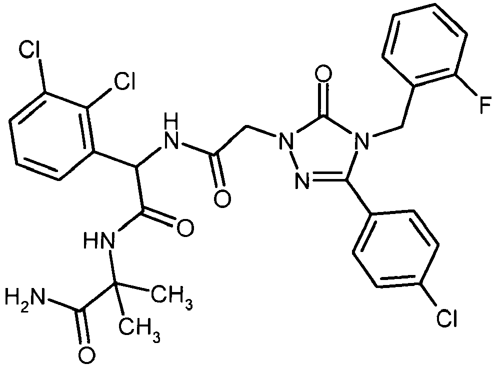

Analog zu Beispiel 1 wurden 25 mg (69 µmol) [3-(4-Chlorphenyl)-4-(2-fluorbenzyl)-5-oxo-4,5-dihydro-1H-1,2,4-triazol-1-yl]essigsäure (Herstellung siehe WO 2007/134862, Beispiel 156A) und 28 mg (76 µmol) der Verbindung aus Beispiel 65A umgesetzt. Nach HPLC-Reinigung [Methode 7] erhielt man 35 mg (78% d. Th.) der Titelverbindung.

LC-MS [Methode 5]: Rₜ = 1.03 min; MS [ESIpos]: m/z = 647 (M+H)⁺

¹H-NMR (400MHz, DMSO-d₆): δ [ppm]= 1.36 (s, 3H), 1.37 (s, 3H), 4.54 - 4.68 (m [AB], 2H), 5.02 (s, 2H), 5.75 (d, 1H), 6.97 (br. s., 1H), 6.99 (br. s., 1H), 7.01 - 7.07 (m, 1H), 7.07 - 7.19 (m, 2H), 7.26 - 7.34 (m, 1H), 7.39 (t, 1H), 7.46 (dd, 1H), 7.53 (s, 4H), 7.62 (dd, 1H), 8.21 (s, 1H), 9.08 (d, 1H).

### Beispiel 37

### N²-{[({3-(4-Chlorphenyl)-5-oxo-4-[(1E)-3,3,3-trifluorprop-1-en-1-yl]-4,5-dihydro-1H-1,2,4-triazol-1-yl}acetyl)amino](2,3-dichlorphenyl)acetyl}-2-methylalaninamid (Racemat)

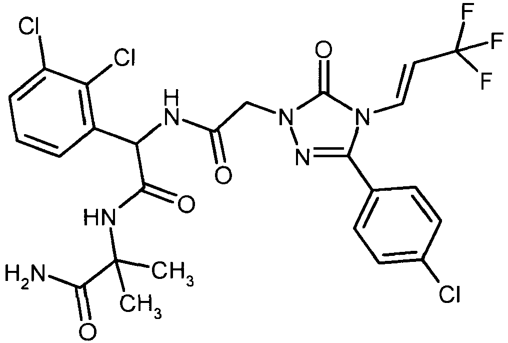

Analog zu Beispiel 1 wurden 25 mg (72 µmol) der Verbindung aus Beispiel 11A und 29 mg (79 µmol) der Verbindung aus Beispiel 65A umgesetzt. Nach HPLC-Reinigung [Methode 7] erhielt man 35 mg (77% d. Th.) der Titelverbindung.

LC-MS [Methode 5]: Rₜ = 1.06 min; MS [ESIpos]: m/z = 633 (M+H)⁺

¹H-NMR (400MHz, DMSO-d₆): δ [ppm]= 1.35 (s, 3H), 1.38 (s, 3H), 4.55 - 4.66 (m [AB], 2H), 5.74 (d, 1H), 6.85 (dq, 1H), 6.96 (br. s., 1H), 7.00 (br. s., 1H), 7.14 - 7.21 (m, 1H), 7.39 (t, 1H), 7.45 (dd, 1H), 7.60 - 7.70 (m, 5H), 8.22 (s, 1H), 9.09 (d, 1H).

### Beispiel 38

### Methyl-N-{(3S)-3-[({3-(4-chlorphenyl)-5-oxo-4-[(2S)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalaninat

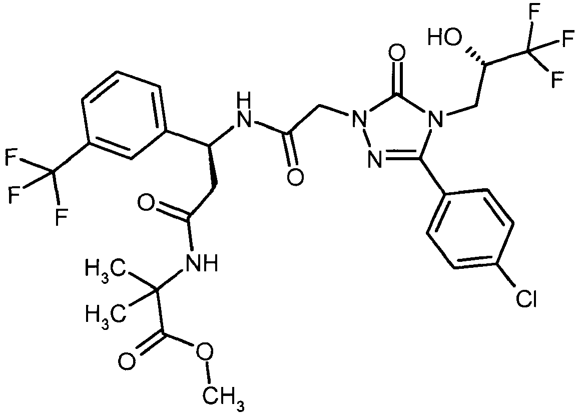

Analog zu Beispiel 1 wurde aus 93 mg (0.26 mmol) der Verbindung aus Beispiel 8A und 108 mg (0.29 mmol) der Verbindung aus Beispiel 67A die Titelverbindung erhalten. Ausbeute: 134 mg (77% d. Th.).

LC-MS [Methode 3]: Rₜ = 1.32 min; MS [ESIpos]: m/z = 680 (M+H)⁺

¹H-NMR (400MHz, DMSO-d₆): δ [ppm]= 1.18 (s, 3H), 1.23 (s, 3H), 2.56 - 2.67 (m, 2H), 3.44 (s, 3H), 3.82 (dd, 1H), 3.96 (dd, 1H), 4.21 - 4.34 (m, 1H), 4.48 (s, 2H), 5.26 (q, 1H), 6.89 (d, 1H), 7.53 - 7.66 (m, 6H), 7.75 (d, 2H), 8.29 (s, 1H), 8.81 (d, 1H).

### Beispiel 39

### N-{ (3S)-3-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalanin

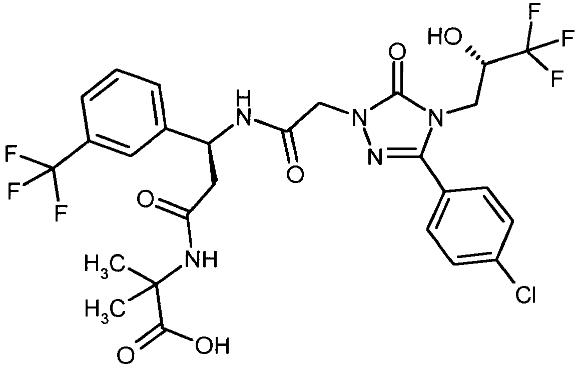

Eine Lösung von 130 mg (0.19 mmol) der Verbindung aus Beispiel 38 in 5 ml Acetonitril wurde mit 478 µl (478 µmol) einer 1N wässrigen Lösung von Lithiumhydroxid versetzt und über Nacht bei RT gerührt. Nach Zugabe von 1 ml 1N Salzsäure wurde die gesamte Mischung über präparative HPLC [Methode 6] getrennt. Man erhielt 103 mg (81% d. Th.) der Titelverbindung.

LC-MS [Methode 1]: Rₜ = 1.97 min; MS [ESIpos]: m/z = 666 (M+H)⁺

¹H-NMR (400MHz, DMSO-d₆): δ [ppm]= 1.18 (s, 3H), 1.22 (s, 3H), 2.53 - 2.66 (m, 2H), 3.82 (dd, 1H), 3.92 - 4.01 (m, 1H), 4.20 - 4.33 (m, 1H), 4.48 (s, 2H), 5.27 (q, 1H), 6.90 (d, 1H), 7.50 - 7.57 (m, 1H), 7.57 - 7.65 (m, 5H), 7.75 (d, 2H), 8.10 (s, 1H), 8.81 (d, 1H), 12.15 (s, 1H).

### Beispiel 40

### N²-{(3S)-3-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-3-[3-(trifluormethyl)phenyl]propanoyl}-2-methylalaninamid

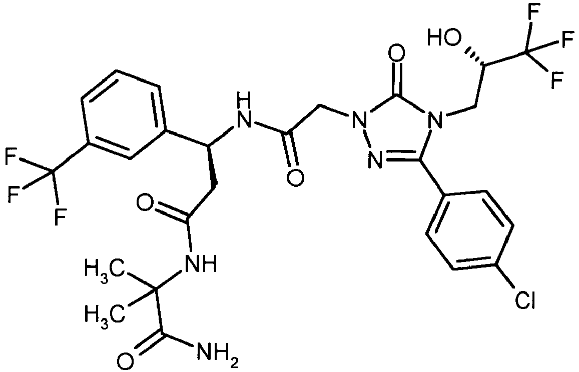

Eine Mischung aus 50 mg (75 µmol) der Verbindung aus Beispiel 39, 29 mg (0.15 mmol) EDC und 20 mg (0.15 mmol) HOBt in 2 ml DMF wurde 20 min bei RT gerührt, dann in 5 ml Ammoniak (35%-ige Lösung in Wasser) gegossen. Das Reaktionsgemisch wurde 30 min bei RT gerührt, dann am Rotationsverdampfer eingeengt, mit 2.5 N Salzsäure sauer gestellt und per präparativer HPLC [Methode 6] getrennt. Die geeignete Fraktion wurde am Rotationsverdampfer eingeengt und der Rückstand im Hochvakuum getrocknet. Man erhielt 42 mg (84% d. Th.) der Titelverbindung.

LC-MS [Methode 1]: Rₜ = 1.85 min; MS [ESIpos]: m/z = 665 (M+H)⁺

¹H-NMR (400MHz, DMSO-d₆): δ [ppm]= 1.21 (s, 3H), 1.22 (s, 3H), 2.56 - 2.68 (m, 2H), 3.82 (dd, 1H), 3.96 (dd, 1H), 4.19 - 4.33 (m, 1H), 4.42 - 4.54 (m [AB], 2H), 5.23 - 5.32 (m, 1H), 6.79 (br. s., 1H), 6.86 - 6.96 (m, 1H), 7.52 - 7.58 (m, 1H), 7.58 - 7.66 (m, 6H), 7.75 (d, 2H), 7.90 (s, 1H), 8.82 (d, 1H).

### Beispiel 41

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(1-hydroxy-2-methylpropan-2-yl)-2-[3-(trifluormethyl)phenyl]acetamid (Diastereomer 1)

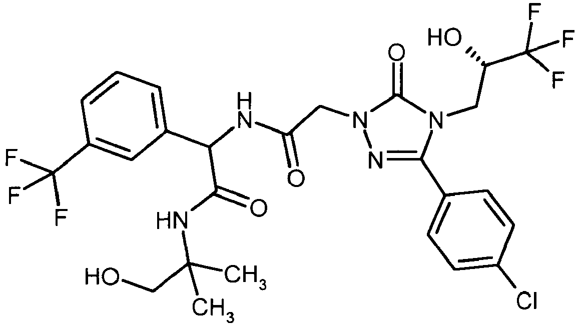

Eine Mischung aus 50 mg (0.14 mmol) der Verbindung aus Beispiel 8A, 67 mg (0.21 mmol) der Verbindung aus Beispiel 50A, 39 mg (0.21 mmol) EDC, 28 mg (0.21 mmol) HOBt und 48 µl (0.27 mmol) N,N'-Diisopropylethylamin in 1.3 ml DMF wurde über Nacht bei RT gerührt, dann mit 1 ml 1N Salzsäure versetzt und per präparativer HPLC [Methode 6] getrennt. Die geeignete Fraktion wurde am Rotationsverdampfer eingeengt und der Rückstand im Hochvakuum getrocknet. Man erhielt 61 mg (70% d. Th.) der Titelverbindung.

LC-MS [Methode 4]: Rₜ = 2.38 min; MS [ESIpos]: m/z = 638 (M+H)⁺

¹H-NMR (400MHz, DMSO-d₆): δ [ppm]= 1.14 (s, 3H), 1.15 (s, 3H), 3.28 - 3-34 (m, 1H), 3.39 - 3.46 (m, 1H), 3.83 (dd, 1H), 3.97 (dd, 1H), 4.19 - 4.33 (m, 1H), 4.54 - 4.66 (m [AB], 2H), 4.78 (t, 1H), 5.63 (d, 1H), 6.89 (d, 1H), 7.56 - 7.68 (m, 4H), 7.69 - 7.76 (m, 3H), 7.79 (s, 1H), 7.99 (s, 1H), 8.93 (d, 1H).

### Beispiel 42

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(1-hydroxy-2-methylpropan-2-yl)-2-[3-(trifluormethyl)phenyl]acetamid (Diastereomer 2)

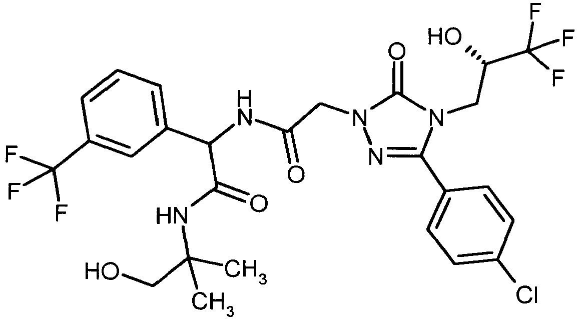

Analog zu Beispiel 41 wurde aus 50 mg (0.14 mmol) der Verbindung aus Beispiel 8A und 67 mg (0.21 mmol) der Verbindung aus Beispiel 51A die Titelverbindung hergestellt. Man erhielt 65 mg (75% d. Th.).

LC-MS [Methode 3]: Rₜ = 1.27 min; MS [ESIpos]: m/z = 638 (M+H)⁺

¹H-NMR (400MHz, DMSO-d₆): δ [ppm]= 1.14 (s, 3H), 1.15 (s, 3H), 3.29 - 3.34 (m, 1H), 3.40 - 3.46 (m, 1H), 3.83 (dd, 1H), 3.96 (dd, 1H), 4.19 - 4.33 (m, 1H), 4.60 (s, 2H), 4.78 (t, 1H), 5.64 (d, 1H), 6.90 (d, 1H), 7.55 - 7.67 (m, 4H), 7.69 - 7.77 (m, 3H), 7.78 (br. s, 1H), 7.98 (s, 1H), 8.93 (d, 1H).

### Beispiel 43

### 2-({[3-(5-Chlor-2-thienyl)-4-(2-methoxyethyl)-5-oxo-4,5-dihydro-1H-1,2,4-triazol-1-yl]acetyl} amino)-N-(1-hydroxy-2-methylpropan-2-yl)-2-[3-(trifluormethyl)phenyl]acetamid (Enantiomer 1)

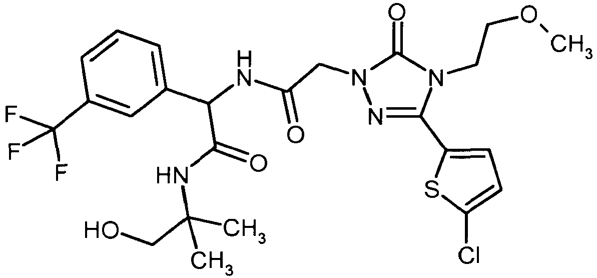

Analog zu Beispiel 1 wurden 25 mg (79 µmol) der Verbindung aus Beispiel 15A und 28 mg (87 µmol) der Verbindung aus Beispiel 50A eingesetzt. Nach HPLC-Reinigung [Methode 7] erhielt man 30 mg (65% d. Th.) der Titelverbindung.

LC-MS [Methode 5]: Rₜ = 0.99 min; MS [ESIpos]: m/z = 590 (M+H)⁺

¹H-NMR (400MHz, DMSO-d₆): δ [ppm]= 1.14 (s, 3H), 1.15 (s, 3H), 3.19 (s, 3H), 3.28 - ca. 3.33 (m, 1H), 3.39 - 3.47 (m, 1H), 3.54 (t, 2H), 3.97 (t, 2H), 4.56 (s, 2H), 4.78 (t, 1H), 5.63 (d, 1H), 7.26 (d, 1H), 7.56 (d, 1H), 7.57 - 7.62 (m, 1H), 7.63 - 7.68 (m, 1H), 7.71 (d, 1H), 7.78 (s, 1H), 7.99 (s, 1H), 8.93 (d, 1H).

### Beispiel 44

### 2-({[3-(5-Chlor-2-thienyl)-4-(2-methoxyethyl)-5-oxo-4,5-dihydro-1H-1,2,4-triazol-1-yl]acetyl}amino)-N-(1-hydroxy-2-methylpropan-2-yl)-2-[3-(trifluormethyl)phenyl]acetamid (Enantiomer 2)

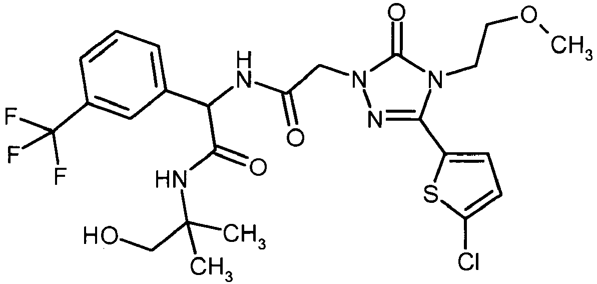

Analog zu Beispiel 1 wurden 25 mg (79 µmol) der Verbindung aus Beispiel 15A und 28 mg (87 µmol) der Verbindung aus Beispiel 51A eingesetzt. Nach HPLC-Reinigung [Methode 7] erhielt man 33 mg (71% d. Th.) der Titelverbindung.

LC-MS [Methode 5]: Rₜ = 0.99 min; MS [ESIpos]: m/z = 590 (M+H)⁺

### Beispiel 45

### 2-(2-Chlorphenyl)-2-({[3-(4-chlorphenyl)-4-(2-fluorbenzyl)-5-oxo-4,5-dihydro-1H-1,2,4-triazol-1-yl]acetyl}amino)-N-(2-methoxyethyl)acetamid (Racemat)

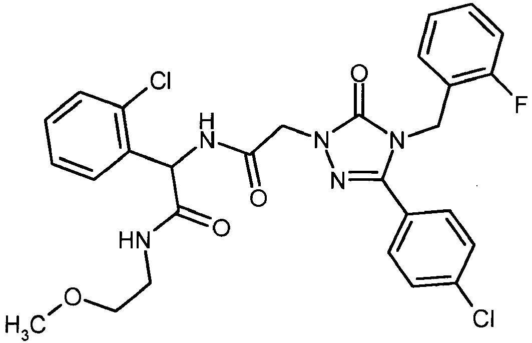

Analog zu Beispiel 1 wurden 25 mg (69 µmol) [3-(4-Chlorphenyl)-4-(2-fluorbenzyl)-5-oxo-4,5-dihydro-1H-1,2,4-triazol-1-yl]essigsäure (Herstellung siehe WO 2007/134862, Beispiel 156A) und 21 mg (76 µmol) der Verbindung aus Beispiel 66A eingesetzt. Nach HPLC-Reinigung [Methode 7] erhielt man 35 mg (86% d. Th.) der Titelverbindung.

LC-MS [Methode 5]: Rₜ = 1.03 min; MS [ESIpos]: m/z = 586 (M+H)⁺

¹H-NMR (400MHz, DMSO-d₆): δ [ppm]= 3.22 (s, 3H), 3.22 - 3.28 (m, 2H), 3.34 - 3.38 (m, 2H), 4.51 - 4.67 (m [AB], 2H), 5.03 (s, 2H), 5.72 (d, 1H), 7.04 (dt, 1H), 7.08 - 7.19 (m, 2H), 7.26 - 7.39 (m, 3H), 7.41 - 7.49 (m, 2H), 7.53 (s, 4H), 8.34 (t, 1H), 8.93 (d, 1H).

### Beispiel 46

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(2-hydroxyethyl)-2-[2-(trifluormethyl)phenyl]acetamid (Diastereomerengemisch)

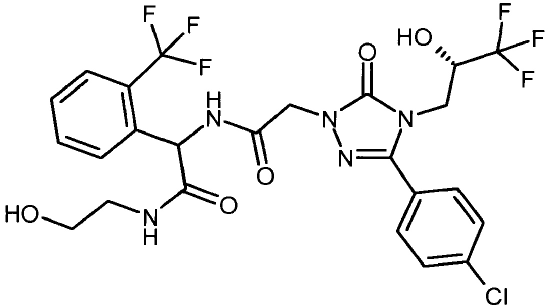

Eine Mischung aus 50 mg (0.14 mmol) der Verbindung aus Beispiel 8A und 33 mg (0.25 mmol) HOBt in 2.9 ml DMF wurde mit 47 mg (0.25 mmol) EDC versetzt und 20 min bei RT gerührt. 49 mg (0.16 mmol) der Verbindung aus Beispiel 52A und 57 µl (0.33 mmol) N,N'-Diisopropylethylamin wurden zugegeben und die Mischung über Nacht gerührt. Nach Zugabe von 1 ml 1N Salzsäure wurde die Reaktionsmischung über präparativer HPLC [Methode 6] getrennt. Die geeignete Fraktion wurde am Rotationsverdampfer eingeengt und der Rückstand im Hochvakuum getrocknet. Man erhielt 24 mg (28% d. Th.) der Titelverbindung als Diastereomerengemisch.

LC-MS [Methode 3]: Rₜ = 1.11 min; MS [ESIpos]: m/z = 610 (M+H)⁺

Durch präparative Chromatographie an einer chiralen Phase (Methode 10) wurden die beiden Diastereomere getrennt: siehe Beispiele 47 und 48.

### Beispiel 47

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(2-hydroxyethyl)-2-[2-(trifluormethyl)phenyl]acetamid (Diastereomer 1)

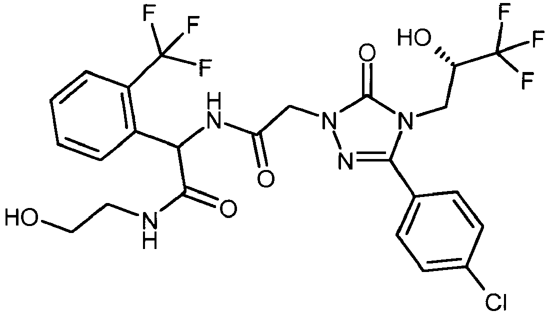

Zuerst eluierendes Diastereomer (11 mg) aus der chromatographischen Diastereomerentrennung nach Methode 10 von 24 mg der Verbindung aus Beispiel 46.

Chirale analytische HPLC [Methode 11]: Rₜ = 1.87 min.

LC-MS [Methode 1]: Rₜ = 1.78 min; MS [ESIpos]: m/z = 610 (M+H)⁺

¹H-NMR (400MHz, DMSO-d₆): δ [ppm]= 3.08 - 3.24 (m, 2H), 3.36 - 3.45 (m, 2H), 3.82 (dd, 1H), 3.96 (dd, 1H), 4.19 - 4.35 (m, 1H), 4.48 (d, 1H), 4.62 (d, 1H), 4.67 (t, 1H), 5.69 (d, 1H), 6.92 (d, 1H), 7.50 - 7.58 (m, 1H), 7.60 - 7.66 (m, 3H), 7.67 - 7.78 (m, 4H), 8.17 (t, 1H), 9.03 (d, 1H).

### Beispiel 48

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(2-hydroxyethyl)-2-[2-(trifluormethyl)phenyl]acetamid (Diastereomer 2)

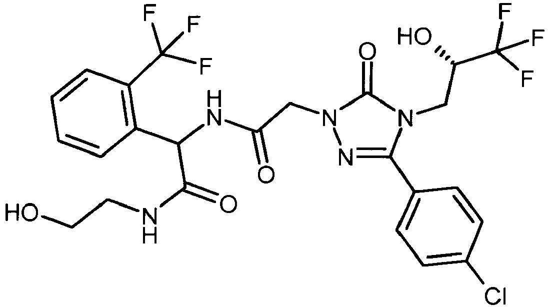

Zuletzt eluierendes Diastereomer (10 mg) aus der chromatographischen Diastereomerentrennung nach Methode 10 von 24 mg der Verbindung aus Beispiel 46.

Chirale analytische HPLC [Methode 11]: Rₜ = 4.12 min.

LC-MS [Methode 1]: Rₜ = 1.76 min; MS [ESIpos]: m/z = 610 (M+H)⁺

¹H-NMR (400MHz, DMSO-d₆): δ [ppm]= 3.07 - 3.24 (m, 2H), 3.36 - 3.46 (m, 2H), 3.82 (dd, 1H), 3.96 (dd, 1H), 4.20 - 4.34 (m, 1H), 4.47 (d, 1H), 4.59 - 4.69 (m, 2H), 5.69 (d, 1H), 6.90 (d, 1H), 7.54 (t, 1H), 7.60 - 7.66 (m, 3H), 7.67 - 7.77 (m, 4H), 8.17 (t, 1H), 9.04 (d, 1H).

### Beispiel 49

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(2-hydroyethyl)-2-[3-(trifluormethyl)phenyl]acetamid (Diastereomerengemisch)

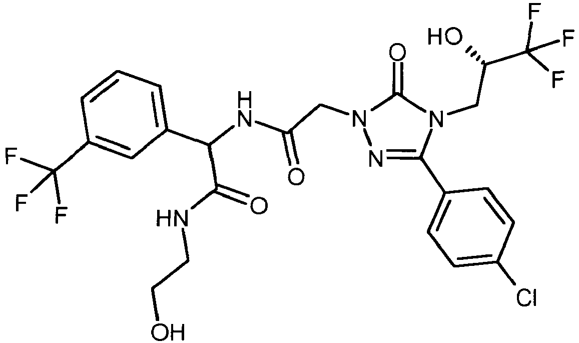

Analog zu Beispiel 46 wurde aus 30 mg (82 µmol) der Verbindung aus Beispiel 8A und 29 mg (98 µmol) der Verbindung aus Beispiel 54A die Titelverbindung hergestellt. Man erhielt 37 mg (74% d. Th.) als Diastereomerengemisch.

LC-MS [Methode 3]: Rₜ = 1.17 min; MS [ESIpos]: m/z = 610 (M+H)⁺

Durch präparative Chromatographie an einer chiralen Phase (Methode 10) wurden die beiden Diastereomere getrennt: siehe Beispiele 50 und 51.

### Beispiel 50

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(2-hydroxyethyl)-2-[3-(trifluormethyl)phenyl]acetamid (Diastereomer 1)

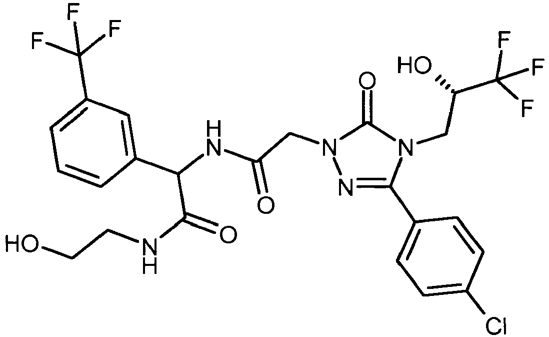

Zuerst eluierendes Diastereomer (11 mg) aus der chromatographischen Diastereomerentrennung nach Methode 10 von 37 mg der Verbindung aus Beispiel 49.

Chirale analytische HPLC [Methode 11]: Rₜ = 2.32 min.

LC-MS [Methode 4]: Rₜ = 2.29 min; MS [ESIpos]: m/z = 610 (M+H)⁺

¹H-NMR (400MHz, DMSO-d₆): δ [ppm]= 3.01 - 3.12 (m, 1H), 3.14 - 3.25 (m, 1H), 3.36 - 3.43 (m, 2H), 3.82 (dd, 1H), 3.96 (dd, 1H), 4.20 - 4.32 (m, 1H), 4.53 - 4.66 (m [AB], 2H), 4.72 (t, 1H), 5.61 (d, 1H), 6.90 (s, 1H), 7.56 - 7.69 (m, 4H), 7.70 - 7.77 (m, 3H), 7.80 (s, 1H), 8.54 (t, 1H), 9.04 (d, 1H).

### Beispiel 51

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(2-hydroxyethyl)-2-[3-(trifluormethyl)phenyl]acetamid (Diastereomer 2)

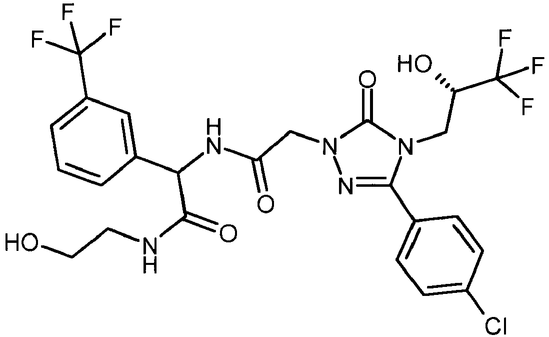

Zuletzt eluierendes Diastereomer (13 mg) aus der chromatographischen Diastereomerentrennung nach Methode 10 von 37 mg der Verbindung aus Beispiel 49.

Chirale analytische HPLC [Methode 11]: Rₜ = 4.01 min.

LC-MS [Methode 3]: Rₜ = 1.16 min; MS [ESIpos]: m/z = 610 (M+H)⁺

¹H-NMR (400MHz, DMSO-d₆): δ [ppm]= 3.01 - 3.11 (m, 1H), 3.15 - 3.25 (m, 1H), 3.35 - 3.44 (m, 2H), 3.82 (dd, 1H), 3.96 (dd, 1H), 4.20 - 4.33 (m, 1H), 4.52 - 4.66 (m [AB], 2H), 4.71 (t, 1H), 5.61 (d, 1H), 6.88 (d, 1H), 7.55 - 7.69 (m, 4H), 7.70 - 7.77 (m, 3H), 7.80 (s, 1H), 8.54 (t, 1H), 9.04 (d, 1H).

### Beispiel 52

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(2-hydroxyethyl)-2-[3-(trifluormethoxy)phenyl]acetamid (Diastereomerengemisch)

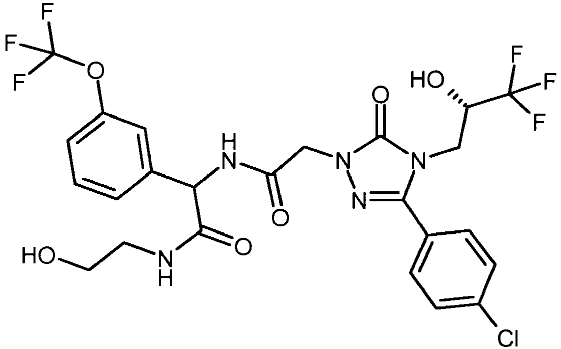

Analog zu Beispiel 1 (Reaktionszeit: über Nacht) wurde aus 40 mg (0.11 mmol) der Verbindung aus Beispiel 8A und 38 mg (0.12 mmol) der Verbindung aus Beispiel 55A die Titelverbindung hergestellt. Man erhielt 64 mg (93% d. Th.) als Diastereomerengemisch.

LC-MS [Methode 3]: Rₜ = 1.20 min; MS [ESIpos]: m/z = 626 (M+H)⁺

¹H-NMR (400MHz, DMSO-d₆): δ [ppm]= 3.02 - 3.12 (m, 1H), 3.14 - 3.24 (m, 1H), 3.34 - 3.43 (m, 2H), 3.82 (dd, 1H), 3.96 (dd, 1H), 4.20 - 4.33 (m, 1H), 4.53 - 4.65 (m [AB], 2H), 4.72 (t, 1H), 5.57 (d, 1H), 6.90 (dd, 1H, interpretiert als je 1 d (6.889 und 6.904) pro Diastereomer), 7.29 (d, 1H), 7.42 (s, 1H), 7.44 - 7.52 (m, 2H), 7.62 (d, 2H), 7.71 - 7.78 (m, 2H), 8.54 (t, 1H), 9.00 (d, 1H).

### Beispiel 53

### (3S)-3-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(2-hydroxyethyl)-3-[2-(trifluormethyl)phenyl]propanamid

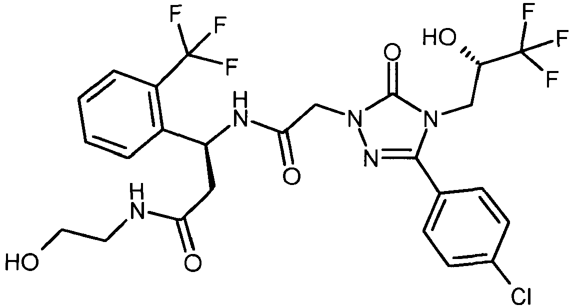

Analog zu Beispiel 46 wurde aus 35 mg (96 µmol) der Verbindung aus Beispiel 8A und 36 mg (115 µmol) der Verbindung aus Beispiel 56A die Titelverbindung hergestellt. Es wurden 25 mg (42% d. Th.) erhalten.

LC-MS [Methode 4]: Rₜ = 2.18 min; MS [ESIpos]: m/z = 624 (M+H)⁺

¹H-NMR (400MHz, DMSO-d₆): δ [ppm]= 2.45 (dd, 1H), 2.58 (dd, 1H), 3.09 (q, 2H), 3.31 - 3.38 (m, 2H), 3.82 (dd, 1H), 3.96 (dd, 1H), 4.19 - 4.33 (m, 1H), 4.39 - 4.51 (m, 2H), 4.64 (t, 1H), 5.46-5.54 (m, 1H), 6.89 (d, 1H), 7.42 - 7.51 (m, 1H), 7.59 - 7.70 (m, 5H), 7.74 (d, 2H), 7.82 (t, 1H), 8.76 (d, 1H).

### Beispiel 54

### (3S)-3-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(2-hydroxyethyl)-3-[3-(trifluormethyl)phenyl]propanamid

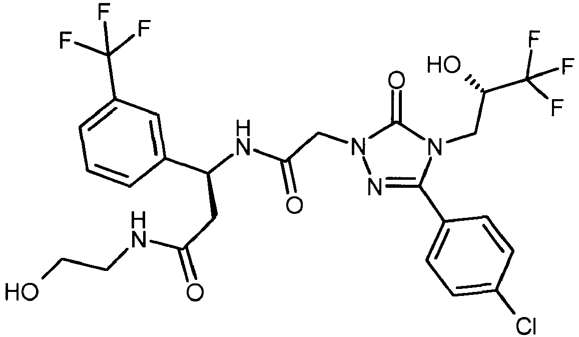

Analog zu Beispiel 1 wurde aus 74 mg (0.20 mmol) der Verbindung aus Beispiel 8A und 70 mg (0.22 mmol) der Verbindung aus Beispiel 57A die Titelverbindung hergestellt. Es wurden 103 mg (81% d. Th.) erhalten.

LC-MS [Methode 3]: Rₜ = 1.16 min; MS [ESIpos]: m/z = 624 (M+H)⁺.

¹H-NMR (400MHz, DMSO-d₆): δ [ppm]= 2.57 - 2.64 (m, 2H), 3.03 (q, 2H), 3.23 - 3.29 (m, 2H), 3.82 (dd, 1H), 3.96 (dd, 1H), 4.20 - 4.35 (m, 1H), 4.48 (s, 2H), 4.57 - 4.63 (m, 1H), 5.27 (q, 1H), 6.89 (br. s., 1H), 7.51 - 7.66 (m, 6H), 7.70 - 7.78 (m, 2H), 7.93 (t, 1H), 8.81 (d, 1H).

### Beispiel 55

### (3S)-3-({[3-(4-Chlorphenyl)-4-cyclopropyl-5-oxo-4,5-dihydro-1H-1,2,4-triazol-1-yl]acetyl}amino)-N-(2-hydroxyethyl)-3-[2-(trifluormethyl)phenyl]propanamid

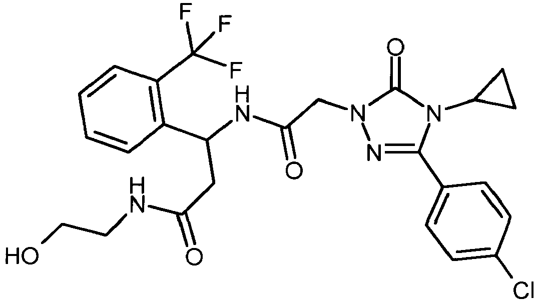

Analog zu Beispiel 46 wurde aus 50 mg (0.17 mmol) [3-(4-Chlorphenyl)-4-cyclopropyl-5-oxo-4,5-dihydro-1*H*-1,2,4-triazol-1-yl]-essigsäure (Herstellung siehe WO 2007/134862, Beispiel 88A) und 64 mg (0.20 mmol) der Verbindung aus Beispiel 56A die Titelverbindung hergestellt. Man erhielt 43 mg (46% d. Th.).

LC-MS [Methode 4: Rₜ = 1.99; MS [ESIpos]: m/z = 552 (M+H)⁺.

¹H-NMR (400MHz, DMSO-d₆): δ [ppm]= 0.50 - 0.66 (m, 2H), 0.81 - 0.95 (m, 2H), 2.43 (dd, 1H), 2.54 - 2.60 (m, 1H), 3.07 (q, 2H), 3.16 (tt, 1H), 3.28 - 3.38 (m, 2H), 4.32 - 4.43 (m [AB], 2H), 4.63 (t, 1H), 5.41 - 5.52 (m, 1H), 7.41 - 7.50 (m, 1H), 7.55 - 7.70 (m, 5H), 7.76 - 7.86 (m, 3H), 8.76 (d, 1H).

### Beispiel 56

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(2-methoxyethyl)-2-[2-(trifluormethyl)phenyl]acetamid (Diastereomerengemisch)

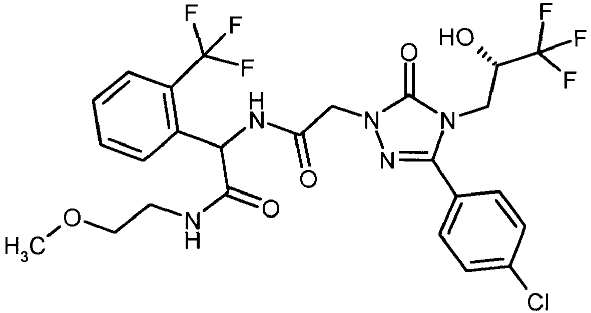

Analog zu Beispiel 46 wurde aus 30 mg (82 µmol) der Verbindung aus Beispiel 8A und 31 mg (98 µmol) der Verbindung aus Beispiel 53A die Titelverbindung hergestellt. Man erhielt 42 mg (82% d. Th.).

LC-MS [Methode 1]: Rₜ = 1.93 min; MS [ESIpos]: m/z = 624 (M+H)⁺

¹H-NMR (400MHz, DMSO-d₆): δ [ppm]= 3.23 (d, 3H, interpretiert als je 1 s pro Diastereomer), 3.20 - 3.29 (m, 2H), 3.29 - 3.40 (m, 2H), 3.82 (dd, 1H), 3.96 (dd, 1H), 4.20 - 4.34 (m, 1H), 4.47 (dd, 1H), 4.62 (dd, 1H), 5.69 (dd, 1H, interpretiert als je 1 d pro Diastereomer), 6.91 (dd, 1H, interpretiert als je 1 d pro Diastereomer), 7.55 (t, 1H), 7.59 - 7.66 (m, 3H), 7.67 - 7.79 (m, 4H), 8.27 (t, 1H), 9.04 (dd, 1H, interpretiert als je 1 d pro Diastereomer).

### Beispiel 57

### (3S)-3-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-(2-methoxyethyl)-3-[3-(trifluormethyl)phenyl]propanamid

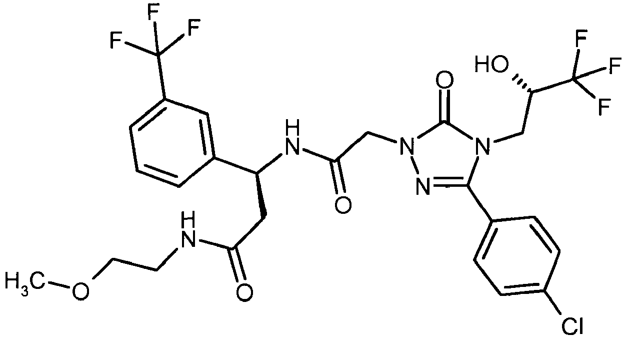

Analog zu Beispiel 46 wurde aus 50 mg (0.14 mmol) der Verbindung aus Beispiel 8A und 54 mg (0.16 mmol) der Verbindung aus Beispiel 59A die Titelverbindung hergestellt. Es wurden 77 mg (88% d. Th.) erhalten.

LC-MS [Methode 3]: R, = 1.25 min; MS [ESIpos]: m/z = 638 (M+H)⁺

¹H-NMR (400MHz, DMSO-d₆): δ [ppm]= 2.57 - 2.65 (m, 2H), 3.04 - 3.24 (m, 4H), 3.15 (s, 3H), 3.82 (dd, 1H), 3.96 (dd, 1H), 4.21 - 4.34 (m, 1H), 4.48 (s, 2H), 5.23 - 5.32 (m, 1H), 6.89 (d, 1H), 7.51 - 7.65 (m, 6H), 7.71 - 7.79 (m, 2H), 8.01 (t, 1H), 8.82 (d, 1H).

### Beispiel 58

### (3S)-3-[({3-(4-Chlorphenyl)-5-oxo-4-[(2S)-3,3,3-trifluor-2-hydroxypropyl]-4,5-dihydro-1H-1,2,4-triazol-1-yl} acetyl)amino]-N-(2-methoxyethyl)-3-[2-(trifluormethyl)phenyl]propanamid

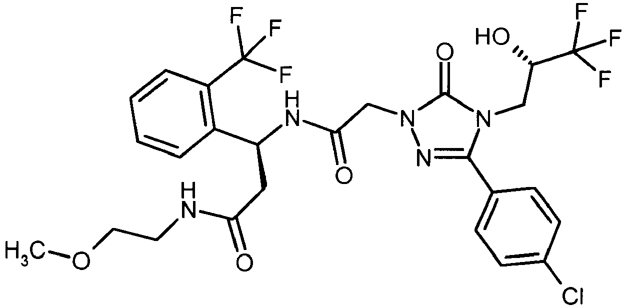

Analog zu Beispiel 46 wurde aus 35 mg (96 µmol) der Verbindung aus Beispiel 8A und 38 mg (115 µmol) der Verbindung aus Beispiel 58A die Titelverbindung hergestellt. Es wurden 53 mg (87% d. Th.) erhalten.

LC-MS [Methode 4]: Rₜ = 2.37 min; MS [ESIpos]: m/z = 638 (M+H)⁺

¹H-NMR (400MHz, DMSO-d₆): δ [ppm]= 2.45 (dd, 1H), 2.58 (dd, 1H), 3.14 - 3.20 (m, 2H), 3.21 (s, 3H), 3.25 - 3.29 (m, 2H), 3.82 (dd, 1H), 3.96 (dd, 1H), 4.21 - 4.32 (m, 1H), 4.44 (s, 2H), 5.45 - 5.55 (m, 1H), 6.89 (d, 1H), 7.44 - 7.50 (m, 1H), 7.59 - 7.70 (m, 5H), 7.72 - 7.77 (m, 2H), 7.90 (t, 1H), 8.77 (d, 1H).

### Beispiel 59

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-[2-(trifluormethoxy)ethyl]-2-[3-(trifluormethyl)phenyl]acetamid (Diastereomer 1)

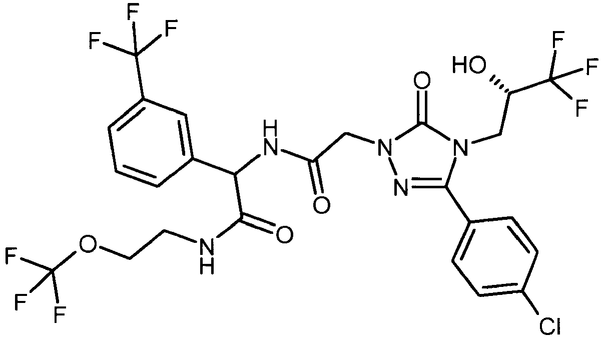

Analog zu Beispiel 1 wurde aus 37 mg (0.10 mmol) der Verbindung aus Beispiel 8A und 45 mg (0.12 mmol) der Verbindung aus Beispiel 60A die Titelverbindung hergestellt. Man erhielt 61 mg (88% d. Th.).

LC-MS [Methode 1]: Rₜ = 2.26 min; MS [ESIpos]: m/z = 678 (M+H)⁺

¹H-NMR (400MHz, DMSO-d₆): δ [ppm]= 3.30 - 3.40 (m, 1H), 3.40 - 3.51 (m, 1H), 3.82 (dd, 1H), 3.96 (dd, 1H), 3.98 - 4.07 (m, 2H), 4.20 - 4.32 (m, 1H), 4.54 - 4.65 (m [AB], 2H), 5.60 (d, 1H), 6.90 (d, 1H), 7.56 - 7.64 (m, 3H), 7.65 - 7.69 (m, 1H), 7.70 - 7.77 (m, 3H), 7.78 (br. s, 1H), 8.76 (t, 1H), 9.08 (d, 1H).

### Beispiel 60

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-N-[2-(trifluormethoxy)ethyl]-2-[3-(trifluormethyl)phenyl]acetamid (Diastereomer 2)

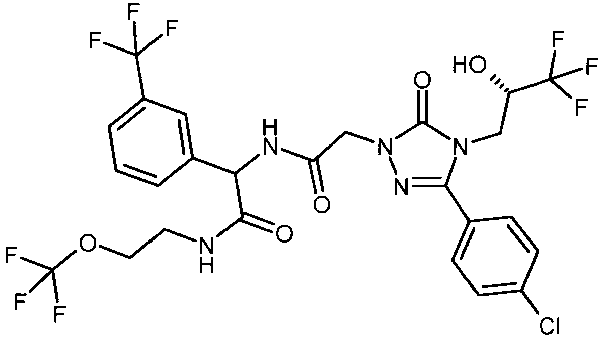

Analog zu Beispiel 1 wurde aus 50 mg (0.14 mmol) der Verbindung aus Beispiel 8A und 60 mg (0.16 mmol) der Verbindung aus Beispiel 61A die Titelverbindung hergestellt. Man erhielt 73 mg (79% d. Th.)

LC-MS [Methode 1]: Rₜ = 2.26 min; MS [ESIpos]: m/z = 678 (M+H)⁺

¹H-NMR (400MHz, DMSO-d₆): δ [ppm]= 3.31 - 3.40 (m, 1H), 3.40 - 3.50 (m, 1H), 3.82 (dd, 1H), 3.96 (dd, 1H), 3.97 - 4.07 (m, 2H), 4.19 - 4.35 (m, 1H), 4.52 - 4.68 (m [AB], 2H), 5.60 (d, 1H), 6.88 (d, 1H), 7.56 - 7.65 (m, 3H), 7.65 - 7.69 (m, 1H), 7.70 - 7.76 (m, 3H), 7.79 (s, 1H), 8.76 (t, 1H), 9.08 (d, 1H).

### B. Bewertung der pharmakologischen Wirksamkeit

### Abkürzungen:

- EDTA: Ethylendiamintetraessigsäure
- DMEM: Dulbecco's Modified Eagle Medium
- FCS: Fötales Kälberserum
- HEPES: 4-(2-Hydroxyethyl)-1-piperazinethansulfonsäure
- SmGM: Smooth Muscle Cell Growth Media
- Tris-HCl: 2-Amino-2-(hydroxymethyl)-1,3-propandiol-Hydrochlorid
- UtSMC: Uterine Smooth Muscle Cells

Die pharmakologische Wirkung der erfindungsgemäßen Verbindungen kann in folgenden Assays gezeigt werden:

### B-1. Zellulärer in vitro-Test zur Bestimmung der Vasopressin-Rezeptor-Aktivität

Die Identifizierung von Agonisten und Antagonisten der V1a und V2 Vasopressin Rezeptoren aus Mensch und Ratte sowie die Quantifizierung der Wirksamkeit der hier beschriebenen Substanzen erfolgte mit Hilfe von rekombinanten Zelllinien. Diese Zellen leiten sich ursprünglich von einer Ovarepithelzelle des Hamsters (Chinese Hamster Ovary, CHO K1, ATCC: American Type Culture Collection, Manassas, VA 20108, USA) ab. Die Testzelllinien exprimieren konstitutiv eine modifizierte Form des Calcium-sensitiven Photoproteins Aequorin, das nach Rekonstitution mit dem Co-Faktor Coelenterazin bei Erhöhungen der freien Calcium-Konzentration im Licht emittiert (Rizzuto R., Simpson A.W., Brini M., Pozzan T.; Nature 358 (1992) 325-327). Zusätzlich sind die Zellen stabil transfiziert mit den V1a oder V2 Rezeptoren des Menschen oder der Ratte. Im Falle der Gs-koppelnden V2-Rezeptoren sind die Zellen mit einem weiteren Gen, das für das promiskuitive G_{α16}-Protein kodiert (Amatruda T.T., Steele D.A., Slepak V.Z., Simon M.I., Proc. Na. Acad. Sci. USA 88 (1991), 5587-5591), entweder unabhängig oder als Fusionsgen stabil transfiziert. Die resultierenden Vasopressin Rezeptor-Testzellen reagieren auf Stimulation der rekombinant exprimierten Vasopressin-Rezeptoren mit einer intrazellulären Freisetzung von Calcium-Ionen, die durch die resultierende Aequorin-Lumineszenz mit einem geeignetem Luminometer quantifiziert werden kann (Milligan G., Marshall F., Rees S., Trends in Pharmaco. Sci. 17 (1996) 235-237).

Testablauf: Die Zellen werden am Tag vor dem Test in Kulturmedium (DMEM, 10% FCS, 2 mM Glutamine, 10 mM HEPES) in 384-Loch-Mikrotiterplatten ausplattiert und in einem Zellinkubator (96% Luftfeuchtigkeit, 5% v/v Kohlendioxid, 37°C) gehalten. Am Testtag wird das Kulturmedium durch eine Tyrodelösung (140 mM Natriumchlorid, 5 mM Kaliumchlorid, 1 mM Magnesiumchlorid, 2 mM Calciumchlorid, 20 mM Glucose, 20 mM HEPES), das zusätzlich den Co-Faktor Coelenterazin (50 µM) enthält, ausgetauscht und die Mikrotiterplatte anschließend für weitere 3-4 Stunden inkubiert. Die Testsubstanzen werden in verschiedenen Konzentrationen für 10 bis 20 Minuten in den Löchern der Mikrotiterplatte vorgelegt, ehe der Agonist [Arg8]-Vasopressin hinzugegeben wird und das resultierende Lichtsignal sofort im Luminometer gemessen wird. Die Berechnung der IC50-Werte erfolgt mit Hilfe des Computerprogramms GraphPad PRISM (Version 3.02).

In der folgenden Tabelle A sind repräsentative IC₅₀-Werte für die erfindungsgemäßen Verbindungen an der mit dem humanen V1a- bzw. V2-Rezeptor transfizierten Zelllinie aufgeführt:

**Tabelle A:**

| **Beispiel Nr.** | **IC₅₀ hV1a [µM]** | **IC₅₀ hV2 [µM]** |
|---|---|---|
| 1 | 0.0092 | 0.058 |
| 3 | 0.554 | 0.467 |
| 4 | 0.0019 | 0.0041 |
| 5 | 0.042 | 1.17 |
| 6 | 0.047 | 0.324 |
| 10 | 0.15 | 0.96 |
| 11 | 0.028 | 0.497 |
| 12 | 0.024 | 0.182 |
| 14 | 0.053 | 0.009 |
| 15 | 0.012 | 0.31 |
| 16 | 0.524 | 0.262 |
| 17 | 1.42 | 0.022 |
| 18 | 0.0012 | 0.0019 |
| 19 | 0.0006 | 0.0011 |
| 20 | 0.0024 | 0.0045 |
| 21 | 0.0010 | 0.0008 |
| 22 | 0.0012 | 0.0027 |
| 25 | 0.502 | 0.282 |
| 26 | 0.0017 | 0.0023 |
| 28 | 1.38 | 0.346 |
| 29 | 0.0010 | 0.0033 |
| 31 | 0.0078 | 1.18 |
| 32 | 0.0071 | 1.27 |
| 33 | 0.017 | 1.05 |
| 35 | 0.0089 | 0.655 |
| 36 | 0.0882 | 0.78 |
| 38 | 0.0328 | 0.0386 |
| 39 | 0.0049 | 0.0008 |
| 40 | 0.0111 | 0.0081 |
| 41 | 0.0015 | 0.0036 |
| 42 | 0.038 | 0.158 |
| 43 | 0.060 | 1.60 |
| 49 | 0.0022 | 0.0076 |
| 52 | 0.0097 | 0.042 |
| 53 | 0.0009 | 0.0026 |
| 54 | 0.0038 | 0.0026 |
| 55 | 0.0060 | 0.569 |
| 56 | 0.0030 | 0.144 |
| 57 | 0.0145 | 0.0074 |
| 58 | 0.0035 | 0.136 |
| 59 | 0.136 | 0.061 |
| 60 | 0.0030 | 0.0197 |

### B-2. Zellulärer in vitro Test zum Nachweis der Wirkung von Vasopressin V1a Rezeptor Antagonisten auf die Regulation pro-fibrotischer Gene

Die als Kardiomyozyten-Typ beschriebene Zelllinie H9C2 (American Type Culture Collection ATCC-Nr. CRL-1446), die aus Herzgewebe der Ratte isoliert wurde, exprimiert endogen den Vasopressin V1A Rezeptor AVPR1A in hoher Kopienzahl, während die AVPR2 Expression nicht nachweisbar ist. Für Zell-Tests zur Hemmung der AVPR1A Rezeptor abhängigen Regulation der Genexpression durch Rezeptor-Antagonisten wird wie folgt verfahren:
H9C2 Zellen werden in 1.0 ml Medium Opti-MEM (Invitrogen Corp. Carlsbad CA, USA, Kat. Nr. 11058-021) mit 2% FCS und 1% Penicillin/Streptomycin Lösung (Invitrogen Kat. Nr. 10378-016) mit einer Zelldichte von 100000 Zellen/Well in 12-Well Mikrotiterplatten für die Zellkultur ausgesät und in einem Zellinkubator (96% Luftfeuchtigkeit, 5% v/v Kohlendioxid, 37°C) gehalten. Nach 24 Stunden werden in je drei Wells (Triplikate) Vehikel-Lösung (Negativ-Kontrolle), Vasopressin-Lösung [Arg8]-Vasopressin Acetat (Sigma Kat. Nr. V9879) oder Testsubstanzen (gelöst in Vehikel Wasser mit 20 Vol% Ethanol) und Vasopressin-Lösung gegeben. In der Zellkultur ist die finale Vasopressin-Konzentration 0.05 µM. Die Testsubstanz Lösung wird in kleinen Volumina zur Zellkultur gegeben, so dass eine finale Konzentration von 0.1% Ethanol im Zelltest nicht überschritten wird. Nach einer Inkubationszeit von 6 Stunden wird der Kulturüberstand abgesaugt, die adhärenten Zellen werden in 250 µl RLT-Puffer (Qiagen, Ratingen, Kat. Nr. 79216) lysiert, und die RNA wird aus diesem Lysat mit dem RNeasy Kit (Qiagen, Kat. Nr. 74104) isoliert. Anschliessend erfolgt der DNAse-Verdau (Invitrogen Kat. Nr. 18068-015), die cDNA Synthese (Promaga ImProm-II Reverse Transcription System Kat. Nr. A3800) und die RTPCR mit dem pPCR MasterMix RT-QP2X-03-075 von Eurogentec, Seraing, Belgien. Alle Prozeduren erfolgen gemäß den Arbeitsprotokollen der Test-Reagenzien Hersteller. Die Primer-Sets für die RTPCR werden anhand der mRNA Gensequenzen (NCBI Genbank Entrez Nucleotide Data Base) mit Hilfe des Programms Primer3Plus mit 6-FAM - TAMRA markierten Sonden ausgewählt. Die RTPCR zur Bestimmung der relativen mRNA Expression in den Zellen der verschiedenen Test-Ansätze erfolgt mit dem Applied Biosystems ABI Prism 7700 Sequence Detector im 96-Well oder 384-Well Mikrotiterplatten-Format gemäß der Betriebsanleitung des Gerätes. Die relative Genexpression wird durch den delta-delta Ct-Wert dargestellt [Applied Biosystems, User Bulletin No. 2 ABI Prism 7700 SDS December 11, 1997 (updated 10/2001)] mit dem Bezug auf die Expressionsstärke des Gens Ribosomales Protein L-32 (Genbank Acc. No. NM_013226) und den Schwellen-Ct-Wert Ct = 35.

### B-3. In vivo-Test zum Nachweis der kardiovaskulären Wirkung: Blutdruckmessung an narkotisierten Ratten (Vasopressin, Challenge' Modell)

Bei männlichen Sprague-Dawley-Ratten (250-350 g Körpergewicht) werden in Ketamin/Xylazin/Pentobarbital-Injektionsnarkose Polyethylenschläuche (PE-50; Intramedic®), die mit Heparin-haltiger (500 1.E./ml) isotoner Natriumchlorid-Lösung vorgefüllt sind, in die Vena jugularis und die Vena femoralis eingeführt und anschließend eingebunden. Über einen venösen Zugang wird mit Hilfe einer Spritze Arginin-Vasopressin injiziert, über den zweiten venösen Zugang werden die Testsubstanzen appliziert. Zur Ermittlung des systolischen Blutdruckes wird ein Druckkatheter (Millar SPR-320 2F) in die A. carotis eingebunden. Der arterielle Katheter wird an einen Druckwandler angeschlossen, der seine Signale in einen mit einer geeigneten Registrierungssoftware ausgestatteten Messcomputer einspeist. In einem typischen Experiment werden dem Versuchstier 3-4 aufeinanderfolgende Bolusinjektionen im Abstand von 10-15 min mit einer definierten Menge Arginin-Vasopressin (30 ng/kg) in isotoner Natriumchlorid-Lösung verabreicht und, nachdem der Blutdruck wieder Ausgangswerte erreicht hat, die zu testende Substanz als Bolus mit anschließender Dauerinfusion in einem geeigneten Lösungsmittel appliziert. Hiernach wird in definierten Abständen (10-15 min) erneut die gleiche Menge Vasopressin wie zu Anfang verabreicht.

Anhand der Blutdruckwerte wird ermittelt, inwieweit die Testsubstanz der blutdrucksteigernden Wirkung des Vasopressins entgegenwirkt. Kontrolltiere erhalten nur Lösungsmittel statt der Testsubstanz.

Die erfindungsgemäßen Verbindungen bewirken nach intravenöser Applikation im Vergleich zu den Lösungsmittelkontrollen eine Hemmung des durch Arginin-Vasopressin verursachten Blutdruckanstiegs.

### B-4. In vivo-Test zum Nachweis der kardiovaskulären Wirkung: Diurese-Untersuchungen an wachen Ratten in Stoffwechselkäfigen

Wistar Ratten (220-400 g Körpergwicht) werden mit freiem Zugang zu Futter (Altromin) und Trinkwasser gehalten. Während des Versuches werden die Tiere für 4 bis 8 Stunden einzeln in für Ratten dieser Gewichtsklasse geeigneten Stoffwechselkäfigen (Tecniplast Deutschland GmbH, D-82383 Hohenpeißenberg) mit freiem Zugang zu Trinkwasser gehalten. Am Versuchsbeginn wird den Tieren die zu prüfende Substanz in einem Volumen von 1 bis 3 ml/kg Körpergewicht eines geeigneten Lösungsmittels mittels einer Schlundsonde in den Magen verabreicht. Als Kontrolle dienende Tiere erhalten nur Lösungsmittel. Kontrollen und Substanztestungen werden am selben Tag parallel durchgeführt. Kontrollgruppen und Substanzdosisgruppen bestehen aus jeweils 4 bis 8 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschiedenen Natrium- bzw. KaliumIonen mittels flammenphotometrischer Standardmethoden gemessen. Um eine ausreichende Urinmenge zu erhalten, wird den Tieren zu Versuchsbeginn per Schlundsonde eine definierte Menge Wasser zugeführt (typischerweise 10 ml pro Kilogramm Körpergewicht). Vor Versuchsbeginn und nach Versuchsende wird das Körpergewicht der einzelnen Tiere bestimmt.

Die erfindungsgemäßen Verbindungen bewirken nach oraler Applikation im Vergleich mit Kontrolltieren eine gesteigerte Ausscheidung von Urin, die im wesentlichen auf einer gesteigerten Wasserauscheidung (Aquarese) beruht.

### B-5. In vivo-Test zum Nachweis der kardiovaskulären Wirkung: Hämodynamische Untersuchungen an narkotisierten Hunden

Männliche oder weibliche Mischlingshunde (Mongrels, Marshall BioResources, USA) mit einem Gewicht zwischen 20 und 30 kg werden mit Pentobarbital (30 mg/kg iv, Narcoren®, Merial, Deutschland) werden für die operativen Eingriffe und die hämodynamische und funktionellen Untersuchungstermine jeweils annarkotisiert. Alcuroniumchlorid (Alloferin®, ICN Pharmaceuticals,

Deutschland, 3 mg/Tier iv) dient dabei zusätzlich als Muskelrelaxans. Die Hunde werden intubiert und mit einem Sauerstoff-Raumluft-Gemisch (40/60%) beatmet (etwa 5-6L/min). Die Beatmung erfolgt mit einem Beatmungsgerät der Firma Draeger (Sulla 808) und wird überwacht mit einem Kohlendioxid-Analysator (Engström).

Die Narkose wird aufrechterhalten durch eine ständige Infusion von Pentobarbital (50µg/kg/min); als Analgetikum dient Fentanyl (10 µg/kg/h). Eine Alternative zu Pentobarbital besteht in der Verwendung von Isofluran (1-2 Vol%).

Die Hunde werden in vorbereitenden Eingriffen mit einem Herzschrittmacher ausgestattet.
- Zum Zeitpunkt 21 Tage vor der ersten Medikamenten-Testung =(Versuchsbeginn) wird eine Herzschrittmacher der Fa. Biotronik (Logos®) in eine subcutane Hauttasche implantiert und über eine Schrittmacher-Elektrode, die durch die Vena jugularis externa unter Durchleuchtung bis in den rechten Ventrikel vorgeschoben wird, mit dem Herzen in Kontakt gebracht.
- Zum gleichen Zeitpunkt der Schrittmacher-Implantation erfolgt durch retrogrades Vorschieben eine 7F-Biopsie-Zange (Fa. Cordis) über eine Schleuse (Avanti+®; Fa. Cordis) in der Arteria femoralis und nach atraumatischer Passager der Aortenklappe eine definierte Läsion der Mitralklappe unter echokardiographischer und Durchleuchtungskontrolle. Im Anschluss werden alle Zugänge entfernt und der Hund wacht spontan aus der Narkose auf.
- Nach 7 weiteren Tagen (=14 Tage vor der ersten Medikamenten-Testung) wird der o.g. Schrittmacher aktiviert und das Herz mit einer Frequenz von 220 Schlägen pro Minute stimuliert.

Die eigentlichen Experimente zur Medikamenten-Testung erfolgen 14 und 28 Tagen nach Beginn der Schrittmacher-Stimulation nach folgender Instrumentierung:
- Blasenkatheter zur Blasenentlastung bzw. zur Messung des Urinflusses
- EKG-Ableitungen an den Extremitäten (zur EKG Messung)
- Einführen eines NaCl-gefüllten Fluidmedic-PE-300-Schlauches in die A. femoralis. Dieser ist verbunden mit einem Drucksensor (Braun Melsungen, Melsungen, Deutschland) zur Messung des systemischen Blutdrucks
- Einführen eines Millar Tip Katheters (Typ 350 PC, Millar Instruments, Houston, USA) über den linken Vorhof oder über eine in der A. carotis eingebundene Schleuse zur Messung der Herz-Hämodynamik
- Einführen eines Swan-Ganz-Katheters (CCOmbo 7.5F, Edwards, Irvine, USA) über die V. jugularis in die A. pulmonalis zur Messung von Herzzeitvolumen, Sauerstoffsättigung, Pulmonalartiendrücken und zentralvenösem Druck.
- Platzieren einer Braunüle in den Venae cephalicae zur Infusion von Pentobarbital, der Flüssigkeitssubstitution und zur Blutentnahme (Bestimmung der Substanz-Plasmaspiegeln oder sonstiger klinischer Blutwerte)
- Platzieren einer Braunüle in der Venae saphenae zur Infusion von Fentanyl und zur Substanzapplikation
- Infusion von Vasopressin (Fa. Sigma) in aufsteigender Dosierung bis zu einer Dosis von 4 mU/kg/min. Unter dieser Dosierung erfolgt dann die Testung pharmakologischer Substanzen.

Die Primärsignale werden eventuell verstärkt (Gould Verstärker, Gould Instrument Systems, Valley View, USA) bzw. Edwards-Vigilance- Monitor (Edwards, Irvine, USA) und anschließend zur Auswertung in das Ponemah-System (DataSciences Inc, Minneapolis, USA) eingespeist. Die Signale werden kontinuierlich über den gesamten Versuchszeitraum aufgezeichnet, von dieser Software digital weiterarbeitet und über 30 s gemittelt.

### B-6. Bestimmung der Löslichkeit

### Benötigte Reagenzien:

- PBS-Puffer pH 6.5: 90.00 g NaCl p.a. (z.B. Fa. Merck, Art.-Nr. 1.06404.1000), 13.61 g KH₂PO₄ p.a. (z.B. Fa. Merck, Art.-Nr. 1.04873.1000) und 83.35 g 1 N Natronlauge (z.B. Fa. Bernd Kraft GmbH, Art.-Nr. 01030.4000) werden in einen 1 Liter-Messkolben eingewogen, mit destilliertem Wasser auf 1 Liter aufgefüllt und für 1 Stunde gerührt. Danach wird mit 1 N Salzsäure (z.B. Fa. Merck, Art.-Nr. 1.09057.1000) der pH-Wert auf 6.5 eingestellt.
- PEG/Wasser-Lösung (30:70 v/v): 30 ml Polyethylenglykol 400 (z.B. Fa. Merck, Art.-Nr. 8.17003.1000) und 70 ml destilliertes Wasser werden in einem 100 ml-Messkolben homogenisiert.
- PEG/PBS-Puffer pH 6.5 (80:20 v/v): 80 ml Polyethylenglykol 400 (z.B. Fa. Merck, Art.-Nr. 8.17003.1000) und 20 ml PBS-Puffer pH 6.5 werden in einem 100 ml-Messkolben homogenisiert.
- Dimethylsulfoxid (z.B. Fa. Baker, Art.-Nr. 7157.2500)
- destilliertes Wasser.

### Herstellung der Ausgangslösung (Urlösung)

Mindestens 4 mg der Testsubstanz werden in ein Weithals-10 mm Screw V-Vial (Fa. Glastechnik Gräfenroda GmbH, Art.-Nr. 8004-WM-H/V15µ) mit passender Schraubkappe und Septum genau eingewogen, in einem Pipettierroboter mit DMSO bis zu einer Konzentration von 50 mg/ml versetzt und 10 Minuten geschüttelt.

### Herstellung der Kalibrierlösungen:

*Herstellung der Ausgangslösung für Kalibrierlösungen (Stammlösung):* In eine Mikrotiterplatte werden 10 µl der Urlösung mit Hilfe eines Pipettierroboters überführt und mit DMSO bis zu einer Konzentration von 600 µg/ml aufgefüllt. Die Probe wird bis zu ihrer vollständigen Lösung geschüttelt.

*Kalibrierlösung 1 (20 µg*/*ml):* 34.4 µl der Stammlösung werden mit 1000 µl DMSO versetzt und homogenisiert.

*Kalibrierlösung 2 (2.5 µg*/*ml):* 100 µl der Kalibrierlösung 1 werden mit 700 µl DMSO versetzt und homogenisiert.

### Herstellung der Probenlösungen:

*Probenlösung für Löslichkeit bis 5 glLiter in PBS-Puffer pH 6.5:* In eine Mikrotiterplatte werden 10 µl Urlösung transferiert und mit 1000 µl PBS-Puffer pH 6.5 versetzt.

*Probenlösung für Löslichkeit bis 5 glLiter in PEG*/*Wasser (30:70)*: In eine Mikrotiterplatte werden 10 µl Urlösung transferiert und mit 1000 µl PEG/Wasser (30:70) versetzt.

*Probenlösung für Löslichkeit bis 5 g*/*Liter in PEG*/*PBS-Puffer pH 6.5 (80:20)*: In eine Mikrotiterplatte werden 10 µl Urlösung transferiert und mit 1000 µl PEG/PBS-Puffer pH 6.5 (80:20) versetzt.

### Durchführung:

Die so hergestellten Probenlösungen werden 24 Stunden bei 1400 rpm mittels eines temperierbaren Schüttlers (z.B. Fa. Eppendorf Thermomixer comfort Art.-Nr. 5355 000.011 mit Wechselblock Art.-Nr. 5362.000.019) bei 20°C geschüttelt. Von diesen Lösungen werden jeweils 180 µl abgenommen und in Beckman Polyallomer Centrifuge Tubes (Art.-Nr. 343621) überführt. Diese Lösungen werden 1 Stunde mit ca. 223.000 x g zentrifugiert (z.B. Fa. Beckman Optima L-90K Ultracentrifuge mit Type 42.2 Ti Rotor bei 42.000 rpm). Von jeder Probenlösung werden 100 µl des Überstandes abgenommen und mit DMSO zu 1:5 und 1:100 verdünnt. Es wird von jeder Verdünnung eine Abfüllung in ein geeignetes Gefäß für die HPLC-Analytik vorgenommen.

### Analytik:

Die Proben werden mittels RP-HPLC analysiert. Quantifiziert wird über eine Zwei-Punkt-Kalibrationskurve der Testverbindung in DMSO. Die Löslichkeit wird in mg/Liter ausgedrückt. Analysensequenz: 1) Kalibrierlösung 2.5 mg/ml; 2) Kalibrierlösung 20 µg/ml; 3) Probenlösung 1:5; 4) Probenlösung 1:100.

### HPLC-Methode für Säuren:

Agilent 1100 mit DAD (G1315A), quat. Pumpe (G1311A), Autosampler CTC HTS PAL, Degaser (G1322A) und Säulenthermostat (G1316A); Säule: Phenomenex Gemini C18, 50 mm x 2 mm, 5 µ; Temperatur: 40°C; Eluent A: Wasser/Phosphorsäure pH 2; Eluent B: Acetonitril; Flussrate: 0.7 ml/min; Gradient: 0-0.5 min 85% A, 15% B; Rampe: 0.5-3 min 10% A, 90% B; 3-3.5 min 10% A, 90% B; Rampe: 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) und Säulenthermostat (G1316A); Säule: VDSoptilab Kromasil 100 C18, 60 mm x 2.1 mm, 3.5 µ; Temperatur: 30°C; Eluent A: Wasser + 5 ml Perchlorsäure/Liter; Eluent B: Acetonitril; Flussrate: 0.75 ml/min; Gradient: 0-0.5 min 98% A, 2% B; Rampe: 0.5-4.5 min 10% A, 90% B; 4.5-6 min 10% A, 90% B; Rampe: 6.5-6.7 min 98% A, 2% B; 6.7-7.5 min 98% A, 2% B.

Repräsentative Löslichkeiten der erfindungsgemäßen Verbindungen der Formel (I) sind in Tabelle B angegeben.

**Tabelle B:**

| **Beispiel** | **Löslichkeit [mg/l] PBS-Puffer pH 6.5** |
|---|---|
| 6 | 340 |
| 10 | 360 |
| 21 | 360 |
| 31 | 110 |
| 52 | 100 |
| 54 | 400 |

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel® (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 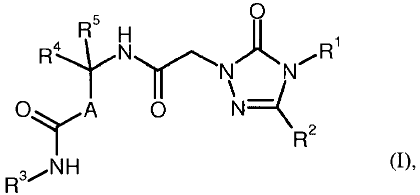 in welcher
A für eine Bindung oder -C(R^{6A}R^{6B})- steht,
wobei
R^{6A} für Wasserstoff, (C₁-C₄)-Alkyl oder Trifluormethyl steht,
R^{6B} für Wasserstoff oder (C₁-C₄)-Alkyl steht,
R¹ für (C₁-C₆)-Alkyl, (C₂-C₆)-Alkenyl, (C₂-C₆)-Alkinyl oder (C₃-C₇)-Cycloalkyl steht,
wobei (C₁-C₆)-Alkyl, (C₂-C₆)-Alkenyl und (C₂-C₆)-Alkinyl mit 1 bis 3 Substituenten unabhängig voneinander ausgewählt aus der Gruppe Deuterium, Halogen, Cyano, Oxo, Hydroxy, Trifluormethyl, (C₃-C₇)-Cycloalkyl, (C₁-C₆)-Alkoxy, Trifluormethoxy und Phenyl substituiert sein können,
worin (C₃-C₇)-Cycloalkyl mit 1 oder 2 Substituenten unabhängig voneinander ausgewählt aus der Gruppe (C₁-C₄)-Alkyl., Oxo, Hydroxy, (C₁-C₄)-Alkoxy und Amino substituiert sein kann,
und
worin (C₁-C₆)-Alkoxy mit 1 oder 2 Substituenten unabhängig voneinander ausgewählt aus der Gruppe Amino, Hydroxy, (C₁-C₄)-Alkoxy, Hydroxycarbonyl und (C₁-C₄)-Alkoxycarbonyl substituiert sein kann
und
worin Phenyl mit 1 bis 3 Substituenten unabhängig voneinander ausgewählt aus der Gruppe Halogen, Cyano, Nitro, (C₁-C₄)-Alkyl, Trifluormethyl, Hydroxy, Hydroxymethyl, (C₁-C₄)-Alkoxy, Trifluormethoxy, (C₁-C₄)-Alkoxy-methyl, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
und
wobei (C₃-C₇)-Cycloalkyl mit 1 oder 2 Substituenten unabhängig voneinander ausgewählt aus der Gruppe Fluor, (C₁-C₄)-Alkyl, (C₁-C₄)-Alkoxy, Hydroxy, Amino und Oxo substituiert sein kann,
R² für Benzothienyl, Phenyl, Thienyl oder Furyl steht,
wobei Benzothienyl, Phenyl, Thienyl und Furyl mit 1 bis 3 Substituenten unabhängig voneinander ausgewählt aus der Gruppe Halogen, Cyano, Nitro, (C₁-C₄)-Alkyl, Trifluormethyl, Hydroxy, (C₁-C₄)-Alkoxy und Trifluormethoxy substituiert sein können,
R³ für eine Gruppe der Formel 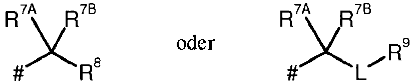 steht, wobei
# für die Anknüpfstelle an das Stickstoffatom steht,
L für (C₁-C₄)-Alkandiyl steht,
wobei (C₁-C₄)-Alkandiyl mit 1 bis 3 Substituenten unabhängig voneinander ausgewählt aus der Gruppe Fluor und (C₁-C₄)-Alkyl substituiert sein kann,
R^{7A} für Wasserstoff oder (C₁-C₄)-Alkyl steht,
R^{7B} für Wasserstoff oder (C₁-C₄)-Alkyl steht,
oder
R^{7A} und R^{7B} zusammen mit dem Kohlenstoffatom, an das sie gebunden sind, einen 3- bis 6-gliedrigen Carbocyclus bilden,
R⁸ für Hydroxycarbonyl, (C₁-C₄)-Alkoxycarbonyl, Aminocarbonyl, Mono-(C₁-C₄)-alkylaminocarbonyl oder Di-(C₁-C₄)-alkylaminocarbonyl steht,
R⁹ für Hydroxy, Trifluormethoxy, (C₁-C₄)-Alkoxy, Hydroxycarbonyl, (C₁-C₄)-Alkoxycarbonyl, Aminocarbonyl, Mono-(C₁-C₄)-alkylaminocarbonyl oder Di-(C₁-C₄)-alkylaminocarbonyl steht,
R⁴ für Phenyl, Naphthyl oder 5- bis 10-gliedriges Heteroaryl steht,
wobei Phenyl, Naphthyl und 5- bis 10-gliedriges Heteroaryl mit 1 bis 3 Substituenten unabhängig voneinander ausgewählt aus der Gruppe Halogen, Cyano, Nitro, (C₁-C₄)-Alkyl, Difluormethyl, Trifluormethyl, Hydroxy, (C₁-C₄)-Alkoxy, Difluormethoxy und Trifluormethoxy substituiert sein können,
R⁵ für Wasserstoff, Deuterium, Trifluormethyl oder (C₁-C₄)-Alkyl steht,
sowie ihre Salze, Solvate und Solvate der Salze.

2. Verbindung der Formel (I) nach Anspruch 1, in welcher
A für eine Bindung oder -C(R^{6A}R^{6B})- steht,
wobei
R^{6A} für Wasserstoff steht,
R^{6B} für Wasserstoff steht,
R¹ für (C₁-C₆)-Alkyl, (C₂-C₆)-Alkenyl oder (C₃-C₆)-Cycloalkyl steht,
wobei (C₁-C₆)-Alkyl und (C₂-C₆)-Alkenyl mit 1 bis 3 Substituenten unabhängig voneinander ausgewählt aus der Gruppe Fluor, Chlor, Cyano, Oxo, Hydroxy, Trifluormethyl, (C₃-C₆)-Cycloalkyl, (C₁-C₄)-Alkoxy, Trifluormethoxy und Phenyl substituiert sein können,
worin (C₃-C₆)-Cycloalkyl mit 1 oder 2 Substituenten unabhängig voneinander ausgewählt aus der Gruppe Methyl, Ethyl, Oxo, Hydroxy, Methoxy, Ethoxy und Amino substituiert sein kann,
und
worin Phenyl mit einem Substituenten ausgewählt aus der Gruppe Fluor, Chlor, Cyano, Methyl, Ethyl, Trifluormethyl, Methoxy, Ethoxy, Trifluormethoxy, Methoxymethyl, Ethoxymethyl, Hydroxycarbonyl, Methoxycarbonyl, Ethoxycarbonyl und Aminocarbonyl substituiert sein kann,
und
wobei (C₃-C₆)-Cycloalkyl mit 1 oder 2 Substituenten unabhängig voneinander ausgewählt aus der Gruppe Fluor, Methyl, Ethyl, Methoxy, Ethoxy, Hydroxy, Amino und Oxo substituiert sein kann,
R² für Phenyl oder Thienyl steht,
wobei Phenyl und Thienyl mit 1 oder 2 Substituenten unabhängig voneinander ausgewählt aus der Gruppe Fluor, Chlor, Brom, Methyl, Ethyl, Trifluormethyl, Hydroxy, Methoxy, Ethoxy und Trifluormethoxy substituiert sein können,
R³ für eine Gruppe der Formel 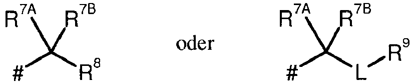 steht, wobei
# für die Anknüpfstelle an das Stickstoffatom steht,
L für Methylen steht,
wobei Methylen mit 1 oder 2 Substituenten Methyl substituiert sein kann,
R^{7A} für Wasserstoff oder Methyl steht,
R^{7B} für Wasserstoff oder Methyl steht,
oder
R^{7A} und R^{7B} zusammen mit dem Kohlenstoffatom, an das sie gebunden sind, einen Cyclopropyl-Ring bilden,
R⁸ für Hydroxycarbonyl, Methoxycarbonyl, Ethoxycarbonyl, Aminocarbonyl, Methylaminocarbonyl, Ethylaminocarbonyl, Dimethylaminocarbonyl oder Diethylaminocarbonyl steht,
R⁹ für Hydroxy, Trifluormethoxy, Methoxy, Ethoxy, Hydroxycarbonyl, Methoxycarbonyl, Ethoxycarbonyl, Aminocarbonyl, Methylaminocarbonyl, Ethylaminocarbonyl, Dimethylaminocarbonyl oder Diethylaminocarbonyl steht,
R⁴ für Phenyl steht,
wobei Phenyl mit 1 bis 3 Substituenten unabhängig voneinander ausgewählt aus der Gruppe Fluor, Chlor, Cyano, Methyl, Ethyl, Difluormethyl, Trifluormethyl, Methoxy, Difluormethoxy und Trifluormethoxy substituiert ist,
R⁵ für Wasserstoff oder Methyl steht,
sowie ihre Salze, Solvate und Solvate der Salze.

3. Verbindung der Formel (I) nach Anspruch 1 oder 2, in welcher
A für eine Bindung oder -C(R^{6A}R^{6B})- steht,
wobei
R^{6A} für Wasserstoff steht,
R^{6B} für Wasserstoff steht,
R¹ für (C₂-C₄)-Alkyl, (C₂-C₄)-Alkenyl oder Cyclopropyl steht,
wobei (C₂-C₄)-Alkyl und (C₂-C₄)-Alkenyl mit 1 oder 2 Substituenten unabhängig voneinander ausgewählt aus der Gruppe Fluor, Oxo, Hydroxy und Trifluormethyl substituiert sein können,
R² für Phenyl steht,
wobei Phenyl mit einem Substituenten ausgewählt aus der Gruppe Fluor oder Chlor substituiert ist,
R³ für eine Gruppe der Formel 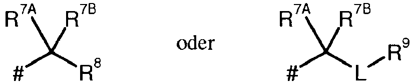 steht, wobei
# für die Anknüpfstelle an das Stickstoffatom steht,
L für Methylen steht,
R^{7A} für Wasserstoff oder Methyl steht,
R^{7B} für Wasserstoff oder Methyl steht,
oder
R^{7A} und R^{7B} zusammen mit dem Kohlenstoffatom, an das sie gebunden sind, einen Cyclopropyl-Ring bilden,
R⁸ für Hydroxycarbonyl, Methoxycarbonyl, Ethoxycarbonyl oder Aminocarbonyl steht,
R⁹ für Hydroxy, Methoxy, Hydroxycarbonyl, Methoxycarbonyl, Ethoxycarbonyl oder Aminocarbonyl steht,
R⁴ für eine Gruppe der Formel 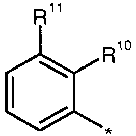 steht, wobei
* für die Anknüpfstelle an die Gruppe -C(R⁵)(AC(=O)NHR³)- steht,
R¹⁰ für Wasserstoff, Chlor, Trifluormethyl, Trifluormethoxy oder Methoxy steht,
A¹¹ für Wasserstoff, Fluor, Chlor, Trifluormethyl, Trifluormethoxy oder Methoxy steht,
wobei mindestens einer der Reste R¹⁰ und R¹¹ von Wasserstoff verschieden ist,
R⁵ für Wasserstoff oder Methyl steht,
sowie ihre Salze, Solvate und Solvate der Salze.

4. Verfahren zur Herstellung von Verbindungen der Formel (I), wie in den Ansprüchen 1 bis 3 definiert, **dadurch gekennzeichnet, dass** man
[A] eine Verbindung der Formel (II) 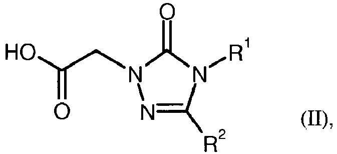 in welcher R¹ und R² jeweils die in den Ansprüchen 1 bis 3 angegebenen Bedeutungen haben,
in einem inerten Lösungsmittel unter Aktivierung der Carbonsäure-Funktion mit einer Verbindung der Formel (III) 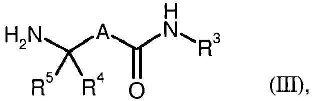 in welcher A, R³, R⁴ und R⁵ jeweils die in den Ansprüchen 1 bis 3 angegebenen Bedeutungen haben,
kuppelt,
oder
[B] eine Verbindung der Formel (IV) 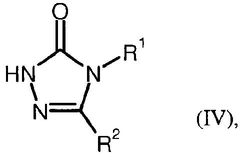 in welcher R¹ und R² jeweils die in den Ansprüchen 1 bis 3 angegebenen Bedeutungen haben,
in einem inerten Lösungsmittel in Gegenwart einer Base mit einer Verbindung der Formel (V) 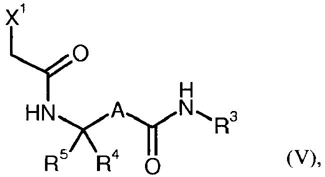 in welcher A, R³, R⁴ und R⁵ jeweils die in den Ansprüchen 1 bis 3 angegebenen Bedeutungen haben
und
X¹ für eine Abgangsgruppe, wie beispielsweise Halogen, Mesylat oder Tosylat, steht,
umsetzt,
oder
[C] eine Verbindung der Formel (VI) 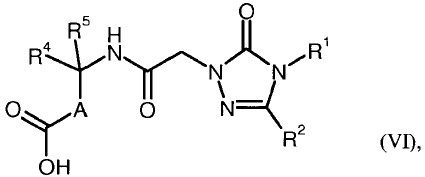 in welcher A, R¹, R², R⁴ und R⁵ jeweils die in den Ansprüchen 1 bis 3 angegebenen Bedeutungen haben,
in einem inerten Lösungsmittel unter Aktivierung der Carbonsäure-Funktion mit einer Verbindung der Formel (VII)
H₂N-R³ (VII),
in welcher R³ die in den Ansprüchen 1 bis 3 angegebene Bedeutung hat,
kuppelt,
und die resultierenden Verbindungen der Formel (I) gegebenenfalls mit den entsprechenden (i) Lösungsmitteln und/oder (ii) Basen oder Säuren in ihre Solvate, Salze und/oder Solvate der Salze überführt.

5. Verbindung der Formel (I), wie in einem der Ansprüche 1 bis 3 definiert, zur Behandlung und/oder Prophylaxe von Krankheiten.

6. Verbindung der Formel (I), wie in einem der Ansprüche 1 bis 3 definiert, zur Verwendung in einem Verfahren zur Behandlung und/ oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

7. Verwendung einer Verbindung der Formel (I), wie in einem der Ansprüche 1 bis 3 definiert, zur Herstellung eines Arzneimittels zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

8. Arzneimittel enthaltend eine Verbindung der Formel (I), wie in einem der Ansprüche 1 bis 3 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 3 definiert, in Kombination mit einem oder mehreren weiteren Wirkstoffen ausgewählt aus der Gruppe bestehend aus Diuretika, Angiotensin AII-Antagonisten, ACE-Hemmer, beta-Rezeptoren-Blocker, Mineralocorticoid-Rezeptor-Antagonisten, organische Nitrate, NO-Donatoren und positiv-inotrop wirksame Substanzen.

10. Arzneimittel nach Anspruch 8 oder 9 zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

## Claims

1. Compound of the formula (I) 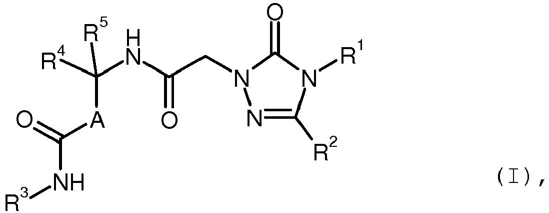 in which
A represents a bond or -C(R^{6A}R^{6B}) -,
where
R^{6A} represents hydrogen, (C₁-C₄)-alkyl or trifluoromethyl,
R^{6B} represents hydrogen or (C₁-C₄)-alkyl,
R¹ represents (C₁-C₆)-alkyl, (C₂-C₆)-alkenyl, (C₂-C₆)-alkynyl or (C₃-C₇)-cycloalkyl,
where (C₁-C₆)-alkyl, (C₂-C₆)-alkenyl and (C₂-C₆)-alkynyl may be substituted by 1 to 3 substituents independently of one another selected from the group consisting of deuterium, halogen, cyano, oxo, hydroxy, trifluoromethyl, (C₃-C₇)-cycloalkyl, (C₁-C₆)-alkoxy, trifluoromethoxy and phenyl,
in which (C₃-C₇)-cycloalkyl may be substituted by 1 or 2 substituents independently of one another selected from the group consisting of (C₁-C₄)-alkyl, oxo, hydroxy, (C₁-C₄)-alkoxy and amino,
and
in which (C₁-C₆)-alkoxy may be substituted by 1 or 2 substituents independently of one another selected from the group consisting of amino, hydroxy, (C₁-C₄)-alkoxy, hydroxycarbonyl and (C₁-C₄) -alkoxycarbonyl
and
in which phenyl may be substituted by 1 to 3 substituents independently of one another selected from the group consisting of halogen, cyano, nitro, (C₁-C₄)-alkyl, trifluoromethyl, hydroxy, hydroxymethyl, (C₁-C₄)-alkoxy, trifluoromethoxy, (C₁-C₄)-alkoxymethyl, hydroxycarbonyl, (C₁-C₄)-alkoxycarbonyl, aminocarbonyl, mono-(C₁-C₄)-alkyl-aminocarbonyl and di-(C₁-C₄)-alkyl-aminocarbonyl,
and
where (C₃-C₇)-cycloalkyl may be substituted by 1 or 2 substituents independently of one another selected from the group consisting of fluorine, (C₁-C₄)-alkyl, (C₁-C₄)-alkoxy, hydroxy, amino and oxo,
R² represents benzothienyl, phenyl, thienyl or furyl,
where benzothienyl, phenyl, thienyl and furyl may be substituted by 1 to 3 substituents independently of one another selected from the group consisting of halogen, cyano, nitro, (C₁-C₄)-alkyl, trifluoromethyl, hydroxy, (C₁-C₄)-alkoxy and trifluoromethoxy,
R³ represents a group of the formula 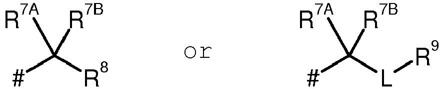 where
# represents the point of attachment to the nitrogen atom,
L represents (C₁-C₄)-alkanediyl,
where (C₁-C₄)-alkanediyl may be substituted by 1 to 3 substituents independently of one another selected from the group consisting of fluorine and (C₁-C₄)-alkyl,
R^{7A} represents hydrogen or (C₁-C₄)-alkyl,
R^{7B} represents hydrogen or (C₁-C₄)-alkyl,
or
R^{7A} and R^{7B} together with the carbon atom to which they are attached form a 3-to 6-membered carbocycle,
R⁸ represents hydroxycarbonyl, (C₁-C₄)-alkoxycarbonyl, aminocarbonyl, mono-(C₁-C₄)-alkylaminocarbonyl or di-(C₁-C₄)-alkylaminocarbonyl,
R⁹ represents hydroxy, trifluoromethoxy, (C₁-C₄)-alkoxy, hydroxycarbonyl, (C₁-C₄)-alkoxycarbonyl, aminocarbonyl, mono-(C₁-C₄)-alkylaminocarbonyl or di-(C₁-C₄)-alkylaminocarbonyl,
R⁴ represents phenyl, naphthyl or 5- to 10-membered heteroaryl,
where phenyl, naphthyl and 5- to 10-membered heteroaryl may be substituted by 1 to 3 substituents independently of one another selected from the group consisting of halogen, cyano, nitro, (C₁-C₄)-alkyl, difluoromethyl, trifluoromethyl, hydroxy, (C₁-C₄)-alkoxy, difluoromethoxy and trifluoromethoxy,
R⁵ represents hydrogen, deuterium, trifluoromethyl or (C₁-C₄)-alkyl,
and its salts, solvates and solvates of the salts.

2. Compound of the formula (I) according to Claim 1, in which
A represents a bond or -C(R^{6A}R^{6B}) -,
where
R^{6A} represents hydrogen,
R^{6B} represents hydrogen,
R¹ represents (C₁-C₆)-alkyl, (C₂-C₆) -alkenyl or (C₃-C₆)-cycloalkyl,
where (C₁-C₆)-alkyl and (C₂-C₆) -alkenyl may be substituted by 1 to 3 substituents independently of one another selected from the group consisting of fluorine, chlorine,
cyano, oxo, hydroxy, trifluoromethyl, (C₃-C₆)-cycloalkyl, (C₁-C₄)-alkoxy, trifluoromethoxy and phenyl,
in which (C₃-C₆)-cycloalkyl may be substituted by 1 or 2 substituents independently of one another selected from the group consisting of methyl, ethyl, oxo, hydroxy, methoxy, ethoxy and amino,
and
in which phenyl may be substituted by a substituent selected from the group consisting of fluorine, chlorine, cyano, methyl, ethyl, trifluoromethyl, methoxy, ethoxy, trifluoromethoxy, methoxymethyl, ethoxymethyl, hydroxycarbonyl, methoxycarbonyl, ethoxycarbonyl and aminocarbonyl,
and
where (C₃-C₆)-cycloalkyl may be substituted by 1 or 2 substituents independently of one another selected from the group consisting of fluorine, methyl, ethyl, methoxy, ethoxy, hydroxy, amino and oxo,
R² represents phenyl or thienyl,
where phenyl and thienyl may be substituted by 1 or 2 substituents independently of one another selected from the group consisting of fluorine, chlorine, bromine, methyl, ethyl, trifluoromethyl, hydroxy, methoxy, ethoxy and trifluoromethoxy,
R³ represents a group of the formula 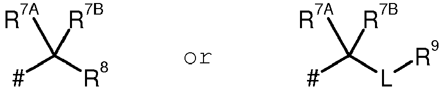 where
# represents the point of attachment to the nitrogen atom,
L represents methylene,
where methylene may be substituted by 1 or 2 methyl substituents,
R^{7A} represents hydrogen or methyl,
R^{7B} represents hydrogen or methyl,
or
R^{7A} and R^{7B} together with the carbon atom to which they are attached form a cyclopropyl ring,
R⁸ represents hydroxycarbonyl, methoxycarbonyl, ethoxycarbonyl, aminocarbonyl, methylaminocarbonyl, ethylaminocarbonyl, dimethylaminocarbonyl or diethylamino-carbonyl,
R⁹ represents hydroxy, trifluoromethoxy, methoxy, ethoxy, hydroxycarbonyl, methoxycarbonyl, ethoxycarbonyl, aminocarbonyl, methylaminocarbonyl, ethylaminocarbonyl, dimethylaminocarbonyl or diethylaminocarbonyl,
R⁴ represents phenyl,
where phenyl is substituted by 1 to 3 substituents independently of one another selected from the group consisting of fluorine, chlorine, cyano, methyl, ethyl, difluoromethyl, trifluoromethyl, methoxy, difluoromethoxy and trifluoromethoxy,
R⁵ represents hydrogen or methyl,
and its salts, solvates and solvates of the salts.

3. Compound of the formula (I) according to Claim 1 or 2, in which
A represents a bond or -C(R^{6A}R^{6B}) -,
where
R^{6A} represents hydrogen,
R^{6B} represents hydrogen,
R¹ represents (C₂-C₄)-alkyl, (C₂-C₄) -alkenyl or cyclopropyl,
where (C₂-C₄) -alkyl and (C₂-C₄) -alkenyl may be substituted by 1 or 2 substituents independently of one another selected from the group consisting of fluorine, oxo, hydroxy and trifluoromethyl,
R² represents phenyl,
where phenyl is substituted by a substituent selected from the group consisting of fluorine and chlorine,
R³ represents a group of the formula 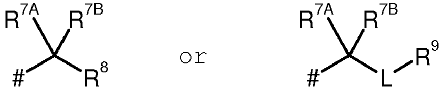 where
# represents the point of attachment to the nitrogen atom,
L represents methylene,
R^{7A} represents hydrogen or methyl,
R^{7B} represents hydrogen or methyl,
or
R^{7A} and R^{7B} together with the carbon atom to which they are attached form a cyclopropyl ring,
R⁸ represents hydroxycarbonyl, methoxycarbonyl, ethoxycarbonyl or aminocarbonyl,
R⁹ represents hydroxy, methoxy, hydroxycarbonyl, methoxycarbonyl, ethoxycarbonyl or aminocarbonyl,
R⁴ represents a group of the formula 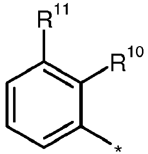 where
* represents the point of attachment to the group -C(R⁵)(AC(=O)NHR³)-,
R¹⁰ represents hydrogen, chlorine, trifluoromethyl, trifluoromethoxy or methoxy,
R¹¹ represents hydrogen, fluorine, chlorine, trifluoromethyl, trifluoromethoxy or methoxy,
where at least one of the radicals R¹⁰ and R¹¹ is different from hydrogen,
R⁵ represents hydrogen or methyl,
and its salts, solvates and solvates of the salts.

4. Process for preparing compounds of the formula (I) as defined in Claims 1 to 3, **characterized in that**
[A] a compound of the formula (II) 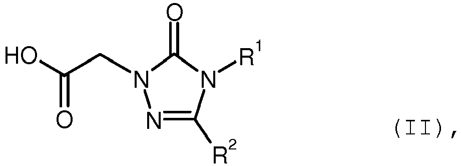 in which R¹ and R² each have the meanings given in any of Claims 1 to 3,
is coupled in an inert solvent with activation of the carboxylic acid function with a compound of the formula (III) 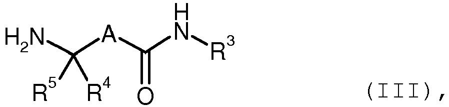 in which A, R³, R⁴ and R⁵ each have the meanings given in any of Claims 1 to 3,
or
[B] a compound of the formula (IV) 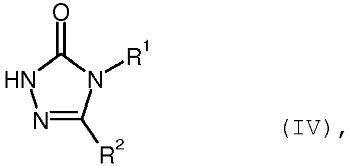 in which R¹ and R² each have the meanings given in any of Claims 1 to 3,
is reacted in an inert solvent in the presence of a base with a compound of the formula (V) 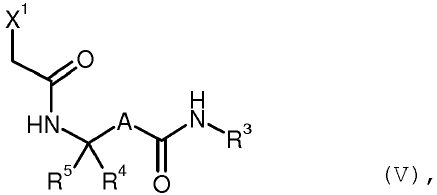 in which A, R³, R⁴ and R⁵ each have the meanings given in any of Claims 1 to 3
and
X¹ represents a leaving group, for example halogen, mesylate or tosylate,
or
[C] a compound of the formula (VI) 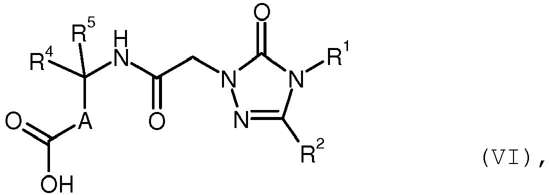 in which A, R¹, R², R⁴ and R⁵ each have the meanings given in any of Claims 1 to 3,
is coupled in an inert solvent with activation of the carboxylic acid function with a compound of the formula (VII)
H₂N-R³ (VII),
in which R³ has the meaning given in any of Claims 1 to 3,
and the resulting compounds of the formula (I) are optionally converted with the appropriate (i) solvents and/or (ii) acids or bases into their solvates, salts and/or solvates of the salts.

5. Compound of the formula (I) as defined in any of Claims 1 to 3 for the treatment and/or prophylaxis of diseases.

6. Compound of the formula (I) as defined in any of Claims 1 to 3 for use in a method for the treatment and/or prophylaxis of acute and chronic heart failure, hypervolaemic and euvolaemic hyponatraemia, cirrhosis of the liver, ascites, oedema and the syndrome of inadequate ADH secretion (SIADH).

7. Use of a compound of the formula (I) as defined in any of Claims 1 to 3 for preparing a medicament for the treatment and/or prophylaxis of acute and chronic heart failure, hypervolaemic and euvolaemic hyponatraemia, cirrhosis of the liver, ascites, oedema and the syndrome of inadequate ADH secretion (SIADH).

8. Medicament comprising a compound of the formula (I) as defined in any of Claims 1 to 3 in combination with an inert, non-toxic, pharmaceutically suitable auxiliary.

9. Medicament comprising a compound of the formula (I) as defined in any of Claims 1 to 3 in combination with one or more further active substances selected from the group consisting of diuretics, angiotensin AII antagonists, ACE inhibitors, beta-receptor blockers, mineralocorticoid receptor antagonists, organic nitrates, NO donors and positive-inotropic active substances.

10. Medicament according to Claim 8 or 9 for the treatment and/or prophylaxis of acute and chronic heart failure, hypervolaemic and euvolaemic hyponatraemia, cirrhosis of the liver, ascites, oedema and the syndrome of inadequate ADH secretion (SIADH).

## Revendications

1. Composé de formule (I) 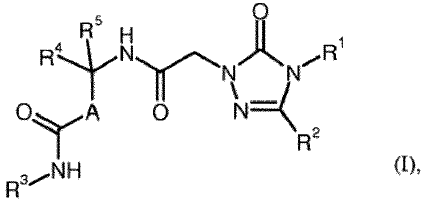 dans lequel
A représente une liaison ou -C(R^{6A}R^{6B})-,
R^{6A} représentant hydrogène, alkyle en (C₁-C₄) ou trifluorométhyle,
R^{6B} représentant hydrogène ou alkyle en (C₁-C₄),
R¹ représente alkyle en (C₁-C₆), alcényle en (C₂-C₆), alcynyle en (C₂-C₆) ou cycloalkyle en (C₃-C₇),
l'alkyle en (C₁-C₆), l'alcényle en (C₂-C₆) et l'alcynyle en (C₂-C₆) pouvant être substitués avec 1 à 3 substituants choisis indépendamment les uns des autres dans le groupe constitué par deutérium, halogène, cyano, oxo, hydroxy, trifluorométhyle, cycloalkyle en (C₃-C₇), alcoxy en (C₁-C₆), trifluorométhoxy et phényle,
le cycloalkyle en (C₃-C₇) pouvant être substitué avec 1 ou 2 substituants choisis indépendamment l'un de l'autre dans le groupe constitué par alkyle en (C₁-C₄), oxo, hydroxy, alcoxy en (C₁-C₄) et amino,
et
l'alcoxy en (C₁-C₆) pouvant être substitué avec 1 ou 2 substituants choisis indépendamment l'un de l'autre dans le groupe constitué par amino, hydroxy, alcoxy en (C₁-C₄), hydroxycarbonyle et alcoxycarbonyle en (C₁-C₄),
et
le phényle pouvant être substitué avec 1 à 3 substituants choisis indépendamment les uns des autres dans le groupe constitué par halogène, cyano, nitro, alkyle en (C₁-C₄), trifluorométhyle, hydroxy, hydroxyméthyle, alcoxy en (C₁-C₄), trifluorométhoxy, alcoxyméthyle en (C₁-C₄), hydroxycarbonyle, alcoxycarbonyle en (C₁-C₄), aminocarbonyle, mono-alkylaminocarbonyle en (C₁-C₄) et di-alkylaminocarbonyle en (C₁-C₄),
et
le cycloalkyle en (C₃-C₇) pouvant être substitué avec 1 ou 2 substituants choisis indépendamment l'un de l'autre dans le groupe constitué par fluor, alkyle en (C₁-C₄), alcoxy en (C₁-C₄), hydroxy, amino et oxo,
R² représente benzothiényle, phényle, thiényle ou furyle,
le benzothiényle, le phényle, le thiényle et le furyle pouvant être substitués avec 1 à 3 substituants choisis indépendamment les uns des autres dans le groupe constitué par halogène, cyano, nitro, alkyle en (C₁-C₄), trifluorométhyle, hydroxy, alcoxy en (C₁-C₄) et trifluorométhoxy,
R³ représente un groupe de formule 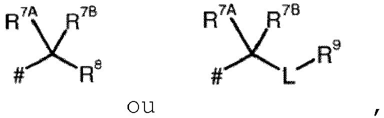
dans lesquelles
# représente l'emplacement de liaison à l'atome d'azote,
L représente alcanediyle en (C₁-C₄),
l'alcanediyle en (C₁-C₄) pouvant être substitué avec 1 à 3 substituants choisis indépendamment les uns des autres dans le groupe constitué par fluor et alkyle en (C₁-C₄),
R^{7A} représente hydrogène ou alkyle en (C₁-C₄),
R^{7B} représente hydrogène ou alkyle en (C₁-C₄),
ou
R^{7A} et R^{7B} forment ensemble avec l'atome de carbone auquel ils sont reliés un carbocycle de 3 à 6 éléments,
R⁸ représente hydroxycarbonyle, alcoxycarbonyle en (C₁-C₄), aminocarbonyle, mono-alkylaminocarbonyle en (C₁-C₄) ou di-alkylaminocarbonyle en (C₁-C₄),
R⁹ représente hydroxy, trifluorométhoxy, alcoxy en (C₁-C₄), hydroxycarbonyle, alcoxycarbonyle en (C₁-C₄), aminocarbonyle, mono-alkylaminocarbonyle en (C₁-C₄) ou di-alkylaminocarbonyle en (C₁-C₄),
R⁴ représente phényle, naphtyle ou hétéroaryle de 5 à 10 éléments,
le phényle, le naphtyle et l'hétéroaryle de 5 à 10 éléments pouvant être substitués avec 1 à 3 substituants choisis indépendamment les uns des autres dans le groupe constitué par halogène, cyano, nitro, alkyle en (C₁-C₄), difluorométhyle, trifluorométhyle, hydroxy, alcoxy en (C₁-C₄), difluorométhoxy et trifluorométhoxy,
R⁵ représente hydrogène, deutérium, trifluorométhyle ou alkyle en (C₁-C₄),
ainsi que leurs sels, solvates et solvates des sels.

2. Composé de formule (I) selon la revendication 1, dans lequel
A représente une liaison ou -C(R^{6A}R^{6B})-,
R^{6A} représentant hydrogène,
R^{6B} représentant hydrogène,
R¹ représente alkyle en (C₁-C₆), alcényle en (C₂-C₆) ou cycloalkyle en (C₃-C₆),
l'alkyle en (C₁-C₆) et l'alcényle en (C₂-C₆) pouvant être substitués avec 1 à 3 substituants choisis indépendamment les uns des autres dans le groupe constitué par fluor, chlore, cyano, oxo, hydroxy, trifluorométhyle, cycloalkyle en (C₃-C₆), alcoxy en (C₁-C₆), trifluorométhoxy et phényle,
le cycloalkyle en (C₃-C₆) pouvant être substitué avec 1 ou 2 substituants choisis indépendamment l'un de l'autre dans le groupe constitué par méthyle, éthyle, oxo, hydroxy, méthoxy, éthoxy et amino
et
le phényle pouvant être substitué avec un substituant choisi dans le groupe constitué par fluor, chlore, cyano, méthyle, éthyle, trifluorométhyle, méthoxy, éthoxy, trifluorométhoxy, méthoxyméthyle, éthoxyméthyle, hydroxycarbonyle, méthoxycarbonyle, éthoxycarbonyle et aminocarbonyle,
et
le cycloalkyle en (C₃-C₆) pouvant être substitué avec 1 ou 2 substituants choisis indépendamment l'un de l'autre dans le groupe constitué par fluor, méthyle, éthyle, méthoxy, éthoxy, hydroxy, amino et oxo,
R² représente phényle ou thiényle,
le phényle et le thiényle pouvant être substitués avec 1 ou 2 substituants choisis indépendamment l'un de l'autre dans le groupe constitué par fluor, chlore, brome, méthyle, éthyle, trifluorométhyle, hydroxy, méthoxy, éthoxy et trifluorométhoxy,
R³ représente un groupe de formule 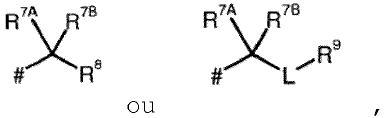 dans lesquelles
# représente l'emplacement de liaison à l'atome d'azote,
L représente méthylène,
le méthylène pouvant être substitué avec 1 ou 2 substituants méthyle,
R^{7A} représente hydrogène ou méthyle,
R^{7B} représente hydrogène ou méthyle,
ou
R^{7A} et R^{7B} forment ensemble avec l'atome de carbone auquel ils sont reliés un cycle cyclopropyle,
R⁸ représente hydroxycarbonyle, méthoxycarbonyle, éthoxycarbonyle, aminocarbonyle, méthylaminocarbonyle, éthylaminocarbonyle, diméthylaminocarbonyle ou diéthylaminocarbonyle,
R⁹ représente hydroxy, trifluorométhoxy, méthoxy,
éthoxy, hydroxycarbonyle, méthoxycarbonyle, éthoxycarbonyle, aminocarbonyle, méthylaminocarbonyle, éthylaminocarbonyle, diméthylaminocarbonyle ou diéthylaminocarbonyle,
R⁴ représente phényle,
le phényle pouvant être substitué avec 1 à 3 substituants choisis indépendamment les uns des autres dans le groupe constitué par fluor, chlore, cyano, méthyle, éthyle, difluorométhyle, trifluorométhyle, méthoxy, difluorométhoxy et trifluorométhoxy,
R⁵ représente hydrogène ou méthyle,
ainsi que leurs sels, solvates et solvates des sels.

3. Composé de formule (I) selon la revendication 1 ou 2, dans lequel
A représente une liaison ou -C(R^{6A}R^{6B})-,
R^{6A} représentant hydrogène,
R^{6B} représentant hydrogène,
R¹ représente alkyle en (C₂-C₄), alcényle en (C₂-C₄) ou cyclopropyle,
l'alkyle en (C₂-C₄) et l'alcényle en (C₂-C₄) pouvant être substitués avec 1 ou 2 substituants choisis indépendamment l'un de l'autre dans le groupe constitué par fluor, oxo, hydroxy et trifluorométhyle,
R² représente phényle,
le phényle pouvant être substitué avec un substituant choisi dans le groupe constitué par fluor ou chlore,
R³ représente un groupe de formule 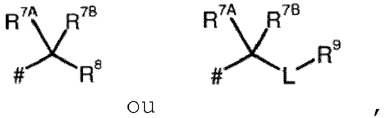 dans lesquelles
# représente l'emplacement de liaison à l'atome d'azote,
L représente méthylène,
R^{7A} représente hydrogène ou méthyle,
R^{7B} représente hydrogène ou méthyle,
ou
R^{7A} et R^{7B} forment ensemble avec l'atome de carbone auquel ils sont reliés un cycle cyclopropyle,
R⁸ représente hydroxycarbonyle, méthoxycarbonyle, éthoxycarbonyle ou aminocarbonyle,
R⁹ représente hydroxy, méthoxy, hydroxycarbonyle, méthoxycarbonyle, éthoxycarbonyle ou aminocarbonyle, R⁴ représente un groupe de formule 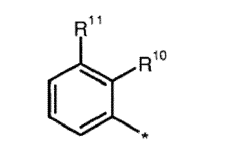 dans laquelle
* représente l'emplacement de liaison au groupe-C(R⁵) (AC(=O)NHR³)-,
R¹⁰ représente hydrogène, chlore, trifluorométhyle, trifluorométhoxy ou méthoxy,
R¹¹ représente hydrogène, fluor, chlore, trifluorométhyle, trifluorométhoxy ou méthoxy,
au moins un des radicaux R¹⁰ et R¹¹ étant différent de l'hydrogène,
R⁵ représente hydrogène ou méthyle,
ainsi que leurs sels, solvates et solvates des sels.

4. Procédé de fabrication de composés de formule (I), tels que définis dans les revendications 1 à 3, **caractérisé en ce que**
[A] un composé de formule (II) 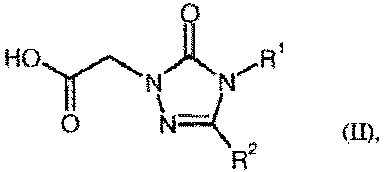 dans laquelle R¹ et R² ont chacun les significations indiquées dans les revendications 1 à 3,
est couplé dans un solvant inerte avec activation de la fonction acide carboxylique avec un composé de formule (III) 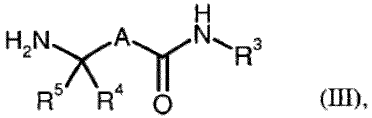 dans laquelle A, R³, R⁴ et R⁵ ont chacun les significations indiquées dans les revendications 1 à 3, ou
[B] un composé de formule (IV) 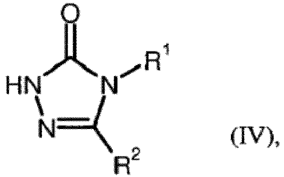 dans laquelle R¹ et R² ont chacun les significations indiquées dans les revendications 1 à 3,
est mis en réaction dans un solvant inerte en présence d'une base avec un composé de formule (V) 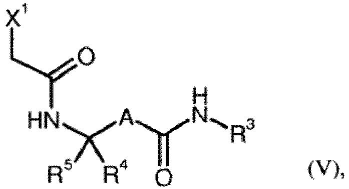 dans laquelle A, R³, R⁴ et R⁵ ont chacun les significations indiquées dans les revendications 1 à 3, et
X¹ représente un groupe partant, tel que par exemple halogène, mésylate ou tosylate,
ou
[C] un composé de formule (VI) 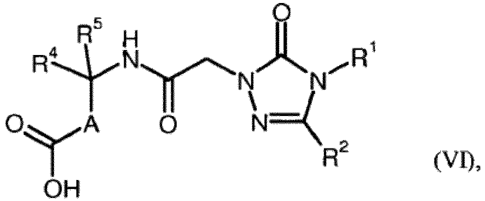 dans laquelle A, R¹, R², R⁴ et R⁵ ont chacun les significations indiquées dans les revendications 1 à 3, est couplé dans un solvant inerte avec activation de la fonction acide carboxylique avec un composé de formule (VII)
**H₂N-R³ (VII),**
dans laquelle R³ a la signification indiquée dans les revendications 1 à 3,
et les composés de formule (I) résultants sont éventuellement transformés avec (i) les solvants et/ou (ii) les bases ou les acides appropriés en leurs solvates, sels et/ou solvates des sels.

5. Composé de formule (I), tel que défini dans l'une quelconque des revendications 1 à 3, pour le traitement et/ou la prophylaxie de maladies.

6. Composé de formule (I), tel que défini dans l'une quelconque des revendications 1 à 3, destiné à une utilisation dans un procédé de traitement et/ou de prophylaxie de l'insuffisance cardiaque aigue et chronique, l'hyponatrémie hypervolémique et euvolémique, la cirrhose du foie, l'ascite, les oedèmes et le syndrome de la sécrétion inappropriée d'ADH (SIADH).

7. Utilisation d'un composé de formule (I), tel que défini dans l'une quelconque des revendications 1 à 3, pour la fabrication d'un médicament pour le traitement et/ou la prophylaxie de l'insuffisance cardiaque aigue et chronique, l'hyponatrémie hypervolémique et euvolémique, la cirrhose du foie, l'ascite, les oedèmes et le syndrome de la sécrétion inappropriée d'ADH (SIADH).

8. Médicament contenant un composé de formule (I), tel que défini dans l'une quelconque des revendications 1 à 3, en combinaison avec un adjuvant inerte, non toxique, pharmaceutiquement approprié.

9. Médicament contenant un composé de formule (I), tel que défini dans l'une quelconque des revendications 1 à 3, en combinaison avec un ou plusieurs autres agents actifs choisis dans le groupe constitué par les diurétiques, les antagonistes d'angiotensine AII, les inhibiteurs d'ACE, les bloquants des récepteurs bêta, les antagonistes des récepteurs minéralocorticoïdes, les nitrates organiques, les donneurs de NO et les substances à effet inotrope positif.

10. Médicament selon la revendication 8 ou 9, pour le traitement et/ou la prophylaxie de l'insuffisance cardiaque aigue et chronique, l'hyponatrémie hypervolémique et euvolémique, la cirrhose du foie, l'ascite, les oedèmes et le syndrome de la sécrétion inappropriée d'ADH (SIADH).
